(19) Europäisches Patentamt / European Patent Office / Office européen des brevets

(11) **EP 4 425 503 A1**

(12) **EUROPEAN PATENT APPLICATION**
published in accordance with Art. 153(4) EPC

(43) Date of publication:
**04.09.2024 Bulletin 2024/36**

(21) Application number: **22886571.3**

(22) Date of filing: **28.09.2022**

(51) International Patent Classification (IPC):
***G16C 20/80*** (2019.01)      ***G16C 60/00*** (2019.01)

(52) Cooperative Patent Classification (CPC):
**G16C 20/80; G16C 60/00**

(86) International application number:
**PCT/JP2022/036261**

(87) International publication number:
**WO 2023/074239 (04.05.2023 Gazette 2023/18)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.10.2021 JP 2021176168**

(71) Applicant: **Panasonic Intellectual Property
Management Co., Ltd.
Kadoma-shi, Osaka 571-0057 (JP)**

(72) Inventor: **WAKASUGI, Kensuke
Kadoma-shi, Osaka 571-0057 (JP)**

(74) Representative: **Novagraaf International SA
Chemin de l'Echo 3
1213 Onex, Geneva (CH)**

(54) **INFORMATION PROCESSING METHOD, INFORMATION PROCESSING DEVICE, AND PROGRAM**

(57)      An information processing method includes: obtaining material information (1) that is information relating to compounds and indicating, for each of the compounds, two or more elements making up the compound and information relating to a component ratio of the two or more elements (step S10); distinguishing an attribute of a group to which the compounds belong, based on the material information (1) (step S11); determining a display method for the material information (1) according to the attribute distinguished (step S12); and outputting the material information (1) according to the display method determined (step S14).

FIG. 12

**EP 4 425 503 A1**

**Description**

[Technical Field]

The present disclosure relates to a technique for displaying information regarding materials such as compounds, for example.

[Background Art]

Conventionally, in development of materials that are compounds, in order to search for a composition formula or processing conditions of a compound having a desired property, it is common practice to perform experiments while changing blending ratios of raw materials and the like to specify a composition formula or processing conditions having a favorable property. Here, Patent Literature (PTL) 1 to 3 disclose information processing methods for displaying a composition formula, and so on, of a compound when the composition formula, and so on, of a compound is presented.

PTL 1 discloses a method of performing cluster classification of a plurality of compounds based on property information of the plurality of compounds and displaying the plurality of compounds. PTL 2 discloses a method of displaying a non-linear map using a similarity among a plurality of compounds. PTL 3 discloses a method of switching between display methods of displaying features of any chemical substance based on an input by a user, a physical property, and the like.

[Citation List]

[Patent Literature]

[PTL 1] Japanese Unexamined Patent Application Publication No. 2006-318048
[PTL 2] Japanese Unexamined Patent Application Publication (Translation of PCT Application) No. 2001-503546
[PTL 3] Japanese Unexamined Patent Application Publication No.

[Summary of Invention]

[Technical Problem]

However, with the methods in each of the patent literature described above, it is difficult to appropriately support raw material development.

The present disclosure is intended to solve the aforementioned problem and provides an information processing method, and so on, capable of appropriately supporting material development.

[Solution to Problem]

In order to solve the aforementioned problem, an information processing method according to an aspect of the present disclosure is an information processing method performed by a computer, the information processing method including: obtaining material information that is information relating to compounds and indicating, for each of the compounds, two or more elements making up the compound and information relating to a component ratio of the two or more elements in the compound; distinguishing an attribute of a group to which the compounds belong, based on the material information; determining a display method for the material information according to the attribute distinguished; and executing an outputting process of outputting the material information according to the display method determined.

It should be noted that these general or specific aspects may be implemented as a system, an integrated circuit, a computer-readable recoding medium such as a CD-ROM, and may be implemented as any combination of a device, a system, a method, an integrated circuit, a computer program, and a recording medium. Furthermore, the recording medium may be a non-transitory recording medium.

[Advantageous Effects of Invention]

According to the present disclosure, material development can be appropriately supported.

Additional benefits and advantages of an aspect of the present disclosure will become apparent from the specification and drawings. The benefits and/or advantages may be individually obtained by the various embodiments and features of the specification and drawings, which need not all be provided in order to obtain one or more of such benefits and/or advantages.

[Brief Description of Drawings]

[FIG. 1]

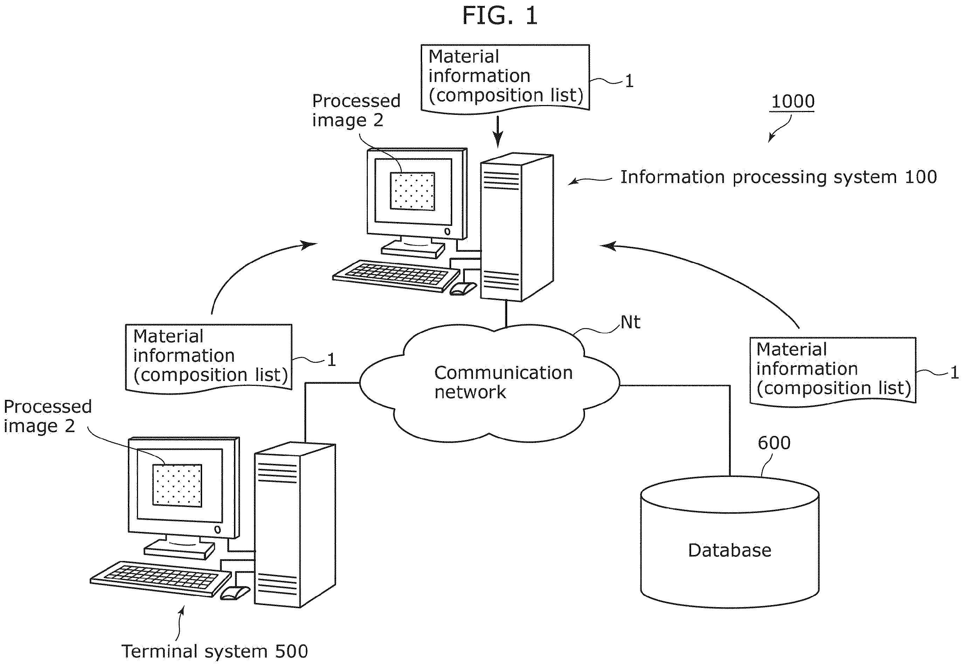

FIG. 1 is a diagram illustrating an example of a configuration of a processing system according to Embodiment 1.
[FIG. 2A]

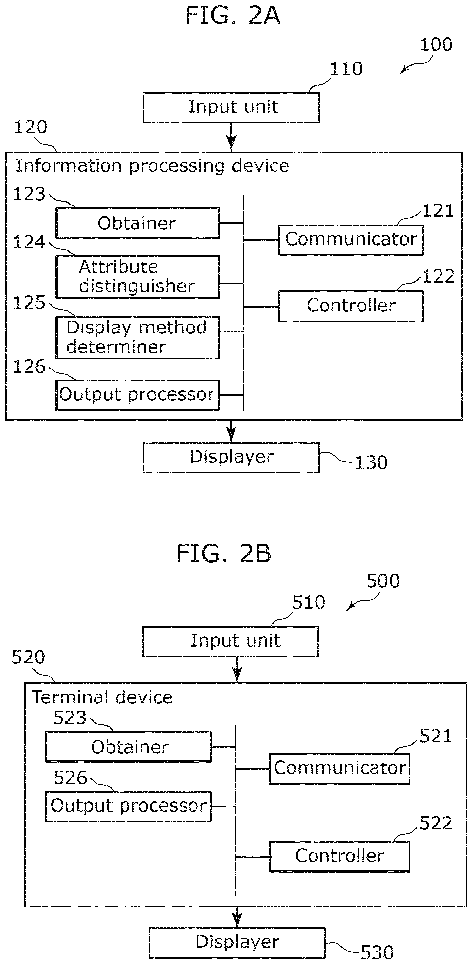

FIG. 2A is a diagram illustrating an example of a configuration of an information processing system according to Embodiment 1.
[FIG. 2B]
FIG. 2B is a diagram illustrating an example of a configuration of a terminal system according to Embodiment 1.
[FIG. 3A]

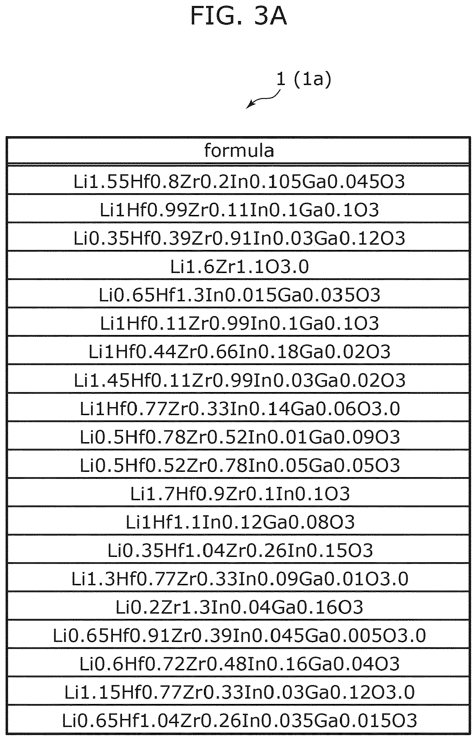

FIG. 3A is a diagram illustrating an example of a composition list according to Embodiment 1.
[FIG. 3B]

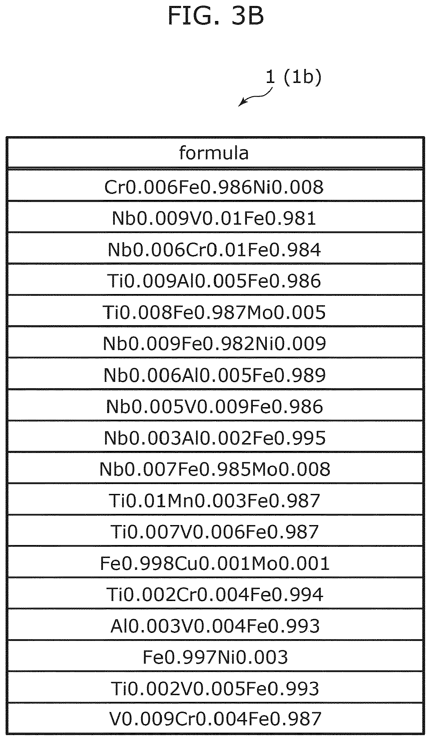

FIG. 3B is a diagram illustrating another example of a composition list according to Embodiment 1.
[FIG. 3C]

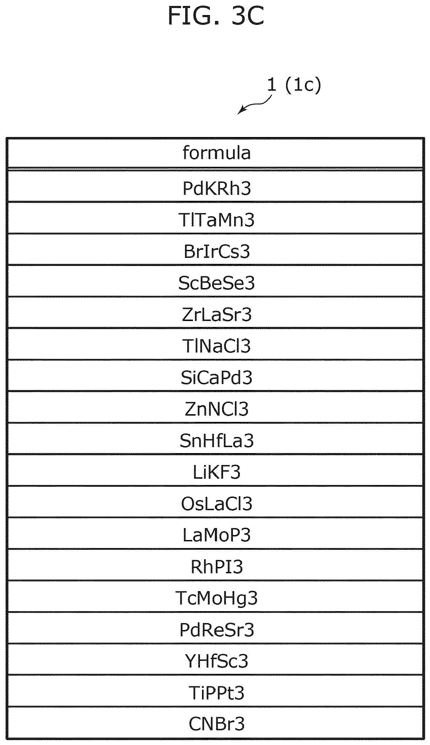

FIG. 3C is a diagram illustrating yet another example of a composition list according to Embodiment 1.
[FIG. 3D]

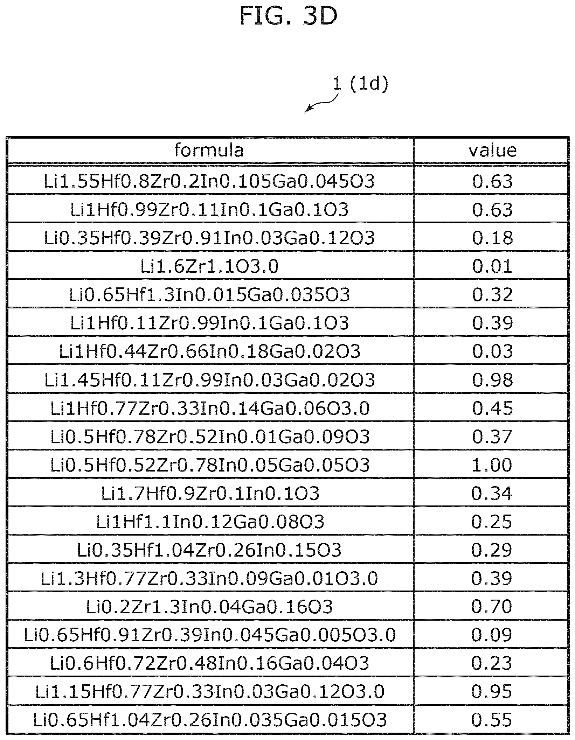

FIG. 3D is a diagram illustrating yet another example of a composition list according to Embodiment 1.
[FIG. 4A]

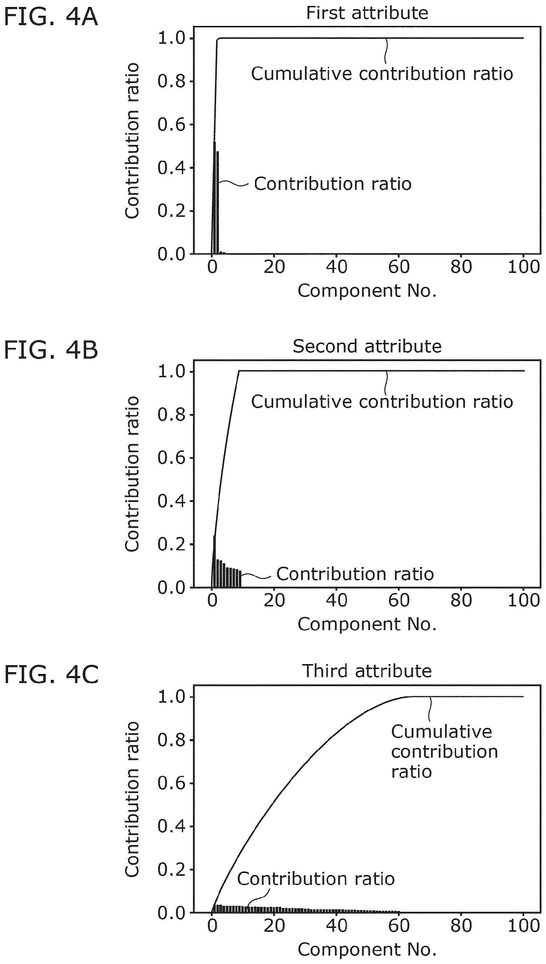

FIG. 4A is a diagram illustrating an example of a result of a principal component analysis according to Embodiment 1.
[FIG. 4B]
FIG. 4B is a diagram illustrating another example of a result of a principal component analysis according to Embodiment 1.
[FIG. 4C]
FIG. 4C is a diagram illustrating yet another example of a result of a principal component analysis according to Embodiment 1.
[FIG. 5]

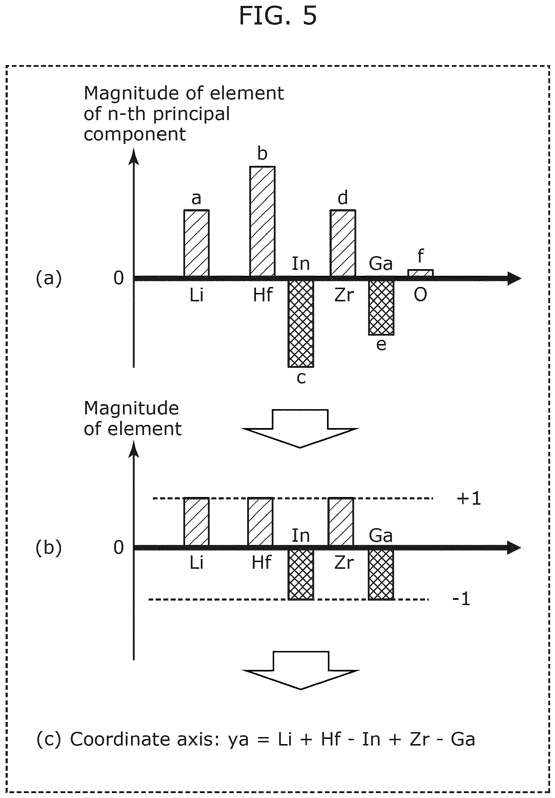

FIG. 5 is a diagram for describing a method of determining an abscissa variable and an ordinate variable used for an abscissa axis and an ordinate axis of a graph according to a first display method according to Embodiment 1.
[FIG. 6A]

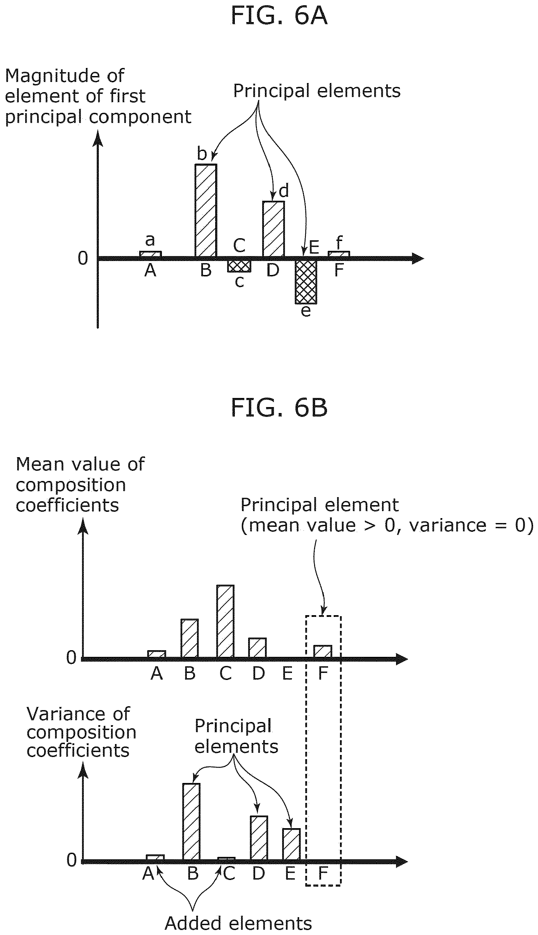

FIG. 6A is a diagram for describing a part of a method of determining an added element according to Embodiment 1.
[FIG. 6B]
FIG. 6B is a diagram for describing another part of the method of determining an added element according to Embodiment 1.
[FIG. 7A]

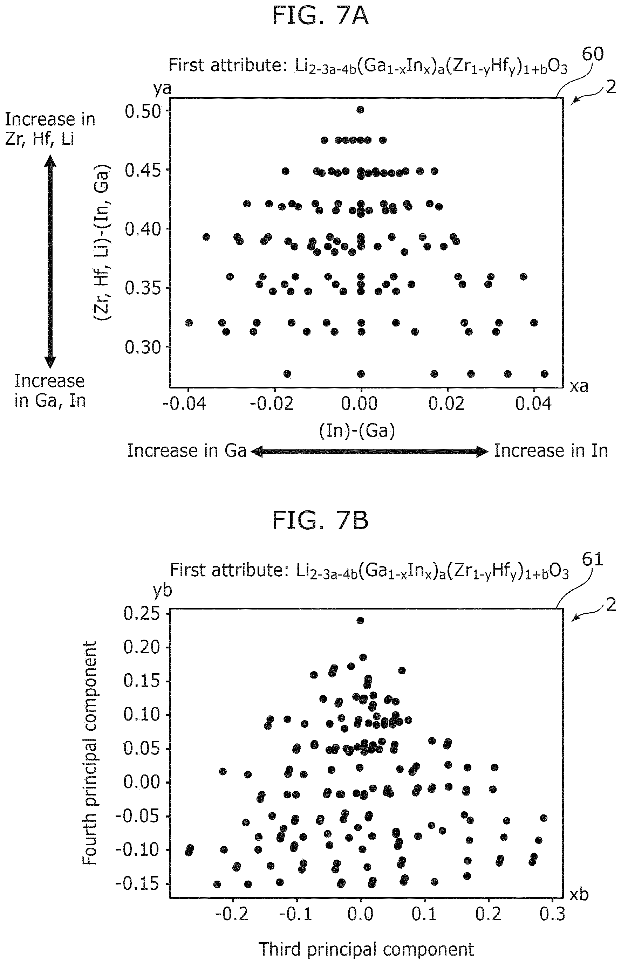

FIG. 7A is a diagram illustrating an example of a processed image generated with respect to a composition list of a first attribute according to Embodiment 1.
[FIG. 7B]
FIG. 7B is a diagram illustrating another example of a processed image generated with respect to a composition list of a first attribute according to Embodiment 1.
[FIG. 8A]

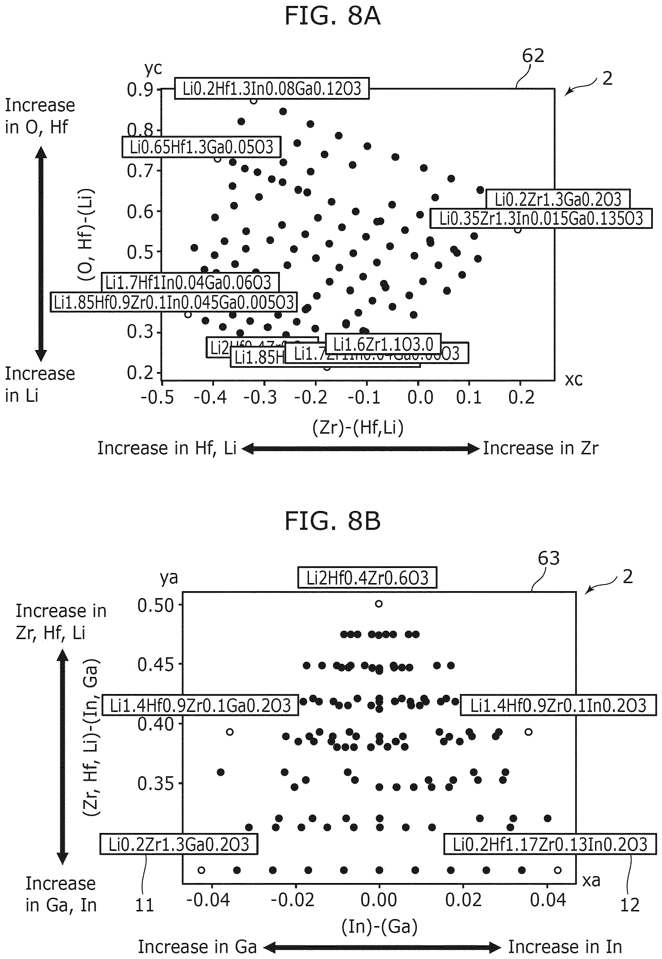

FIG. 8A is a diagram illustrating yet another example of a processed image generated with respect to a composition list of the first attribute according to Embodiment 1.
[FIG. 8B]
FIG. 8B is a diagram illustrating yet another example of a processed image generated with respect to a composition list of the first attribute according to Embodiment 1.
[FIG. 9]

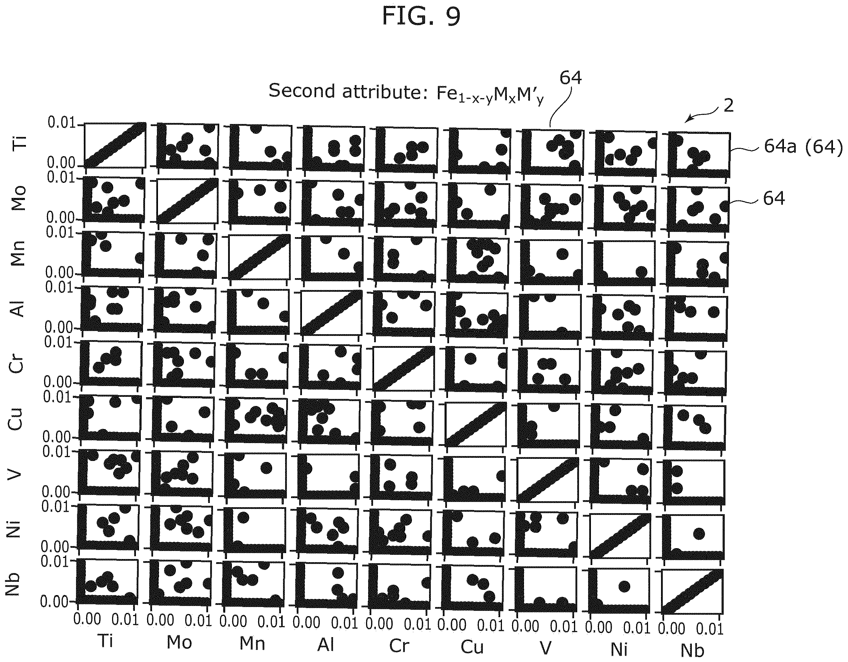

FIG. 9 is a diagram illustrating an example of a processed image generated with respect to a composition list of a second attribute according to Embodiment 1.
[FIG. 10A]

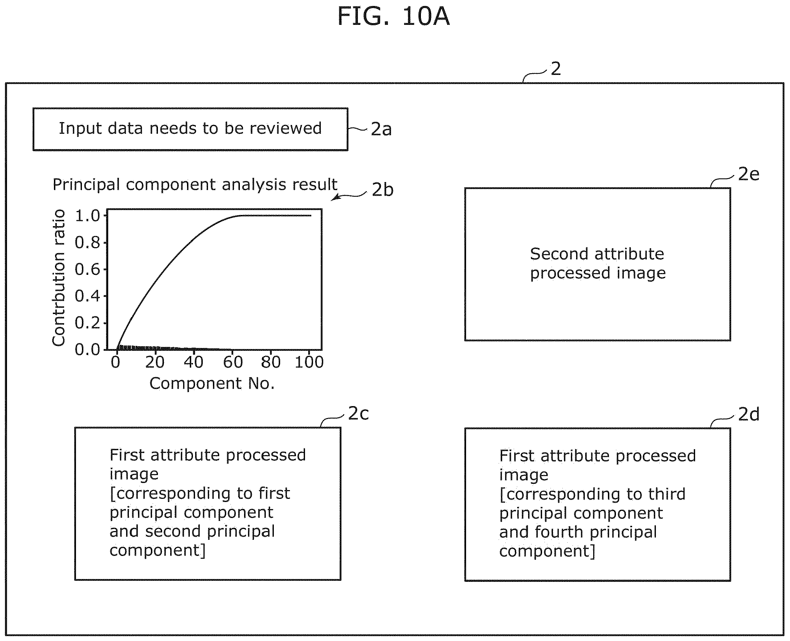

FIG. 10A is a diagram illustrating an example of a processed image generated with respect to a composition list of a third attribute according to Embodiment 1.
[FIG. 10B]

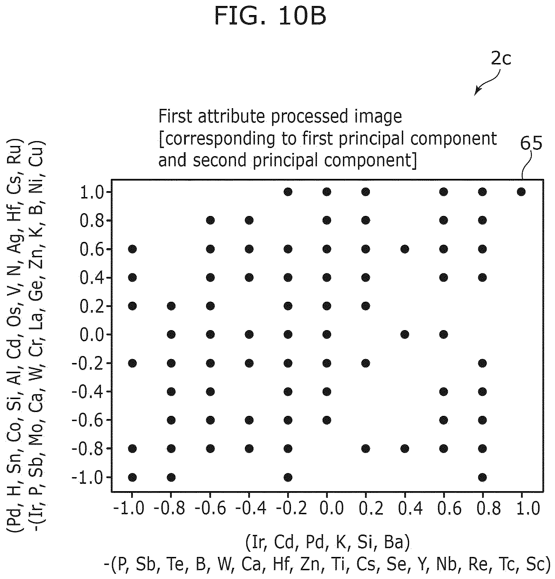

FIG. 10B is a diagram illustrating, in an enlarged manner, a part of the processed image generated with respect to the composition list of the third attribute according to Embodiment 1.

[FIG. 10C]

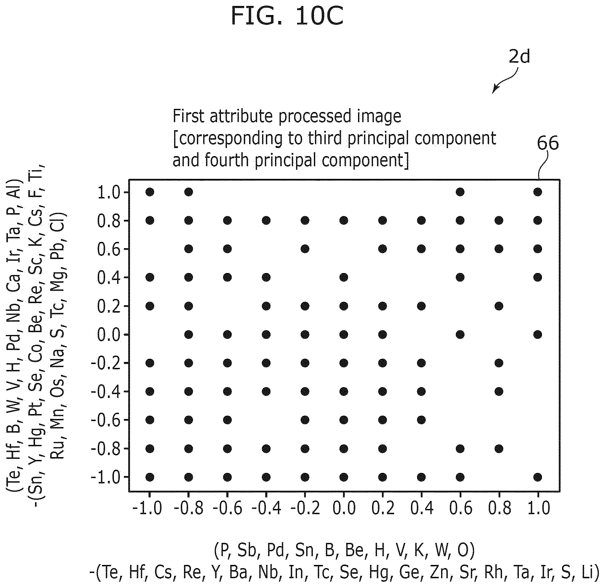

FIG. 10C is a diagram illustrating, in an enlarged manner, another part of the processed image generated with respect to the composition list of the third attribute according to Embodiment 1.

[FIG. 10D]

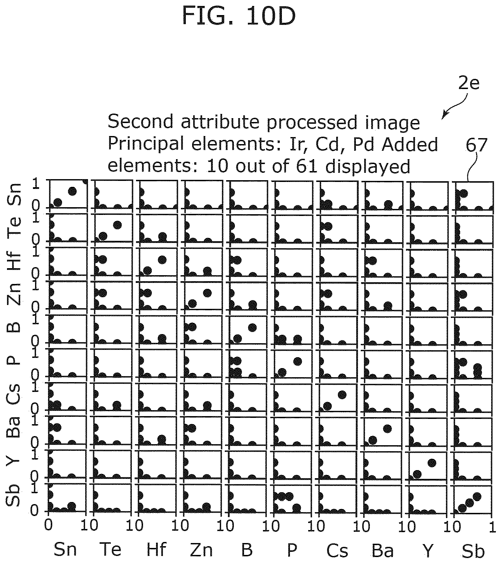

FIG. 10D is a diagram illustrating, in an enlarged manner, another part of the processed image generated with respect to the composition list of the third attribute according to Embodiment 1.

[FIG. 11]

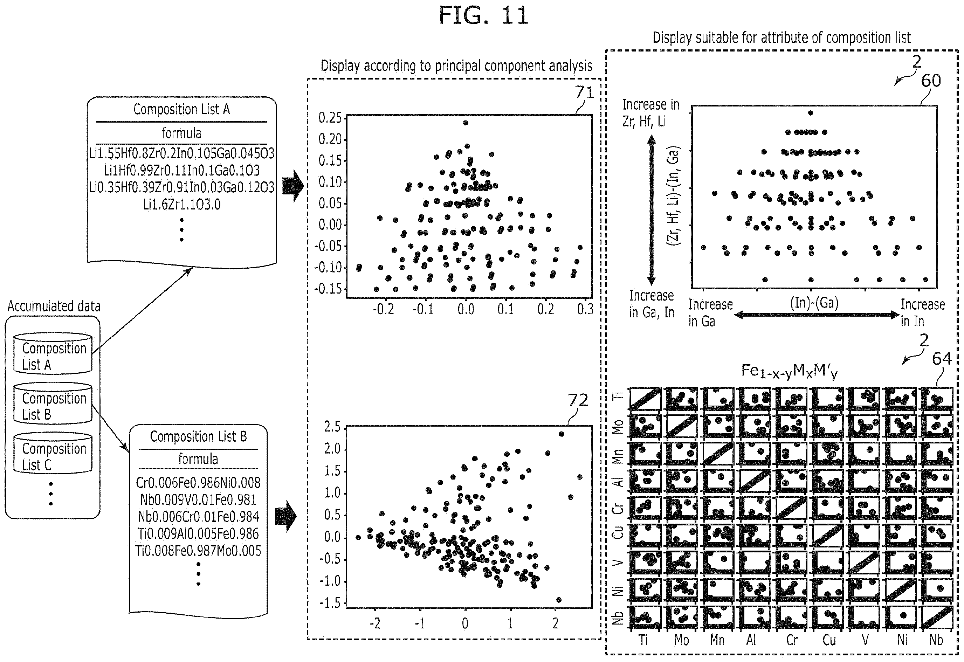

FIG. 11 is a diagram for describing an effect of the information processing device according to Embodiment 1.

[FIG. 12]

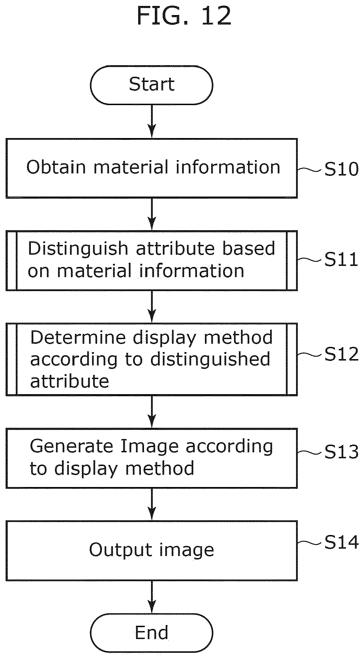

FIG. 12 is a flowchart illustrating an example of the processing operations of the information processing device according to Embodiment 1.

[FIG. 13A]

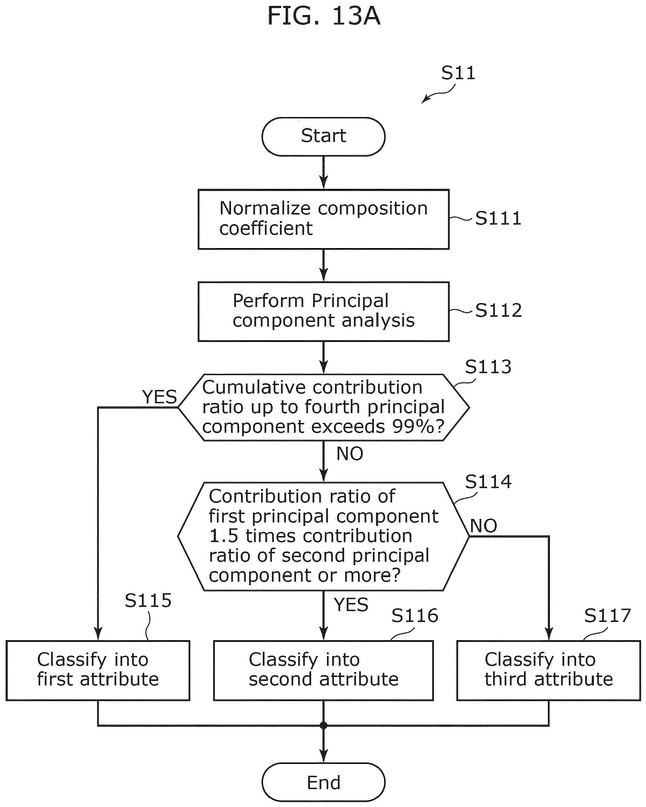

FIG. 13A is a flowchart illustrating an example of processing operations of an attribute distinguisher according to Embodiment 1.

[FIG. 13B]

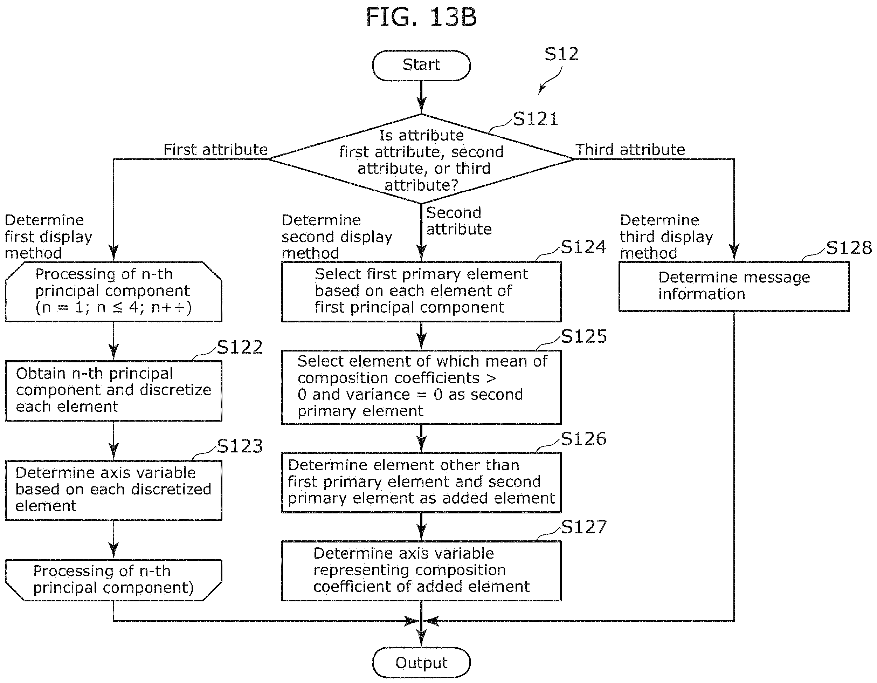

FIG. 13B is a flowchart illustrating an example of processing operations of a display method determiner according to Embodiment 1.

[FIG. 14]

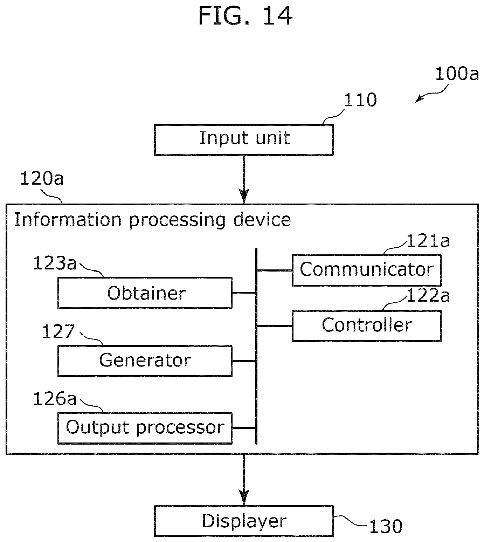

FIG. 14 is a diagram illustrating an example of a configuration of an information processing system according to Embodiment 2.

[FIG. 15A]

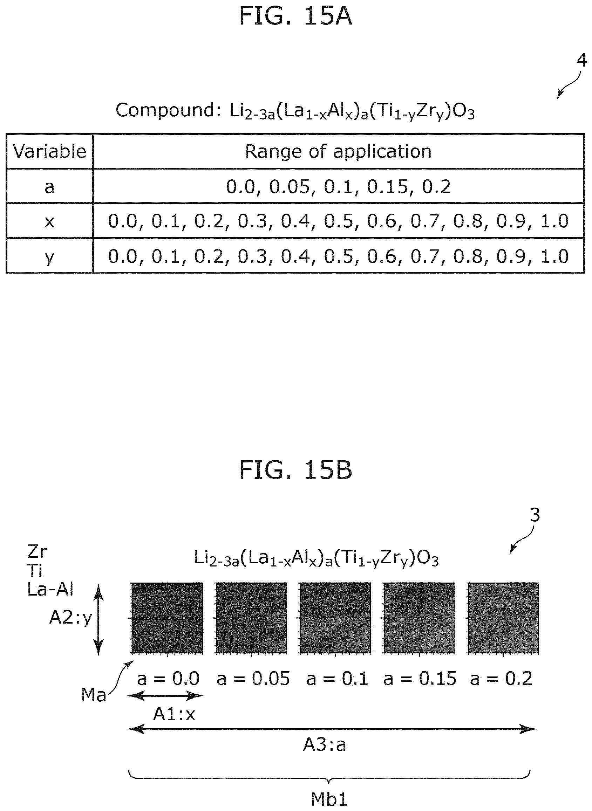

FIG. 15A is a diagram illustrating an example of variable information according to Embodiment 2.

[FIG. 15B]

FIG. 15B is a diagram illustrating an example of a processed image according to Embodiment 2.

[FIG. 16A]

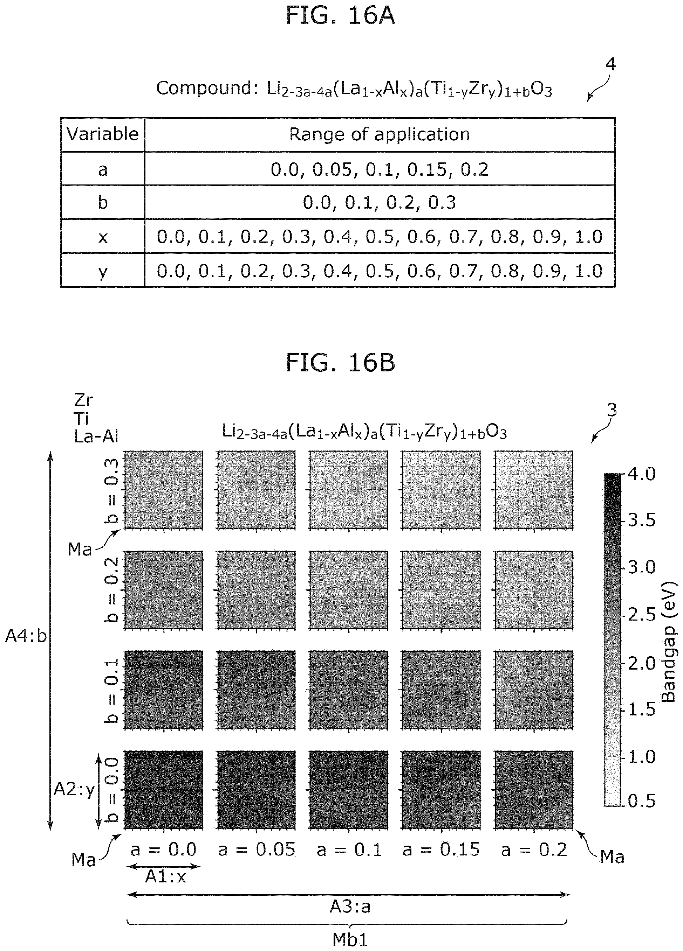

FIG. 16A is a diagram illustrating an example of variable information according to Embodiment 2.

[FIG. 16B]

FIG. 16B is a diagram illustrating another example of a processed image according to Embodiment 2.

[FIG. 16C]

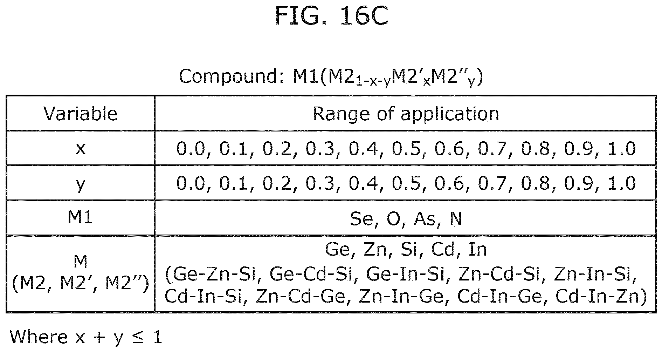

FIG. 16C is a diagram illustrating yet another example of variable information according to Embodiment 2.

[FIG. 16D]

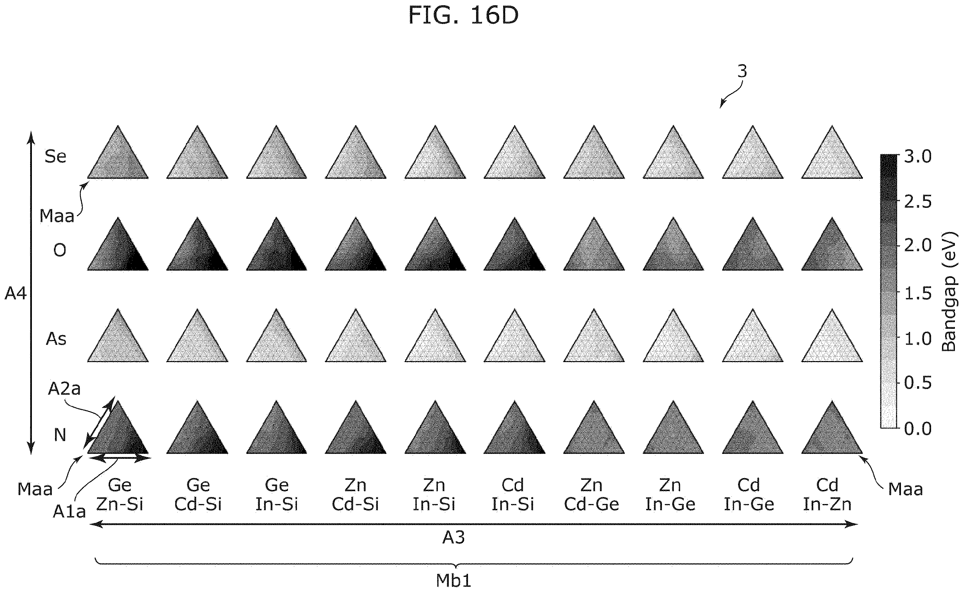

FIG. 16D is a diagram illustrating yet another example of a processed image according to Embodiment 2.

[FIG. 17A]

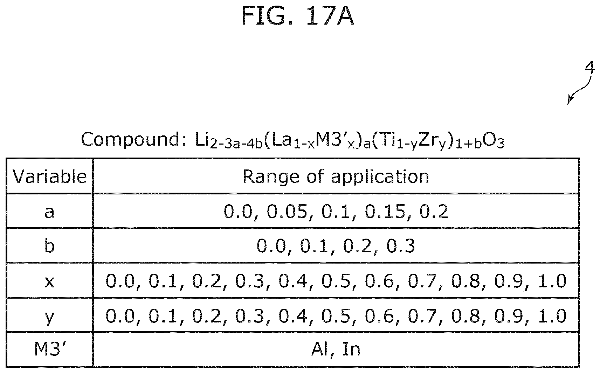

FIG. 17A is a diagram illustrating yet another example of variable information according to Embodiment 2.

[FIG. 17B]

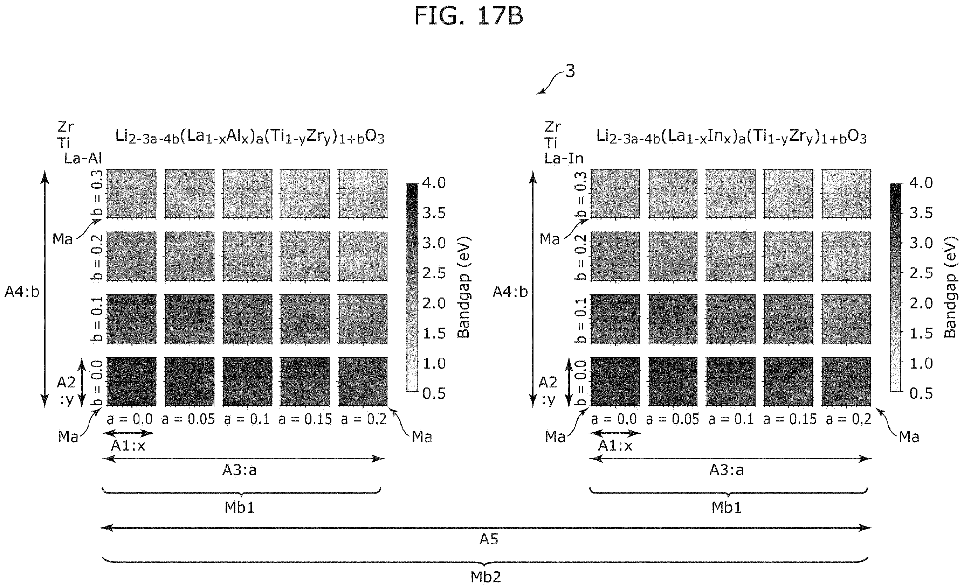

FIG. 17B is a diagram illustrating yet another example of a processed image according to Embodiment 2.

[FIG. 18A]

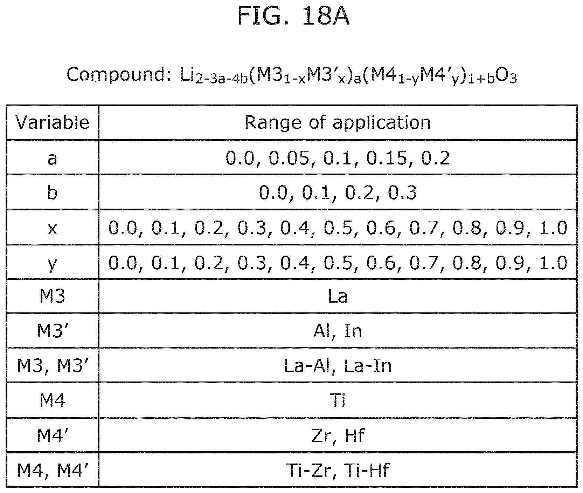

FIG. 18A is a diagram illustrating yet another example of variable information according to Embodiment 2.

[FIG. 18B]

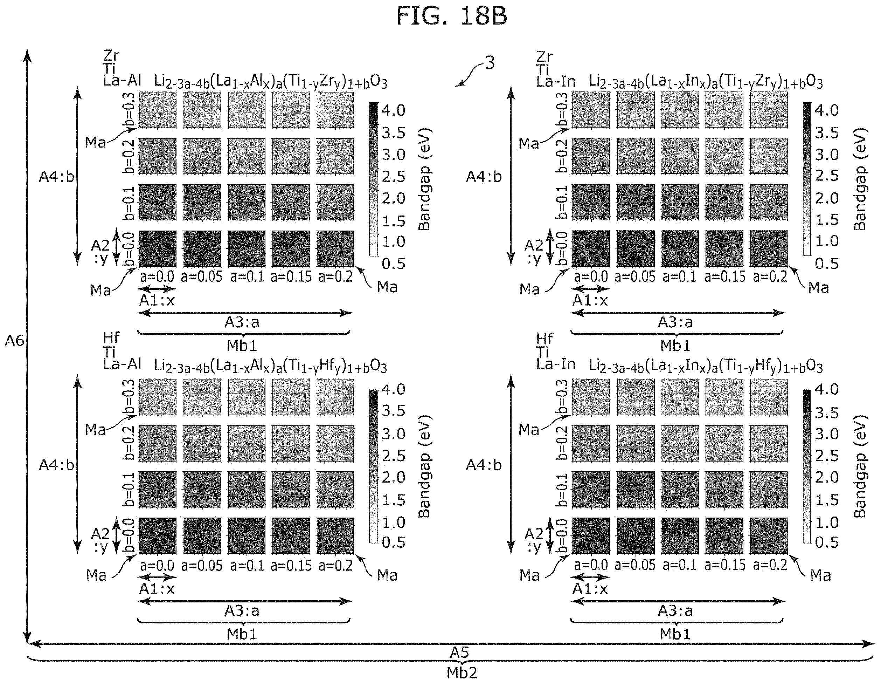

FIG. 18B is a diagram illustrating yet another example of a processed image according to Embodiment 2.

[FIG. 19]

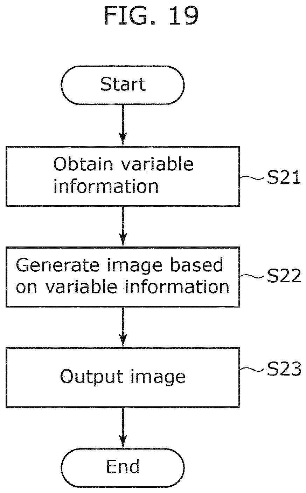

FIG. 19 is a flowchart illustrating an example of processing operations of an information processing device according Embodiment 2.

[Description of Embodiments]

(Underlying Knowledge Leading to Present Disclosure)

In recent years, recognition and identification methods based on machine learning and the like have made remarkable progress in fields such as image recognition and natural language processing, and are beginning to be applied to prediction of material properties. Accordingly, the number of pieces of data to be handled has become enormous and a method of appropriately displaying and understanding a large number of pieces of amassed data is being sought.

For example, when a small number of pieces of data are obtained by changing a plurality of elements and a mixing ratio of the elements, a user can observe the data and appropriately select a display method of the data based on the mixing ratio. On the other hand, amassing past experimental data in order to secure a large number of pieces of data may result in a mixture of pieces of data by different experimenters and pieces of data of experiments with different intentions. Therefore, in such a case, the user may find it difficult to appropriately select a display method of the data.

In addition, a display method for getting an overview of all of the pieces of data is also not self-evident.

On the other hand, while there are display methods of big data, an attribute of data which is based on an element and a component ratio of the element and which is important in material development is not taken into consideration. Examples of the attribute of data include an attribute of a group to which a compound obtained by changing a mixing ratio of a plurality of elements belongs and an attribute of a group to which a compound obtained by adding a small amount of an element to a compound with a given composition belongs. In other words, conventional information processing methods are unable to distinguish an attribute of data and determine a display method according to the result of the distinguishing.

For example, PTL 1 discloses a method of performing cluster classification of a plurality of compounds based on a property value of each of the plurality of compounds and displaying a recursive nested structure on a two-dimensional plane. However, with this method, it is difficult to comprehend how elements contained in one of the plurality of compounds and component ratios of the elements differ from elements contained in other compounds and component ratios of the elements. In addition, a method of distinguishing an attribute of data and selecting an appropriate display method according to a distinguishing result is not disclosed.

In addition, PTL 2 discloses a method of performing non-linear mapping based on a similarity between organic molecules and displaying a result of the non-linear mapping on a two-dimensional plane. However, a display method based on an element and a component ratio of the element is not disclosed. Alternatively, a method of distinguishing an attribute of data and selecting an appropriate display method according to a distinguishing result is not disclosed.

Furthermore, PTL 3 discloses a method of switching between display methods of a chemical substance based on an input by a user and a physical property. However, a display method based on an element and a component ratio of the element is not disclosed.

As described above, an appropriate display method based on an element and a component ratio thereof is not selected in the information processing methods used in each of the patent literature described above. The patent literature also does not disclose a method of distinguishing an attribute of data and selecting an appropriate display method. As a result, support of material development is inadequate.

In view of such circumstances, the inventors have found an information processing method of distinguishing attributes of data which is material information, and automatically determining a display method that is in accordance with the attribute.

In view of this, an information processing method according to an aspect of the present disclosure is an information processing method performed by a computer and includes: obtaining material information that is information relating to compounds and indicating, for each of the compounds, two or more elements making up the compound and information relating to a component ratio of the two or more elements in the compound; distinguishing an attribute of a group to which the compounds belong, based on the material information; determining a display method for the material information according to the attribute distinguished; and executing an outputting process of outputting the material information according to the display method determined.

Accordingly, an attribute of a group to which a plurality of compounds belong is distinguished based on two or more elements of each of the plurality of compounds and a component ratio of the two or more elements and material information is outputted according to a display method appropriate to the attribute. Therefore, the two or more elements of each of the plurality of compounds and a component ratio of the two or more elements can be output and displayed in a mode appropriate for the plurality of compounds. As a result, an appropriate display of material information can be automatically obtained and efficiency of a material search of searching for a novel material from the plurality of compounds can be improved. In other words, material development can be appropriately supported. For example, by analyzing the two or more elements of each of the plurality of compounds and a component ratio of the two or more elements shown in the material information, the attribute of the plurality of compounds can be distinguished and the material information can be displayed in a mode suitable for the attribute. In this case, for example, when a component ratio of the two or more elements is considered a vector, the attribute of the plurality of compounds is distinguished by a degree of freedom of the plurality of compounds as a whole. More specifically, a principal component analysis may be performed with respect to a vector of a component ratio of two or more elements and the number of principal components that exceed 99% in a cumulative contribution ratio may be used as the degree of freedom. Note that while material information may be information relating to a plurality of compounds used in an experiment for a material search, material information is not limited thereto and may be information relating to an untested compound or information relating to a compound for uses other than an experiment. For example, material information may be information relating to a compound used in a numerical calculation or information relating to a compound obtained by machine learning.

Furthermore, in the executing of the outputting process, the outputting process may be performed by generating an image showing, for each of the compounds, coordinates derived, according to the display method, from at least one of (i) the two or more elements making up the compound or (ii) the component ratio of the two or more elements in the compound, and outputting the image.

Accordingly, a coordinate of each of the plurality of compounds is derived from at least one of the two or more elements constituting the compound and the component ratio of the two or more elements and an image showing the coordinate of each of the plurality of compounds is displayed. For example, the coordinates are indicated by coordinate points on a graph included in the image. In addition, the coordinates are derived according to a determined display method. Therefore, a distribution of respective coordinate points of the plurality of compounds can be displayed in an appropriate mode in accordance with an attribute of a group to which the plurality of compounds belong. As a result, for example, a searcher who performs a material search can automatically obtain a display result in accordance with the attribute of the plurality of compounds. In other words, even when there are two or more groups of the plurality of compounds and attributes of the groups differ from each other, the searcher can automatically obtain, with respect to each of the two or more groups, a display result appropriate to the group. Furthermore, a distribution of the plurality of compounds can be readily comprehended from a higher perspective and efficiency of material search can be improved.

Furthermore, in the distinguishing of the attribute, the attribute may be distinguished based on a degree of freedom regarding compositions of the compounds, the compositions being identified from the material information.

Accordingly, an attribute of a group to which a plurality of compounds belong is distinguished based on a distribution of the compounds in a space for expressing a composition of the plurality of compounds and material information is outputted according to a display method appropriate to the distribution. Therefore, the two or more elements of each of the plurality of compounds and a component ratio of the two or more elements can be displayed in a readily understood manner from the perspective of a distribution of the plurality of compounds and efficiency of a material search of searching for a novel material from the plurality of compounds can be improved.

Furthermore, in the distinguishing of the attribute: the attribute may be distinguished as a first attribute when the degree of freedom is less than or equal to a first threshold; the attribute may be distinguished as a second attribute when the degree of freedom is greater than the first threshold and less than or equal to a second threshold; and the attribute may be distinguished as a third attribute when the degree of freedom is greater than the second threshold. The second threshold may be a value greater than the first threshold.

Accordingly, since the attribute of a group is classified into the first attribute, the second attribute, or the third attribute depending on the magnitude of the degree of freedom, the appropriate display method that is in accordance with the distribution of the compounds can be determined from among three types of display methods.

Furthermore, the determining of the display method may include: for each of independent variables, determining, as a coordinate axis included in the image, a coordinate axis indicating the independent variable, a total number of the independent variables being dependent on the degree of freedom. In the executing of the outputting process, the outputting process may be performed by generating the image including the coordinate axis determined, and outputting the image.

Accordingly, for example, when the degree of freedom is four, with respect to each of four independent variables, a coordinate axis representing the independent variable is determined as a coordinate axis of an image showing a coordinate of each of the plurality of compounds. Therefore, the two or more elements of each of the plurality of compounds and a component ratio of the two or more elements can be displayed in a readily understood manner using coordinate axes with the number corresponding to the degree of freedom.

Furthermore, the information processing method may further include: deriving, as the degree of freedom, a total number of principal components having a cumulative contribution ratio exceeding 99 percent, by performing a principal component analysis on the compounds.

Accordingly, the degree of freedom can be appropriately derived.

Furthermore, in the distinguishing of the attribute, the attribute may be distinguished as the second attribute when the degree of freedom is greater than the first threshold and less than or equal to the second threshold and a contribution ratio of a first principal component obtained by performing a principal component analysis on the compounds is at least 1.5 times a contribution ratio of a second principal component.

Accordingly, since the attribute of the group is distinguished as the second attribute when the contribution ratio of the first principal component is 1.5 times of the contribution ratio of the second principal component or more, an attribute of a group to which a plurality of compounds respectively containing a dopant can be distinguished as the second attribute. Therefore, material information can be displayed in a readily understood manner according to a display method suitable for a compound containing a dopant. In other words, a composition coefficient of an element corresponding to the dopant can be displayed in a readily understood manner.

Furthermore, in the distinguishing of the attribute: the attribute may be distinguished as a first attribute when each of the compounds is a solid solution; the attribute may be distinguished as a second attribute when each of the compounds includes a dopant; and the attribute may be distinguished as a third attribute when each of the compounds is not a solid solution and does not include a dopant. It should be noted that a solid solution is a compound obtained when two or more types of elements melt together and the entirety is in a uniform solid phase. Furthermore, in the present disclosure, a compound that is filled with element Li is treated as a solid solution.

Accordingly, when the plurality of compounds are a solid solution, material information can be displayed in a readily understood manner according to a display method suitable for the solid solution. In other words, a magnitude relationship or a component ratio of each of the two or more elements contained in the solid solution can be displayed in a readily understood manner. Furthermore, when the plurality of compounds contain a dopant, material information can be displayed in a readily understood manner according to a display method suitable for a compound containing a dopant. In other words, a composition coefficient of an element with respect to the dopant can be displayed in a readily understood manner. Moreover, when the plurality of compounds are not a solid solution and do not contain a dopant, material information can be displayed according to, for example, an error notification-type display method.

Furthermore, in the distinguishing of the attribute: at least one type of feature for the compounds may be identified based on a variation in the component ratio of the two or more elements included in each of the compounds; the attribute may be distinguished as a first attribute when the at least one type of feature satisfies a first condition; the attribute may be distinguished as a second attribute when the at least one type of feature satisfies a second condition; and the attribute may be distinguished as a third attribute when the at least one type of feature satisfies a third condition.

Accordingly, an attribute of a group to which the plurality of compounds belong is classified into a first attribute, a second attribute, or a third attribute based on variability of a component ratio of the two or more elements. Therefore, a distribution of respective coordinate points of the plurality of compounds can be displayed by an appropriate display method in accordance with a tendency of the variability. In other words, when the plurality of compounds are to be used for a material search, a policy of the material search can be read from the material information and a distribution of respective coordinate points of the plurality of compounds can be displayed by a display method in accordance with the policy. As a result, material search can be made more efficient.

Furthermore, in the obtaining of the material information, for each of the compounds, the material information indicating a normalized component ratio of the two or more elements may be obtained. In the distinguishing of the attribute, a principal component analysis may be performed on the compounds using the normalized component ratio of the two or more elements, and the attribute may be distinguished as the first attribute, the second attribute, or the third attribute based on a result of the principal component analysis. In the determining of the display method, when the attribute is distinguished as the first attribute or the second attribute, the display method of the material information may be determined by determining, as the coordinate axis included in the image, a coordinate axis indicating variables obtained from the result of the principal component analysis.

Accordingly, an attribute of a group to which the plurality of compounds belong is classified into a first attribute, a second attribute, or a third attribute based on a result of a principal component analysis with respect to the plurality of compounds. As a result, the attribute can be appropriately classified. In addition, for example, when a characteristic result of the principal component analysis has been obtained with respect to the first attribute or the second attribute, the coordinate of each of the plurality of compounds is defined by a coordinate axis representing a variable obtained from the characteristic result of the principal component analysis. Therefore, in each of the first attribute and the second attribute, a distribution of respective coordinate points of the plurality of compounds can be displayed using an appropriate coordinate axis in accordance with the result of the principal component analysis. Accordingly, when the plurality of compounds are to be used for a material search, a policy of the material search can be read from the material information and a distribution of respective coordinate points of the plurality of compounds can be displayed by a display method in accordance with the policy. As a result, material search can be made more efficient.

Furthermore, when the attribute is distinguished as the first attribute in the distinguishing of the attribute, in the determining of the display method: each of one or more coefficients to be used for an n-th principal component obtained in the principal component analysis is discretized, where n may be an integer greater than or equal to 1; and a coordinate axis indicating a variable represented based on the one or more coefficients discretized may be determined as the coordinate axis included in the image.

Accordingly, when the attribute of the group to which the plurality of compounds belong is the first attribute, for example, each of one or more nonzero coefficients used in an n-th principal component is discretized to -1 or 1. In addition, a variable used for a coordinate axis included in the image indicates, for example, a sum of composition coefficients of two or more elements, each of which having been multiplied by -1 or 1. Therefore, in this case, a magnitude relationship of composition coefficients and a component ratio of two or more elements contained in the compound can be readily read from coordinate points of the compound having been plotted according to the coordinate axis. As a result, material search can be made more efficient.

Furthermore, the executing of the outputting process may include: calculating a convex hull for the coordinates of each of the compounds; superimposing, on the image, composition information indicating a composition of a compound corresponding to a vertex of the convex hull calculated; and outputting the image on which the composition information is superimposed. For example, the composition information may be a composition formula.

Accordingly, a distribution of respective coordinate points of the plurality of compounds shown in the image and compositions of the compounds can be readily comprehended from the image. In other words, when composition information is superimposed on every one of the compounds, a plurality of pieces of composition information may overlap with each other or a coordinate point may become hidden by composition information and, in such cases, both a distribution of coordinate points and compositions of compounds become difficult to understand. However, in an aspect of the present disclosure, since the composition information of a compound corresponding to a vertex of a convex hull is superimposed but the composition information of other compounds is not superimposed, overlapping of a plurality of pieces of composition information and concealment of coordinate points by composition information can be suppressed. As a result, a distribution of coordinate points and a composition of each compound can be readily comprehended.

Furthermore, when the attribute is distinguished as the second attribute in the distinguishing of the attribute, in the determining of the display method: elements included in the compounds may be classified into one or more first elements and one or more second elements, based on one or more coefficients to be used for a first principal component obtained in the principal component analysis; at least one element from the one or more second elements may be identified as at least one added element; and, for each of the at least one added element, a coordinate axis indicating the at least one added element as a composition coefficient may be determined as the coordinate axis included in the image. In the first principal component, an absolute value of a coefficient of each of the one or more second elements may be smaller than an absolute value of a coefficient of each of the one or more first elements. Here, a variance of the composition coefficients of each of the at least one added element may be greater than 0. For example, a first element is referred to as a primary element or a first primary element. Furthermore, a second element is an element other than a first element among the plurality of elements.

Accordingly, for example, when the second attribute is distinguished with respect to a plurality of compounds, each containing an added element such as a dopant, respective coordinate points of the plurality of compounds are plotted on a graph having a coordinate axis representing a composition coefficient of the added element. Therefore, the presence of a plurality of compounds, which are slightly different in composition coefficients of the added element from each other, can be readily comprehended from an image or a graph having the coordinate axis. As a result, material search can be made more efficient.

Furthermore, an information processing method according to an aspect of the present disclosure is an information processing method performed by a computer and includes: obtaining variable information relating to k variables used for representing a composition of a compound, where k is an integer greater than or equal to 3 and less than or equal to 6; generating an image including an array map composed of an array of maps, by arraying the maps based on the variable information, the maps indicating information of compounds each having a composition represented by the k variables; and outputting the image. Here, the maps are each represented by a first coordinate axis indicating a first variable included in the k variables and a second coordinate axis indicating a second variable included in the k variables, and the maps included in the array map are arrayed according to a third variable other than the first variable and the second variable among the k variables.

Accordingly, at positions corresponding to the first variable, the second variable, and the third variable on the plurality of maps, information of a compound with a composition expressed by the three variables is mapped. The information of the compound is, for example, a property value of the compound. Therefore, property values and the like of a plurality of compounds having the composition expressed by the three variables can be readily comprehended from the plurality of maps. As a result, material search can be made more efficient, and material development can be appropriately supported.

Furthermore, in the generating of the image, the image including the array map may be generated by arraying the maps along a third coordinate axis indicating the third variable. The third coordinate axis may be parallel to the first coordinate axis or the second coordinate axis.

Accordingly, the plurality of maps classified according to the third variable are displayed arrayed in the direction of the first coordinate axis or the second coordinate axis. Therefore, a map corresponding to the third variable can be readily found and, furthermore, information on a compound having the composition expressed by the first variable, the second variable, and the third variable can be readily comprehended from the map.

Furthermore, the first variable, the second variable, and the third variable may each be a variable used for representing a composition coefficient of an element included in the compound, and a total number of the maps that are arrayed along the third coordinate axis in the generating of the image may be a value that can be assumed by the third variable.

Accordingly, since each of the first variable, the second variable, and the third variable is a variable used for expressing a composition coefficient of an element contained in a compound, information of a plurality of compounds having a composition coefficient expressed by the three variables can be readily comprehended from the plurality of maps. Note that the number of composition coefficients expressed by the three variables may be one or may be two or more.

Furthermore, in the generating of the image, the image including the array map may be generated by arraying the maps along a third coordinate axis indicating the third variable and a fourth coordinate axis indicating a fourth variable. Here, the fourth variable may be a variable other than the first variable, the second variable, and the third variable among the k variables, the third coordinate axis may be parallel to the first coordinate axis, and the fourth coordinate axis may be parallel to the second coordinate axis or orthogonal to the third coordinate axis.

Accordingly, the plurality of maps classified according to the third variable and the fourth variable are displayed arrayed in, for example, a matrix pattern, in the direction of the first coordinate axis and the second coordinate axis. Therefore, a map corresponding to the third variable and fourth variable can be readily found and, furthermore, information on a compound having the composition expressed by the first variable to the fourth variable can be readily comprehended from the map.

Furthermore, the first variable, the second variable, and the third variable, and the fourth variable may each be a variable used for representing a composition coefficient of an element included in the compound, and, a total number of the maps that are arrayed along the fourth coordinate axis in the generating of the image may be a value that can be assumed by the fourth variable.

Accordingly, since each of the first variable to the fourth variable is a variable used for expressing a composition coefficient of an element contained in a compound, the property values of a plurality of compounds having a composition coefficient expressed by the four variables can be readily comprehended from the plurality of maps. Note that the number of composition coefficients expressed by the four variables may be one or may be two or more.

Furthermore, a composition formula of each of the compounds may be represented by ApDqEr, where: A in the composition formula denotes at least one element; D in the composition formula is the third variable indicating an element; E in the composition formula is the fourth variable indicating an element; p in the composition formula is a composition coefficient of the at least one element indicated by A; q in the composition formula is the first variable indicating a composition coefficient of the element indicated by D; and r in the composition formula is the second variable indicating a composition coefficient of the element indicated by E. Here, a total number of maps arrayed along the third coordinate axis in the generating of the image may be equal to a total number of elements that can be assumed by the third variable, and a total number of maps arrayed along the fourth coordinate axis in the generating of the image may be a total number of elements that can be assumed by the fourth variable.

Accordingly, the first variable and the second variable are variables used for expressing a composition coefficient of an element contained in the compound, and the third variable and the fourth variable are variables used for expressing an element contained in the compound. Therefore, as many maps corresponding to combinations of elements that can be respectively assumed by the third variable and the fourth variable as the number of the combinations can be arrayed in, for example, a matrix pattern. In addition, a map corresponding to a combination of elements that can be respectively expressed by the third variable and the fourth variable can be readily found. In addition, information of a plurality of compounds having the composition expressed by the first variable to the fourth variable can be readily comprehended from the plurality of maps

Furthermore, in the generating of the image: for each compound group identified by a fifth variable, the array map may be generated for the compounds included in the compound group; and the image including a multi-array map composed of the array maps may be generated by arraying the array maps along a fifth coordinate axis indicated by the fifth variable. Here, the fifth variable may be a variable other than the first variable, the second variable, the third variable, and the fourth variable among the k variables, and the fifth coordinate axis may be parallel to one of the third coordinate axis or the fourth coordinate axis.

Accordingly, the plurality of array maps classified according to the fifth variable are displayed arrayed in the direction of the first coordinate axis or the second coordinate axis, that is, in the direction of the third coordinate axis or the fourth coordinate axis. Therefore, an array map corresponding to the fifth variable can be readily found and, furthermore, information on a compound having the composition expressed by the first variable to the fifth variable can be readily comprehended from the array map.

Furthermore, the fifth variable may indicate an element included in the compound, and, a total number of the array maps that are arrayed along the fifth coordinate axis in the generating of the image may be a total number of elements that can be assumed by the fifth variable.

Accordingly, since the fifth variable is a variable used for expressing an element contained in a compound, an array map corresponding to a plurality of compounds respectively having the element expressed by the fifth variable can be readily found from a plurality of array maps. In addition, information on a compound having the composition expressed by the first variable to the fifth variable can be readily comprehended from the array map.

Furthermore, in the generating of the image: for each compound group identified by a fifth variable and a sixth variable, the array map may be generated for the compounds included in the compound group; and the image including a multi-array map composed of the array maps may be generated by arraying the array maps along a fifth coordinate axis indicated by the fifth variable and a sixth coordinate axis indicated by the sixth variable. Here, the fifth variable may be one of two variables other than the first variable to the fourth variable among the k variables, the sixth variable may be a remaining one of the two variables, the fifth coordinate axis may be one of the third coordinate axis or the fourth coordinate axis, and the sixth coordinate axis may be a remaining one of the third coordinate axis or the fourth coordinate axis.

Accordingly, the plurality of array maps classified according to the fifth variable and the sixth variable are displayed arrayed in, for example, a matrix pattern, in the direction of the first coordinate axis and the second coordinate axis, that is, in the direction of the third coordinate axis and the fourth coordinate axis. Therefore, an array map corresponding to the fifth variable and the sixth variable can be readily found and, furthermore, information on a compound having the composition expressed by the first variable to the sixth variable can be readily comprehended from the array map.

Furthermore, the fifth variable may indicate an element included in the compound, the sixth variable may indicate an element included in the compound and different from the element indicated by the fifth variable, a total number of the array maps that are arrayed along the fifth coordinate axis in the generating of the image may be a total number of elements that can be assumed by the fifth variable, and a total number of the array maps that are arrayed along the sixth coordinate axis in the generating of the image may be a total number of elements that can be assumed by the sixth variable.

Accordingly, since each of the fifth variable and the sixth variable is a variable used for expressing an element contained in a compound, an array map corresponding to a plurality of compounds respectively having the element expressed by the fifth variable and the element expressed by the sixth variable can be readily found from a plurality of array maps. In addition, information on a compound having the composition expressed by the first variable to the sixth variable can be readily comprehended from the array map.

It should be noted that the respective steps included in the above-described information processing method are executed by a computer.

Hereinafter, embodiments of the present disclosure will be described with reference to the drawings. Each of the subsequently-described embodiments shows a preferred specific example of the present disclosure. Therefore, numerical values, shapes, materials, elements, the arrangement and connection of the elements, etc., indicated in the following embodiments are mere examples, and thus are not intended to limit the present disclosure. Therefore, among the elements described in the following embodiments, elements not recited in any one of the independent claims that indicate the broadest concepts are described as optional elements.

It should be noted that the respective figures are schematic illustrations and are not necessarily accurate depictions. Furthermore, elements which are substantially the same have the same reference signs in the figures, and duplicate description may be omitted or simplified. The effects of the information processing method are likewise realized even with the information processing device and the program.

[EMBODIMENT 1]

[Configuration of processing system 1000]

FIG. 1 is a diagram illustrating an example of a configuration of processing system 1000 according to the present embodiment.

Processing system 1000 according to the present embodiment is a system for processing information regarding materials, and includes information processing system 100, terminal system 500, and database 600.

Information processing system 100 is a computer system that determines a display method for material information 1 which is information regarding materials. It should be noted that materials are, for example, compounds, and more specifically, may be inorganic compounds (also referred to as inorganic materials). Information processing system 100 such as that described above is, for example, connected to terminal system 500 and database 600 via communication network Nt.

Terminal system 500 is a computer system that communicates with information processing system 100 via communication network Nt.

Database 600 is a recording medium that stores various kinds of information. Database 600 is a hard disk drive, a Random Access Memory (RAM), a Read Only Memory (ROM), a semiconductor memory, or the like. It should be noted that such database 600 may be volatile or non-volatile.

In processing system 1000 described above, for example, information processing system 100 obtains material information 1 that is information relating to a plurality of materials in response to an input operation by a user of information processing system 100. Material information 1 is, for example, a composition list indicating composition formulas of the plurality of materials. In addition, information processing system 100 determines a display method of material information 1 and generates and displays processed image 2 showing material information 1 according to the determined display method. Alternatively, information processing system 100 may transmit processed image 2 to terminal system 500 via communication network Nt and cause terminal system 500 to display processed image 2.

Alternatively, information processing system 100 obtains material information 1 via communication network Nt from terminal system 500. Even when information processing system 100 obtains material information 1 from terminal system 500, information processing system 100 determines a display method of material information 1 and displays processed image 2 showing material information 1 according to the determined display method in a similar manner to that described above. Alternatively, information processing system 100 may notify terminal system 500 via communication network Nt of the determined display method. In this case, terminal system 500 instead of information processing system 100 generates and displays processed image 2 showing material information 1 according to the notified display method.

Alternatively, when material information 1 is stored in database 600, information processing system 100 may read material information 1 via communication network Nt from database 600. Even when information processing system 100 reads material information 1 from database 600, information processing system 100 determines a display method of material information 1 and generates and displays processed image 2 showing material information 1 according to the determined display method in a similar manner to that described above. Alternatively, information processing system 100 may transmit display information indicating the determined display method and material information 1 to terminal system 500 via communication network Nt. In this case, terminal system 500 receives display information and material information 1 from information processing system 100. In addition, terminal system 500 generates and displays processed image 2 showing material information 1 according to the display method indicated by the display information.

Note that material information 1 transmitted and received among information processing system 100, terminal system 500, and database 600 may be anonymized or encrypted. Alternatively, a component ratio of two or more elements contained in a plurality of composition formulas shown in material information 1 may be normalized or subjected to another processing determined in advance. Accordingly, material information 1 can be transmitted and received in a state where a specific composition formula of each of the plurality of materials is concealed.

[Configuration of information processing system 100]

FIG. 2A is a diagram illustrating an example of a configuration of information processing system 100.

Information processing system includes input unit 110, information processing device 120, and displayer 130.

Input unit 110 receives an input operation by a user of information processing system 100 and outputs an input signal in accordance with the input operation to information processing device 120. Such input unit 110 is constituted of, for example, a keyboard, a touch sensor, a touchpad, or a mouse.

Information processing device 120 is a computer and includes communicator 121, controller 122, obtainer 123, attribute distinguisher 124, display method determiner 125, and output processor 126.

Communicator 121 has a communication function and communicates with terminal system 500 and database 600 via communication network Nt. Communication by communicator 121 may be wireless communication or wired communication. In addition, wireless communication may be performed using, for example, Wi-Fi (registered trademark), Bluetooth (registered trademark), ZigBee (registered trademark), or specified low-power radio. Note that types of wireless communication are not limited to the above.

Controller 122 controls communicator 121, obtainer 123, attribute distinguisher 124, display method determiner 125, and output processor 126.

Obtainer 123 obtains an input signal from input unit 110. In addition, obtainer 123 obtains a communication signal from terminal system 500 or database 600 via communicator 121. In this case, the input signal or the communication signal obtained by obtainer 123 is, for example, material information 1 described above. Material information 1 is information relating to a plurality of compounds and indicates, with respect to each of the plurality of compounds, contents relating to two or more elements constituting the compound and a component ratio of the two or more elements in the compound. In other words, obtainer 123 according to the present embodiment obtains material information 1.

Attribute distinguisher 124 distinguishes an attribute of a group to which a plurality of compounds belong based on material information 1 obtained by obtainer 123. Display method determiner 125 determines a display method of material information 1 according to the attribute distinguished by attribute distinguisher 124.

Output processor 126 performs an outputting process of outputting material information 1 according to the display method determined by display method determiner 125. Specifically, in the outputting process, output processor 126 generates processed image 2 showing material information 1 according to the determined display method and outputs processed image 2 as an image signal. For example, output processor 126 outputs the image signal to displayer 130. In this case, output processor 126 may transmit the image signal to terminal system 500 as a communication signal via communicator 121. In addition, output processor 126 may transmit display information indicating the display method determined by display method determiner 125 to terminal system 500 as a communication signal via communicator 121.

Displayer 130 obtains the image signal outputted from output processor 126 of information processing device 120 and displays processed image 2 according to the image signal.

Such information processing device 120 may be constituted of, for example, a processor such as a CPU (Central Processing Unit), a volatile memory and a non-volatile memory, and a program stored in the non-volatile memory. In this case, a functional configuration of information processing device 120 is realized as the processor executes the program.

[Configuration of terminal system 500]

FIG. 2B is a diagram illustrating an example of a configuration of terminal system 500.

Terminal system 500 includes input unit 510, terminal device 520, and displayer 530.

Input unit 510 receives an input operation by a user of terminal system 500 and outputs an input signal in accordance with the input operation to terminal device 520. Such input unit 510 is constituted of, for example, a keyboard, a touch sensor, a touchpad, or a mouse in a similar manner to input unit 110 described above.

Terminal device 520 is a computer and includes communication unit 521, controller 522, obtainer 523, and output processor 526.

Communicator 521 has a communication function and communicates with communicator 121 of information processing system 100 via communication network Nt. Communication by communicator 521 may be wireless communication or wired communication in a similar manner to communication by communicator 121. In addition, a type of wireless communication is not particularly limited.

Controller 522 controls communication unit 521, obtainer 523, and output processor 526.

Obtainer 523 obtains an input signal from input unit 510. In addition, obtainer 523 obtains a communication signal from communicator 121 of information processing system 100 via communicator 521. In this case, the input signal obtained by obtainer 523 is, for example, material information 1 described above. In addition, the communication signal obtained by obtainer 523 may be the display information, material information 1, or the like described above.

When the input signal obtained by obtainer 523 is material information 1, output processor 526 transmits the input signal as a communication signal to information processing device 120 of information processing system 100 via communicator 521. In addition, for example, when the communication signal obtained by obtainer 523 is display information, output processor 526 generates processed image 2 showing material information 1 according to the display method indicated by the display information and outputs processed image 2 as an image signal to displayer 530. Furthermore, for example, when the communication signal obtained by obtainer 523 is processed image 2 generated by information processing system 100, output processor 526 outputs processed image 2 as an image signal to displayer 530.

Displayer 530 obtains the image signal outputted from output processor 526 of terminal device 520 and displays processed image 2 according to the image signal. Accordingly, even when the user of terminal system 500 possesses a composition list indicating composition formulas of a plurality of materials and desires to conceal the specific composition formula of each of the plurality of materials shown on the composition list, an image for displaying an overall picture of the composition list can be obtained.

Like information processing device 120, such terminal device 520 may be constituted of, for example, a processor such as a CPU (Central Processing Unit), a volatile memory and a non-volatile memory, and a program stored in the non-volatile memory. In this case, a functional configuration of terminal device 520 is realized as the processor executes the program.

[Contents of processing of information processing device 120]

(Processing of obtainer 123)

For example, obtainer 123 of information processing device 120 obtains a composition list indicating a composition formula of each of a plurality of materials as material information 1 described above. Hereinafter, material information 1 may be represented as composition list 1.

Each of FIGS. 3A to 3D is a diagram illustrating an example of composition list 1.

Obtainer 123 of information processing device 120 obtains, for example, composition list 1 of any of FIGS. 3A to 3D. By showing a composition formula of each of a plurality of compounds, composition list 1 shows the plurality of compounds or, in other words, a group including the plurality of compounds. Each of the plurality of compounds shown by such composition list 1 is, for example, a compound used in an experiment for a search (in other words, a material search) for a novel material or a novel compound. In addition, a searcher who carries out a material search creates composition list 1 by, for example, organizing the plurality of compounds used in the experiment. Note that respective composition lists 1 illustrated in FIGS. 3A to 3D are merely an example and composition lists 1 may further include other composition formulas.

In this case, for example, an attribute of a group of the plurality of compounds shown by composition list 1 in FIG. 3A is distinguished as a first attribute by attribute distinguisher 124 of information processing device 120. Note that composition list 1 in FIG. 3A will also be hereinafter described as composition list 1a. In addition, an attribute of a group of the plurality of compounds shown by composition list 1 of FIG. 3B is distinguished as a second attribute by attribute distinguisher 124 of information processing device 120. Note that composition list 1 in FIG. 3B will also be hereinafter described as composition list 1b. Furthermore, an attribute of a group of the plurality of compounds shown by composition list 1 of FIG. 3C is distinguished as a third attribute by attribute distinguisher 124 of information processing device 120. Note that composition list 1 in FIG. 3C will also be hereinafter described as composition list 1c.

In addition, as illustrated in FIG. 3D, composition list 1 may show a property value of each of the plurality of compounds in association with a composition formula of the compound. Composition list 1 illustrated in FIG. 3D is a list indicating a property value of each of the plurality of compounds with respect to composition list 1a in FIG. 3A. For example, a property value may be a physical property value such as conductivity or density of the compound, a predicted value of a property of the compound obtained by machine learning, or a calculated value obtained by computational science. Note that composition list 1 in FIG. 3D will also be hereinafter described as composition list 1d.

Note that, in the present disclosure, a coefficient of each element included in a composition formula is denoted by subscripts in some cases and denoted by ordinary characters in a similar manner to elements in other cases. In addition, a coefficient of each element contained in a composition formula is also referred to as a composition coefficient. A ratio of the composition coefficients is also referred to as a component ratio or a composition ratio.

A plurality of compounds belonging to a group of the first attribute is expressed by, for example, (Expression 1) below. In the first attribute, each of the plurality of compounds is expressed by mutually differentiating a component ratio of a small number of elements among the plurality of compounds. For example, the component ratio of the small number of elements differs according to four variables a, b, x, and y as shown in (Expression 1) below.

$$Li_{2-3a-4b}(Ga_{1-x}In_x)_a(Zr_{1-y}Hf_y)_{1+b}O_3 \ldots \qquad \text{(Expression 1)}$$

A compound belonging to such a group of the first attribute may be described as being a solid solution or a solid solution type. A solid solution is, for example, a compound in which two or more elements dissolve into each other and which has a uniform solid phase as a whole. In addition, compounds filled with the element Li are also handled as solid solutions in the present disclosure. Note that the plurality of compounds belonging to a group of the first attribute may contain elements having a common characteristic among the plurality of compounds as shown in (Expression 1) above. The elements having a common characteristic may be a same element such as the element Li or the element 0 or homologous elements such as the element Ga and the element In or the element Zr and the element Hf.

A plurality of compounds belonging to a group of the second attribute are expressed by, for example, (Expression 2) below. Note that M and M' in (Expression 2) denote a variable indicating an arbitrary element and represent, for example, the element Mn, the element Cr, the element Mo, the element Ni, the element V, the element Al, the element Ti, or the element Cu. In the second attribute, the plurality of compounds are expressed by slightly changing a part of a component ratio of a plurality of elements (a component ratio of around ten elements at a maximum). In other words, in each of the plurality of compounds, one or more elements among the plurality of elements contained in the compound has a smaller composition coefficient than the other elements. For example, the variables x and y in (Expression 2) below are numerical values within a range of 0.001 to 0.009. Therefore, an element represented by the variables M and M' has a smaller composition coefficient represented by the variables x and y as compared to the element Fe.

$$Fe_{1-x-y}M_xM'_y \ldots \qquad \text{(Expression 2)}$$

A compound belonging to such a group of the second attribute may be described as being a compound containing a dopant or a dopant type. A dopant is, for example, an element represented by the variable M or M' in (Expression 2).

A plurality of compounds belonging to a group of the third attribute are expressed by, for example, (Expression 3) below. Note that A, B, and X in (Expression 3) are variables representing an arbitrary element and, for example, represent an element belonging to the first to third periods of the periodic table other with the exception of noble gases. The third attribute is an attribute which does not fall under the first attribute and the second attribute. For example, in the third attribute, a large number of mutually different elements are expressed by mutually different component ratios among the plurality of compounds.

$$ABX_3 \ldots \qquad \text{(Expression 3)}$$

A compound belonging to such a group of the third attribute may be described as being a multi-element combination type. In other words, a wide variety of elements are represented by the variables A, B, and X in (Expression 3) and there is no similarity or commonality of elements among the plurality of compounds.

Note that (Expression 1), (Expression 2), and (Expression 3) are expressions for the sake of convenience of description. Obtainer 123 obtains information relating to specific composition formulas such as composition lists 1a to 1d without obtaining information in which elements contained in a composition formula and composition coefficients of the elements are notated by variables as in (Expression 1), (Expression 2), and (Expression 3). In addition, obtainer 123 may obtain composition list 1, obtain anonymized or encrypted composition list 1, or obtain composition list 1 that has been normalized in a component ratio.

In this case, while composition list 1 being material information 1 according to the present embodiment indicates, for each of a plurality of compounds, two or more elements which constitute the compound and a component ratio of the two or more elements, the component ratio may be a ratio other than the component ratio contained in the composition formula.

In other words, in composition list 1, each of the plurality of compounds is indicated by a composition formula. A composition formula indicates a composition coefficient of each of a plurality of elements as a component ratio. Specifically, in composition list 1, $Li_{1.55}Hf_{0.8}Zr_{0.2}In_{0.105}Ga_{0.045}O_3$ is indicated as a composition formula. In addition, the component ratio in the composition formula is element Li: element Hf: element Zr: element In: element Ga = 1.55: 0.8: 0.2: 0.105: 0.045. However, the component ratio may be a blending ratio of raw materials of the compound. For example, raw materials of $Li_{1.55}Hf_{0.8}Zr_{0.2}In_{0.105}Gd_{0.045}O_3$ are, for example, respective oxides of the element Li, the element Hf, the element Zr, the element In, and the element Ga. Therefore, $Li_{1.55}Hf_{0.8}Zr_{0.2}In_{0.105}Ga_{0.045}O_3$ is expressed by (Expression 4) below. Note that variables a, b, c, d, and e in (Expression 4) represent blending amounts of the raw materials.

$$aLi_2O+bGa_2O_3+cIn_2O_3+dZrO_2+eHfO_2 \ldots \qquad \text{(Expression 4)}$$

Therefore, for example, composition list 1 may indicate the element Li, the element Hf, the element Zr, the element In, and the element Ga and a blending ratio "a: b: c: d: e" instead of the composition formula "$Li_{1.55}Hf_{0.8}Zr_{0.2}In_{0.105}Ga_{0.045}O_3$".

In addition, in the plurality of compounds included in composition list 1, for example, only composition coefficients of the element Li may mutually differ as in, for example, (Expression 5) below. By assigning mutually different values to variable x in (Expression 5) below, composition formulas of a plurality of compounds which only differ from each other in the composition coefficient of the element Li are expressed.

$$Li_{1.7-x}(Ga_{0.5}In_{0.5})_{0.1}Zr_{0.5}Hf_{0.5}O_3 \ldots \qquad \text{(Expression 5)}$$

Furthermore, a component ratio of a plurality of elements may be a ratio of respective percentages by mass of the plurality of elements in a compound. For example, the component ratio may be percentages by mass of elements respectively represented by the variables M and M' in (Expression 2). Specifically, the percentages by mass of the elements may range from 0% to 5%. In a specific example, two or more elements shown in composition list 1b and a component ratio of the two or more elements may be expressed as (Expression 6) below. Note that variables a and b in (Expression 6) denote percentages by mass. In addition, a percentage by mass of an element is converted into a composition coefficient of the element by dividing the percentage by mass of the element by an atomic weight of the element.

$$(100 - a - b)Fe + aM + bM' \ldots \text{(Expression 6)}$$

In addition, a component ratio of a plurality of elements indicated by a composition formula in composition list 1 may be normalized. For example, as in composition list 1b in FIG. 3B, the component ratio of a plurality of elements that constitute a compound is normalized so that a sum of composition coefficients of the plurality of elements equals one. In other words, obtainer 123 obtains composition list 1 which indicates, with respect to each of a plurality of compounds, a normalized component ratio of two or more elements.

(Processing of attribute distinguisher 124)

Based on composition list 1, attribute distinguisher 124 distinguishes an attribute of a group to which belong a plurality of compounds, each of which being represented by a composition formula in composition list 1. Specifically, for example, attribute distinguisher 124 first obtains composition list 1 from obtainer 123. At this point, when a component ratio of a plurality of elements represented by a composition formula of composition list 1 is not normalized, attribute distinguisher 124 may normalize the component ratio. For example, attribute distinguisher 124 may normalize any composition list 1 of composition lists 1a, 1c, and 1d illustrated in FIGS. 3A, 3C, and 3D.

Next, attribute distinguisher 124 performs a principal component analysis with respect to composition list 1. At this point, attribute distinguisher 124 counts the number of elements contained in any of the plurality of composition formulas shown in composition list 1. In other words, attribute distinguisher 124 counts the number (for example, g-number) of all of the elements contained in the plurality of composition formulas. In addition, with respect to each of the plurality of composition formulas shown in composition list 1, attribute distinguisher 124 converts the composition formula into a vector, the number of elements of which is the counted number or, in other words, a vector made up of g-number of vector elements. An i-th (where i is an integer of 1 or more and g or less) vector element of the vector represents a composition coefficient of an element corresponding to the i-th vector element. Next, attribute distinguisher 124 performs a principal component analysis with respect to the plurality of vectors. For example, with respect to composition list 1a in FIG. 3A, attribute distinguisher 124 converts a composition formula into a vector made up of vector elements corresponding to respective elements of the element Li, the element Hf, the element Zr, the element In, the element Ga, and the element 0 or, in other words, a vector made up of six vector elements. In a specific example, attribute distinguisher 124 converts a composition formula "$Li_{1.55}Hf_{0.8}Zr_{0.2}In_{0.105}Ga_{0.045}O_3$" into (1.55, 0.8, 0.2, 0.105, 0.045, 3). A vector element "1.55" corresponds to the element Li, a vector element "0.8" corresponds to the element Hf, a vector element "0.2" corresponds to the element Zr, a vector element "0.105" corresponds to the element In, a vector element "0.045" corresponds to the element Ga, and vector element "3" corresponds to the element O.

FIGS. 4A to 4C are diagrams illustrating an example of a result of a principal component analysis. Specifically, FIG. 4A illustrates a result of a principal component analysis with respect to composition list 1a. In other words, FIG. 4A illustrates a result of a principal component analysis with respect to a group of the first attribute. FIG. 4B illustrates a result of a principal component analysis with respect to composition list 1b. In other words, FIG. 4B illustrates a result of a principal component analysis with respect to a group of the second attribute. FIG. 4C illustrates a result of a principal component analysis with respect to composition list 1c. In other words, FIG. 4C illustrates a result of a principal component analysis with respect to a group of the third attribute. In addition, a result of the principal component analyses are illustrated as graphs in FIGS. 4A to 4C. An axis of abscissa of the graphs represents a principal component number of a plurality of principal components and an axis of ordinate represents a contribution ratio. Furthermore, a curved line or a broken line in the graphs represents a cumulative contribution ratio from a first principal component to an h-th principal component (where h is a principal component number of the axis of abscissa of the graphs) and a column in the graphs represents a contribution ratio of the h-th principal component. The principal component number h is an integer of 1 or more.

As illustrated in FIG. 4A, in a group of the first attribute, contribution ratios are biased toward highly ranked principal components. The highly ranked principal components include the first principal component and a second principal component. Such a trend in contribution ratios is observed when each of the plurality of compounds is a solid solution. In consideration thereof, when the cumulative contribution ratio from the first principal component to an n-th principal component exceeds a first threshold, attribute distinguisher 124 distinguishes that the attribute of the group is the first attribute. In this case, n is an integer of 1 or more and, for example, may be 4. The first threshold may be, for example, 0.99.

As described above, the plurality of compounds belonging to a group of the first attribute may contain elements having a common characteristic among the plurality of compounds. Therefore, when each of the plurality of compounds belonging to a group contains elements having a common characteristic among the plurality of compounds, attribute distinguisher 124 may distinguish the first attribute as the attribute of the group.

As illustrated in FIG. 4B, in a group of the second attribute, not only the first principal component to a fourth principal component but each of a fifth principal component and subsequent principal components has a nonzero contribution ratio. In addition, the contribution ratio of the first principal component is obviously larger than the contribution ratio of each of the second principal component and subsequent principal components. Such a trend in contribution ratios is observed when each of the plurality of compounds contains a dopant. In consideration thereof, when the contribution ratio of the first principal component is m-times (where m is a numerical value larger than 1) the contribution ratio of the second principal component or more, attribute distinguisher 124 distinguishes that the attribute of the group is the second attribute. In this case, m may be, for example, 1.5.

Note that a group of the second attribute is a set of compounds expressed by composition formulas containing a small amount of an added element (for example, a dopant). In addition, when the component ratio has been normalized or, in other words, when a ratio of composition coefficients has been normalized, in the plurality of compounds, a composition coefficient of primary elements other than the added element is always smaller than 1 by an amount corresponding to the composition coefficient of the added element. In other words, a variability in the composition coefficients of primary elements is created in accordance with a variability in the composition coefficient of the added element. As a result, the trend in contribution ratios illustrated in FIG. 4B is observed in a group of the second attribute. Note that a primary element is also referred to as a parent substance.

As illustrated in FIG. 4C, in a group of the third attribute, contribution ratios are not biased. In other words, a contribution ratio of highly ranked principal components is not obviously larger than the contribution ratio of other principal components and a difference between contribution ratios of the respective principal components is small. In consideration thereof, when an attribute of a group is not distinguished as the first attribute or the second attribute, attribute distinguisher 124 distinguishes the attribute of the group to be the third attribute.

In a group of the third attribute, each of the plurality of compounds tends to contain many elements that are not contained in other compounds. In other words, arrangements of nonzero vector elements significantly differ among a plurality of vectors that are expressed with respect to a group of the third attribute. As a result, as described above, in a group of the third attribute, contribution ratios tend to be unbiased.

When an attribute is designated in accordance with an input operation by a user with respect to input unit 110, attribute distinguisher 124 may distinguish the designated attribute as the attribute of a group to which a plurality of compounds shown in composition list 1 belong.

As described above, attribute distinguisher 124 according to the present embodiment performs a principal component analysis with respect to a plurality of compounds using a normalized component ratio of two or more elements contained in each of the plurality of compounds and, based on a result of the principal component analysis, distinguishes the first attribute, the second attribute, or the third attribute as an attribute of a group. In other words, based on a variability of a component ratio of two or more elements contained in each of the plurality of compounds, attribute distinguisher 124 specifies one or more feature amounts with respect to the plurality of compounds, and when the one or more feature amounts satisfy a first condition, distinguishes the first attribute as the attribute of the group. In addition, when the one or more feature amounts satisfy a second condition, attribute distinguisher 124 distinguishes the second attribute as the attribute of the group, and when the one or more feature amounts satisfy a third condition, attribute distinguisher 124 distinguishes the third attribute as the attribute of the group. Note that the attribute of the group described above is an attribute of a group of a plurality of compounds shown in composition list 1. Furthermore, the one or more feature amounts is, for example, the contribution ratio of the first principal component, the second principal component, the third principal component, the fourth principal component, and the like obtained from a result of a principal component analysis. For example, the first condition is that the cumulative contribution ratio from the first principal component to the fourth principal component exceeds the first threshold. The second condition is that the contribution ratio of the first principal component is m-times the contribution ratio of the second principal component or more. The third condition is that the contribution ratios of the first principal component, the second principal component, the third principal component, the fourth principal component, and the like neither satisfy the first condition nor satisfy the second condition.

In addition, it can be said that each of the plurality of compounds belonging to a group of the first attribute is a solid solution and each of the plurality of compounds belonging to a group of the second attribute contains a dopant. Therefore, it can be said that when each of the plurality of compounds belonging to a group is a solid solution, attribute distinguisher 124 according to the present embodiment distinguishes the first attribute as the attribute of the group, and when each of the plurality of compounds contains a dopant, attribute distinguisher 124 according to the present embodiment distinguishes the second attribute as the attribute of the group. Furthermore, it can be said that when each of a plurality of compounds is neither a solid solution nor contains a dopant, attribute distinguisher 124 distinguishes the third attribute as the attribute.

(Processing by display method determiner 125)

<In case of first attribute>

When attribute distinguisher 124 distinguishes that an attribute of a group is the first attribute, display method determiner 125 determines a first display method as a display method of composition list 1 of the group. The first display method is a method of determining variables based on a result of a principal component analysis, generating processed image 2 by plotting a coordinate point of each compound on a graph having coordinate axes representing the variables, and displaying processed image 2. The coordinate axes of the graph include an abscissa axis and an ordinate axis. In addition, an abscissa variable is determined as a variable used for the abscissa axis and an ordinate variable is determined as a variable used for the ordinate axis.

In other words, when attribute distinguisher 124 distinguishes that an attribute of a group is the first attribute, display method determiner 125 determines an abscissa variable and an ordinate variable based on a result of the principal component analysis. In addition, output processor 126 generates processed image 2 by plotting a coordinate point of each compound on a graph having the abscissa axis representing the abscissa variable and the ordinate axis representing the ordinate variable and outputs processed image 2.

For example, display method determiner 125 may determine a variable representing the first principal component as the abscissa variable of the first display method and determine a variable representing the second principal component as the ordinate variable of the first display method. Alternatively, display method determiner 125 may determine a variable representing the third principal component as the abscissa variable of the first display method and determine a variable representing the fourth principal component as the ordinate variable of the first display method. In addition, display method determiner 125 may determine the abscissa variable or the ordinate variable using a plurality of elements contained in an n-th principal component being any one principal component of the first principal component to the fourth principal component.

FIG. 5 is a diagram for describing a method of determining the abscissa variable and the ordinate variable used for the abscissa axis and the ordinate axis of a graph according to the first display method.

The n-th principal component has an element corresponding to the composition coefficient of each element. For example, the n-th principal component $j(n)$ is expressed by an expression "$j(n) = a \times Li + b \times Hf + c \times In + d \times Zr + e \times Ga + f \times O$". Li, Hf, In, Zr, Ga, and O in the expression above are variables to which the composition coefficient of the element Li, the composition coefficient of the element Hf, the composition coefficient of the element In, the composition coefficient of the element Zr, the composition coefficient of the element Ga, and the composition coefficient of the element O are to be assigned. Chemical symbols (for example, Li and Al) contained in the respective mathematical expressions below are also variables to which composition coefficients of elements represented by the chemical symbols are assigned. In addition, each of a, b, c, d, e, and f in the expressions above is an element (in other words, a coefficient) corresponding to a composition coefficient of an element. A magnitude of the elements is dependent on a composition formula of the plurality of compounds.

As illustrated in (a) in FIG. 5, display method determiner 125 specifies a magnitude of each of the plurality of elements contained in the n-th principal component. In addition, display method determiner 125 selects one element at a time in a descending order of absolute values from a plurality of elements included in a population. In other words, display method determiner 125 selects elements from the population in a descending order of absolute values. For example, the plurality of elements included in the population are a, b, c, d, e, and f. In addition, every time display method determiner 125 selects one element, display method determiner 125 determines whether or not a proportion of a norm of a vector made up of one or more elements selected until then to a norm of the original vector exceeds a second threshold. The original vector is a vector made up of a plurality of elements included in the population and is, for example, (a, b, c, d, e, f). The second threshold is, for example, 99%. When display method determiner 125 determines that the proportion of the norm exceeds the second threshold, display method determiner 125 stops selecting the elements. For example, as illustrated in (b) in FIG. 5, display method determiner 125 selects an element a that corresponds to the variable Li, an element b that corresponds to the variable Hf, an element c that corresponds to the variable In, an element d that corresponds to the variable Zr, and an element e that corresponds to the variable Ga. When these elements are selected, display method determiner 125 does not select an element f corresponding to the variable O if the proportion of the norm made up of the selected elements exceeds the second threshold. The one or more elements selected in this manner are elements that have nonzero values.

While display method determiner 125 selects elements using a norm in the example described above, a norm need not be used. For example, display method determiner 125 may only select elements with a magnitude equal to or larger than a threshold and need not select elements with a magnitude smaller than the threshold.

In addition, display method determiner 125 performs discretization with respect to the one or more selected elements. For example, as illustrated in (a) and (b) in FIG. 5, display method determiner 125 discretizes the selected elements a, b, c, d, and e. Specifically, display method determiner 125 converts an element having a positive value into an element having a value of "1" and converts an element having a negative value into an element having a value of "-1". In other words, display method determiner 125 converts an absolute value of each of the one or more selected elements into 1 and retains a sign of the element. Furthermore, display method determiner 125 may convert a value which was not selected (for example, the element f) into 0.

As a result, for example, as illustrated in (c) in FIG. 5, display method determiner 125 determines an ordinate variable ya used for the ordinate axis of the graph as "ya = Li + Hf - In + Zr - Ga".

The n-th principal component described above may be the fourth principal component or may be the first principal component, the second principal component, or the third principal component. In addition, in a similar manner to the ordinate variable ya, display method determiner 125 also determines the abscissa variable based on another principal component that differs from the principal component used for the ordinate variable ya. The other principal component may be the first principal component, the second principal component, the third principal component, or the fourth principal component. Accordingly, variables respectively used for the abscissa axis and the ordinate axis of the graph are determined based on the principal components. In other words, display method determiner 125 determines the coordinate axes of the graph. Specifically, display method determiner 125 determines the abscissa axis and the ordinate axis representing the abscissa variable and the ordinate variable obtained from a result of the principal component analysis as coordinate axes of the graph in processed image 2. Accordingly, the first display method is determined.

Display method determiner 125 notifies output processor 126 of the abscissa variable and the ordinate variable determined in this manner.

<In case of second attribute>

On the other hand, when attribute distinguisher 124 distinguishes that an attribute of a group is the second attribute, display method determiner 125 determines a second display method as a display method of composition list 1 of the group. The second display method is a method of determining an added element corresponding to coordinate axes of each of one or more graphs based on a result of a principal component analysis, generating processed image 2 by plotting a coordinate point of each compound on the graphs, and displaying processed image 2. The coordinate axes of each graph include an abscissa axis and an ordinate axis. In addition, each of the abscissa axis and the ordinate axis represents a composition coefficient of the single added element determined as described above.

In other words, when attribute distinguisher 124 distinguishes that an attribute of a group is the second attribute, display method determiner 125 determines one or more added elements based on a result of the principal component analysis. Output processor 126 specifies all combinations of two added elements from the one or more determined added elements. In addition, for each combination, output processor 126 generates a graph having an abscissa axis and an ordinate axis representing composition coefficients of the two added elements contained in the combination. Note that the two added elements contained in the combination may be the same or may differ from one another. In addition, output processor 126 generates processed image 2 by plotting a coordinate point of each compound on the graphs.

Specifically, display method determiner 125 determines the one or more added elements based on the first principal component obtained from a result of the principal component analysis.

FIGS. 6A and 6B are diagrams for describing a method of determining an added element.

As illustrated in FIG. 6A, display method determiner 125 specifies a magnitude of each of the plurality of elements contained in the first principal component. In addition, in a similar manner to that described above, display method determiner 125 selects one element at a time in a descending order of absolute values from a plurality of elements included in a population. For example, the first principal component $j(1)$ is expressed by expression "$j(1) = a \times A + b \times B + c \times C + d \times D + e \times E + f \times F$". In this case, each of A, B, C, D, E, and F in the expression above is a variable to which a composition coefficient of an element is to be assigned and each of a, b, c, d, e, and f is an element (in other words, a coefficient) corresponding to a composition coefficient of an element. The plurality of elements included in the population are a, b, c, d, e, and f. In addition, every time display method determiner 125 selects one element, display method determiner 125 determines whether or not a proportion of a norm of a vector made up of one or more elements selected until then to a norm of the original vector exceeds a third threshold. The original vector is a vector made up of a plurality of elements included in the population and is, for example, (a, b, c, d, e, f). The third threshold is, for example, 80% or 90%.

When display method determiner 125 determines that the proportion of the norm exceeds the third threshold, display method determiner 125 stops selecting the elements. For example, as illustrated in FIG. 6A, display method determiner 125 selects an element b that corresponds to the variable B, an element d that corresponds to the variable D, and an element e that corresponds to the variable E. When these elements are selected, display method determiner 125 does not select an element a that corresponds to the variable A, an element c that corresponds to the variable C, and an element f that corresponds to the variable F if the proportion of the norm made up of the selected elements exceeds the third threshold. The one or more elements selected in this manner are elements that have nonzero values. In addition, display method determiner 125 determines the three elements corresponding to the selected element b, element d, and element e (in other words, the three elements corresponding to the variable B, the variable D, and the variable E) as primary elements. Hereinafter, a primary element determined as a result of a principal component analysis in this manner or, in other words, a primary element determined based on the first principal component is also referred to as a first primary element.

Furthermore, as illustrated in FIG. 6B, display method determiner 125 also determines elements other than the first primary element determined based on the first principal component as primary elements. In other words, display method determiner 125 further determines an element other than the first primary element as a primary element without using the result of the principal component analysis. Hereinafter, a primary element determined without using the result of a principal component analysis in this manner will also be referred to as a second primary element.

Specifically, display method determiner 125 calculates a mean value and a variance of composition coefficients of the respective elements contained in the plurality of composition formulas shown in composition list 1. For example, display method determiner 125 calculates a mean value and a variance of composition coefficients of the element corresponding to the variable A, a mean value and a variance of composition coefficients of the element corresponding to the variable B, a mean value and a variance of composition coefficients of the element corresponding to the variable C, and the like in the composition formulas of the plurality of compounds. In addition, in the plurality of elements contained in the plurality of composition formulas shown in composition list 1, display method determiner 125 specifies an element that has the mean value of composition coefficients of larger than 0 and a variance of the composition coefficients of 0 from one or more remaining elements with the exception of the first primary element determined based on the first principal component. Display method determiner 125 also determines an element specified in this manner as a primary element. In other words, display method determiner 125 determines an element specified in this manner as a second primary element.

For example, among the six elements corresponding to the variables A to F, the three elements corresponding to the variable B, the variable D, and the variable E are determined as first primary elements based on the first principal component. Therefore, among the six elements corresponding to the variables A to F, the one or more remaining elements excluding the three first primary elements are the three elements corresponding to the variable A, the variable C, and the variable F. Display method determiner 125 determines the element corresponding to the variable F as an element that has the mean value of composition coefficients of larger than 0 and a variance of the composition coefficients of 0 from the three elements corresponding to the variable A, the variable C, and the variable F. Display method determiner 125 further determines the specified element corresponding to the variable F as a primary element. In other words, display method determiner 125 determines the element corresponding to the variable F specified in this manner as the second primary element.

If an element that has the mean value of composition coefficients of larger than 0 and a variance of the composition coefficients of 0 is not present, the second primary element is not determined. Therefore, one or more primary elements at least including the first primary element is determined. In addition, a variance of the composition coefficients being 0 need not be limited to the variance being precisely 0. For example, a variance may be considered 0 when the variance is within an error range including 0.

In addition, display method determiner 125 determines each element excluding the one or more determined primary elements among the plurality of elements contained in the plurality of composition formulas shown in composition list 1 as an added element. At this point, even if a same element having a same composition coefficient in all of the plurality of composition formulas above is not determined as the first primary element, the element is determined as the second primary element due to the variance of composition coefficients thereof being 0, and elements excluding the first primary element and the second primary element are determined as added elements. Therefore, an added element such as a dopant can be appropriately determined.

For example, as illustrated in FIG. 6B, display method determiner 125 respectively determines the element corresponding to the variable A and the element corresponding to the variable C as added elements. In other words, display method determiner 125 determines the coordinate axes of the graph. Specifically, display method determiner 125 determines coordinate axes corresponding to the added elements obtained from a result of the principal component analysis as coordinate axes of the graph in processed image 2. Accordingly, the second display method is determined. Note that the coordinate axes represent the composition coefficient of added elements as variables.

Display method determiner 125 notifies output processor 126 of the at least one added element determined in this manner.

<In case of third attribute>

On the other hand, when attribute distinguisher 124 distinguishes that an attribute of a group is the third attribute, display method determiner 125 determines a third display method as a display method of composition list 1 corresponding to the group. For example, the third display method is a method of notifying a user that the plurality of compounds shown in composition list 1 lack coherence by displaying composition list 1 according to the first display method and the second display method or a method of prompting the user to review the compounds shown in composition list 1. In other words, the third display method is an error notification-type method. In addition, display method determiner 125 notifies output processor 126 of the determined third display method.

While display method determiner 125 determines the third display method when the attribute of a group is distinguished as the third attribute in the present embodiment, a display method of composition list 1 corresponding to the group need not be determined.

<Summary of determination of display method>

As described above, display method determiner 125 according to the present embodiment determines a display method of material information 1 that is composition list 1 by determining a coordinate axis representing a variable obtained from a result of a principal component analysis as a coordinate axis of a graph included in processed image 2.

In addition, when the first attribute is distinguished as an attribute of a group by attribute distinguisher 124, display method determiner 125 discretizes each of one or more coefficients used in an n-th principal component obtained by the principal component analysis. Furthermore, display method determiner 125 determines a coordinate axis representing a variable expressed based on one or more discretized coefficients as a coordinate axis of the graph included in processed image 2. For example, the variable represented by the coordinate axis is the ordinate variable ya described above and the ordinate variable ya is expressed based on one or more discretized coefficients or, in other words, 1 or -1 as "ya = Li + Hf - In + Zr - Ga".

In addition, when the second attribute is distinguished as an attribute of a group by attribute distinguisher 124, display method determiner 125 classifies a plurality of elements contained in the plurality of compounds into one or more first elements and one or more second elements based on one or more coefficients used in the first principal component obtained by a principal component analysis. For example, the first element is the first primary element described above and the second element is an element other than the first primary element. In addition, display method determiner 125 specifies at least one element as an added element from the one or more second elements. Furthermore, with respect to each of the at least one added element, display method determiner 125 determines a coordinate axis representing a composition coefficient of the added element as a variable as a coordinate axis of the graph of processed image 2. In this case, in the first principal component, an absolute value of a coefficient of each of the one or more second elements is smaller than an absolute value of a coefficient of each of the one or more first elements and a variance of composition coefficients of each of the at least one added element is larger than 0.

(Processing of output processor 126)

Output processor 126 generates processed image 2 showing composition list 1 according to the display method determined by display method determiner 125 and outputs processed image 2 as an image signal. Specifically, with respect to each of the plurality of compounds shown in composition list 1, output processor 126 generates processed image 2 showing coordinates derived according to the display method described above from at least one of two or more elements constituting the compound and a component ratio of the two or more elements in the compound and outputs processed image 2. As a result, processed image 2 is displayed on, for example, displayer 130.

<In case of first attribute>

FIGS. 7A and 7B are diagrams illustrating an example of processed image 2 generated with respect to composition list 1a of the first attribute.
As illustrated in FIG. 7A, output processor 126 generates processed image 2 showing composition list 1a according to the first display method. Processed image 2 has an abscissa axis and an ordinate axis and includes graph 60 on which a coordinate point of each compound shown in composition list 1a is plotted.
The abscissa axis is a coordinate axis representing an abscissa variable xa. The abscissa variable xa is a variable determined by display method determiner 125 based on, for example, the third principal component. In a specific example, the abscissa variable xa is expressed by expression "xa = In - Ga" or "xa = (In) - (Ga)". Ga or (Ga) in the expression is a variable to which a composition coefficient of the element Ga is to be assigned and In or (In) is a variable to which a composition coefficient of the element In is to be assigned. Note that the composition coefficient to be assigned to the abscissa variable xa may be normalized so that a sum of respective composition coefficients contained in the composition formula equals 1.
The ordinate axis is a coordinate axis representing an ordinate variable ya. The ordinate variable ya is a variable determined by display method determiner 125 based on, for example, the fourth principal component. In a specific example, the ordinate variable ya is expressed by expression "ya = Li + Hf - In + Zr - Ga" or "ya = (Zr, Hf, Li) - (In, Ga)". Note that (Zr, Hf, Li) signifies a sum of a composition coefficient of the element Li, a composition coefficient of the element Hf, and a composition coefficient of the element Zr and (In, Ga) signifies a sum of a composition coefficient of the element In and a composition coefficient of the element Ga. Note that the composition coefficient to be assigned to the ordinate variable ya may be normalized so that a sum of respective composition coefficients contained in the composition formula equals 1. In addition, an array of a plurality of chemical symbols enclosed in parentheses in each expression below signifies a sum of composition coefficients of elements corresponding to the chemical symbols.
In the example described above, in the expression "xa = In - Ga" representing the abscissa variable xa and the expression "ya = (Zr, Hf, Li) - (In, Ga)" representing the ordinate variable ya, an increase or decrease of elements preceding "-" and elements following "-" indicates that the elements are complementary.
As illustrated in FIG. 7A, a coordinate point of a compound having each composition formula shown in composition list 1a or, in other words, each composition formula expressed by (Expression 1) "$Li_{2-3a-4b}(Ga_{1-x}In_x)_a(Zr_{1-y}Hf_y)_{1+b}O_3$" above is plotted on graph 60. A coordinate point of a compound has coordinates made up of a value of the abscissa variable xa and a value of the ordinate variable ya and is denoted by a black dot in the example in FIG. 7A. The value of the abscissa variable xa is obtained by assigning respective composition coefficients of the element Ga and the element In which are contained in the composition formula of the compound to the expression "xa = (Ga) - (In)". The value of the ordinate variable ya is obtained by assigning respective composition coefficients of the element Zr, the element Hf, the element Li, the element In, and the element Ga which are contained in the composition formula of the compound to the expression "ya = (Zr, Hf, Li) - (In, Ga)".
In other words, output processor 126 generates graph 60 having an abscissa axis representing the abscissa variable xa determined by display method determiner 125 and an ordinate axis representing the ordinate variable ya determined by display method determiner 125. Next, with respect to each of the plurality of composition formulas shown in composition list 1a, output processor 126 assigns composition coefficients of the plurality of elements contained in the composition formula to the expression "xa = (Ga) - (In)" and the expression "ya = (Zr, Hf, Li) - (In, Ga)" described above. Due to the assignment, output processor 126 calculates coordinates of each of the plurality of compounds corresponding to the plurality of composition formulas. In addition, output processor 126 plots a coordinate point at the calculated coordinates on graph 60.
In such graph 60, a value indicated by the abscissa axis is a value obtained by subtracting the composition coefficient of the element Ga from the composition coefficient of the element In. Therefore, it can be readily read from graph 60 that the closer the coordinate point of a compound to a left side of graph 60, the higher a content of the element Ga of the compound, and the closer the coordinate point of the compound to a right side of graph 60, the higher a content of the element In of the compound. In addition, in the example in FIG. 7A, a large number of coordinate points are displayed lined up in a vertical direction where the abscissa variable xa = 0 on the abscissa axis. Accordingly, it can be readily read from graph 60 that many compounds that contain the element Ga and the element In in equal amounts are shown in composition list 1. In other words, when a plurality of compounds used in an experiment are shown in composition list 1, it can be readily comprehended that many compounds that contain the element Ga and the element In in equal amounts have been used in the experiment. Furthermore, in graph 60, many other coordinate points are symmetrically distributed with a line along which a plurality of coordinate points line up in the vertical direction where the abscissa variable xa = 0 as an axis of symmetry. Therefore, with respect to a plurality of compounds used in an experiment, symmetricalness of respective contents of the element Ga and the element In can be readily comprehended.

In addition, a similar effect to the abscissa axis is also produced with respect to the ordinate axis of graph 60. Therefore, in graph 60, with respect to both the abscissa axis and the ordinate axis, variables used for the coordinate axis are expressed by at least one of addition and subtraction of composition coefficients of one or more elements. As a result, a difference in composition formulas or, in other words, a difference in composition coefficients or a difference in component ratios of a plurality of elements between two coordinate points along the abscissa axis or the ordinate axis can be readily comprehended.

Alternatively, as illustrated in FIG. 7B, output processor 126 generates processed image 2 showing composition list 1a according to the first display method. As in the example illustrated in FIG. 7A, processed image 2 has an abscissa axis and an ordinate axis and includes graph 61 on which a coordinate point of each compound shown in composition list 1a is plotted. However, in the example illustrated in FIG. 7B, the variable indicated by each of the abscissa axis and the ordinate axis of the graph is different from the example illustrated in FIG. 7A.

The abscissa axis is a coordinate axis representing an abscissa variable xb. The abscissa variable xb is a variable representing, for example, the third principal component. The ordinate axis is a coordinate axis representing an ordinate variable yb. The ordinate variable yb is a variable representing, for example, the fourth principal component. In other words, in graph 61 illustrated in FIG. 7B, the third principal component and the fourth principal component are used as-is as coordinate axes.

As illustrated in FIG. 7B, a coordinate point of a compound having each composition formula shown in composition list 1a or, in other words, each composition formula expressed by (Expression 1) "$Li_{2-3a-4b}(Ga_{1-x}In_x)_a(Zr_{1-y}Hf_y)_{1+b}O_3$" above is plotted on graph 61. A coordinate point of a compound has coordinates made up of a value of the abscissa variable xb and a value of the ordinate variable yb and is denoted by a black dot in the example in FIG. 7B. The value of the abscissa variable xb is obtained by assigning composition coefficients of the respective elements contained in the composition formula of the compound to an expression for deriving the third principal component. For example, the expression is "$xb = a1 \times Li + b1 \times Ga + c1 \times In + d1 \times Zr + e1 \times Hf + f1 \times O$". Note that each of a1, b1, c1, d1, e1, and f1 is a coefficient corresponding to a composition coefficient of an element. In addition, the value of the ordinate variable yb is obtained by assigning composition coefficients of the respective elements contained in the composition formula of the compound to an expression for deriving the fourth principal component. For example, the expression is "$yb = a2 \times Li + b2 \times Ga + c2 \times In + d2 \times Zr + e2 \times Hf + f2 \times O$". Note that each of a2, b2, c2, d2, e2, and f2 is a coefficient corresponding to a composition coefficient of an element.

In other words, output processor 126 generates graph 61 having an abscissa axis representing the abscissa variable xb determined by display method determiner 125 and an ordinate axis representing the ordinate variable yb determined by display method determiner 125. Next, with respect to each of the plurality of composition formulas shown in composition list 1a, output processor 126 assigns composition coefficients of the plurality of elements contained in the composition formula to the expression "$xb = a1 \times Li + b1 \times Ga + c1 \times In + d1 \times Zr + e1 \times Hf + f1 \times O$" and the expression "$yb = a2 \times Li + b2 \times Ga + c2 \times In + d2 \times Zr + e2 \times Hf + f2 \times O$" described above. Due to the assignment, output processor 126 calculates coordinates of each of the plurality of compounds corresponding to the plurality of composition formulas. In addition, output processor 126 plots a coordinate point at the calculated coordinates on graph 61.

In such graph 61, from the perspectives of the third principal component and the fourth principal component, a distribution of each compound shown in composition list 1a can be readily comprehended.

In the examples illustrated in FIGS. 7A and 7B, variables represented by respective coordinate axes of graph 60 and graph 61 are obtained based on the third principal component or the fourth principal component. However, a variable obtained based on the first principal component or the second principal component may be used for the coordinate axes.

FIGS. 8A and 8B are diagrams illustrating other examples of processed image 2 generated with respect to composition list 1a of the first attribute.

As illustrated in FIG. 8A, output processor 126 generates processed image 2 showing composition list 1a according to the first display method. Processed image 2 has an abscissa axis and an ordinate axis and includes graph 62 on which a coordinate point of each compound shown in composition list 1a is plotted.

The abscissa axis is a coordinate axis representing an abscissa variable xc. The abscissa variable xc is a variable determined by display method determiner 125 based on, for example, the first principal component. In a specific example, the abscissa variable xc is expressed by expression "$xc = Zr - Hf - Li$" or "$xc = (Zr) - (Hf, Li)$". Zr or (Zr) in the expression denotes a variable to which a composition coefficient of the element Zr is to be assigned and Hf and Li are variables to which a composition coefficient of the element Hf and a composition coefficient of the element Li are to be respectively assigned. Note that (Hf, Li) signifies a sum of a composition coefficient of the element Hf and a composition coefficient of the element Li. In addition, the composition coefficient to be assigned to the abscissa variable xc may be normalized so that a sum of respective composition coefficients contained in the composition formula equals 1.

The ordinate axis is a coordinate axis representing an ordinate variable yc. The ordinate variable yc is a variable determined by display method determiner 125 based on, for example, the second principal component. In a specific example, the ordinate variable yc is expressed by expression "yc = O + Hf - Li" or "yc = (O, Hf) - (Li)". Li or (Li) in the expression denotes a variable to which a composition coefficient of the element Li is to be assigned and O and Hf are variables to which a composition coefficient of the element O and a composition coefficient of the element Hf are to be respectively assigned. Note that (O, Hf) signifies a sum of a composition coefficient of the element O and a composition coefficient of the element Hf. In addition, the composition coefficient to be assigned to the ordinate variable yc may be normalized so that a sum of respective composition coefficients contained in the composition formula equals 1.

As illustrated in FIG. 8A, a coordinate point of a compound having each composition formula shown in composition list 1a or, in other words, each composition formula expressed by (Expression 1) "$Li_{2-3a-4b}(Ga_{1-x}In_x)_a(Zr_{1-y}Hf_y)_{1+b}O_3$" above is plotted on graph 62. A coordinate point of a compound has coordinates made up of a value of the abscissa variable xc and a value of the ordinate variable yc and is denoted by a black dot in the example in FIG. 8A. The value of the abscissa variable xc is obtained by assigning respective composition coefficients of the element Zr, the element Hf, and the element Li which are contained in the composition formula of the compound to the expression "xc = (Zr) - (Hf, Li)". The value of the ordinate variable yc is obtained by assigning respective composition coefficients of the element O, the element Hf, and the element Li which are contained in the composition formula of the compound to the expression "yc = (O, Hf) - (Li)".

In other words, output processor 126 generates graph 62 having an abscissa axis representing the abscissa variable xc determined by display method determiner 125 and an ordinate axis representing the ordinate variable yc determined by display method determiner 125. Next, with respect to each of the plurality of composition formulas shown in composition list 1a, output processor 126 assigns composition coefficients of the plurality of elements contained in the composition formula to the expression "xc = (Zr) - (Hf, Li)" and the expression "yc = (O, Hf) - (Li)" described above. Due to the assignment, output processor 126 calculates coordinates of each of the plurality of compounds corresponding to the plurality of composition formulas. In addition, output processor 126 plots a coordinate point at the calculated coordinates on graph 62.

Furthermore, as in FIG. 8A, output processor 126 may superimpose a composition formula corresponding to a coordinate point shown in graph 62 in association with the coordinate point. Output processor 126 may superimpose composition formulas respectively corresponding to all coordinate points or specify one or more coordinate points among all of the coordinate points and superimpose composition formulas respectively corresponding to the one or more specified coordinate points. A component ratio or, in other words, a ratio of composition coefficients indicated in a composition formula to be superimposed need not be normalized or may be normalized. In addition, such a composition formula may be handled as a legend.

For example, by performing convex hull processing, output processor 126 may specify a coordinate point to be handled as a vertex as a convex hull coordinate point from coordinate points of a plurality of compounds. In other words, output processor 126 calculates a convex hull with respect to coordinates of each of the plurality of compounds shown in graph 62 and superimposes composition information indicating a composition of a compound corresponding to a vertex of the calculated convex hull on processed image 2. The composition information is, for example, a composition formula. In addition, output processor 126 outputs processed image 2 on which the composition information has been superimposed. Furthermore, output processor 126 may differentiate a display mode of the convex hull coordinate point to be shown on graph 62 of processed image 2 from a display mode of other coordinate points. In other words, as illustrated in FIG. 8A, output processor 126 generates processed image 2 including graph 62 on which a convex hull coordinate point is denoted by a white dot and a coordinate point other than the convex hull coordinate point is denoted by a black dot and displays processed image 2 on displayer 130.

Accordingly, a composition formula (in other words, composition information) to be displayed can be automatically determined and an overall picture of composition list 1a can be readily comprehended by displaying a necessary minimum amount of composition formulas. In other words, the user can readily comprehend what kind of composition formulas are included in composition list 1. Alternatively, the user can readily comprehend a region where there is a bias in composition formulas or where there is a lack of composition formulas. Therefore, for example, the user can readily comprehend a necessity of an experiment using a compound having a composition formula included in the region.

Alternatively, as illustrated in FIG. 8B, output processor 126 generates processed image 2 which is an image representing composition list 1a and which includes graph 63 according to the first display method. A composition formula is superimposed on graph 63 in a similar manner to the example in FIG. 8A. Graph 63 is a graph generated by superimposing a composition formula on graph 60 in FIG. 7A. In addition, in graph 63, a convex hull coordinate point specified by convex hull processing is denoted by a white dot and a composition formula corresponding to the convex hull coordinate point is superimposed in association with the convex hull coordinate point. In other words, output processor 126 specifies one or more convex hull coordinate points by performing convex hull processing with respect to a plurality of coordinate points shown on graph 60 in FIG. 7A. Furthermore, with respect to each of the one or more convex hull coordinate points, output processor 126 represents the convex hull coordinate point with a white dot and superimposes a composition formula corresponding to the convex hull coordinate point on graph 63 in association with the convex hull coordinate point. Accordingly, graph 63 clearly showing a convex hull coordinate point and a composition formula is generated.

In this case, among a plurality of coordinate points with a minimum value of the ordinate variable ya on graph 63, composition formula 11 and composition formula 12 are respectively superimposed in association with a convex hull coordinate point that is a coordinate point with a minimum value of the abscissa variable xa and a convex hull coordinate point that is a coordinate point with a maximum value of the abscissa variable xa. Composition formula 11 and composition formula 12 are "$Li_{0.2}Zr_{1.3}Ga_{0.2}O_3$" and "$Li_{0.2}Hf_{1.17}Zr_{0.13}In_{0.2}O_3$". Comparing two composition formulas 11 and 12 enables the fact that composition formula 11 "$Li_{0.2}Zr_{1.3}Ga_{0.2}O_3$" contains a large amount of the element Ga to be readily comprehended. Conversely, the fact that composition formula 12 "$Li_{0.2}Hf_{1.17}Zr_{0.13}In_{0.2}O_3$" contains a large amount of the element In can be readily comprehended. Therefore, the user can readily comprehend from, for example, a plurality of coordinate points with a minimum value of the ordinate variable ya in graph 63 that a plurality of compounds that are different in component ratios of the element Ga and the element In from each other are included in composition list 1a.

As in the examples illustrated in FIGS. 8A and 8B, output processor 126 only superimposes composition formulas corresponding to convex hull coordinate points among all coordinate points on graph 62 or graph 63. When composition formulas respectively corresponding to all coordinate points are to be superimposed, composition formulas end up being covered by other composition formulas and become less visible to the user. In other words, there is a possibility that visibility of the composition formulas may decline. On the other hand, in the present embodiment, such a decline in visibility can be suppressed by superimposing only composition formulas corresponding to convex hull coordinate points among all coordinate points. Furthermore, even only the composition formulas corresponding to the convex hull coordinate points can enable the user to readily comprehend an outline of a composition formula corresponding to every one of the coordinate points. In other words, in the present embodiment, both suppression of a decline in visibility of composition formulas and ease of comprehension of all composition formulas shown in composition list 1a from a higher perspective can be achieved.

While only composition formulas corresponding to convex hull coordinate points are superimposed in the present embodiment, a decline in visibility of respective composition formulas corresponding to all of the coordinate points may be suppressed by arranging the composition formulas so as not to overlap with each other. In addition, output processor 126 may display a coordinate point by adding a color or a marker in accordance with a property value of the coordinate point to the coordinate point.

Furthermore, in the present embodiment, since composition formulas are superimposed on a graph, the user can readily determine whether or not the graph displayed on displayer 130 is appropriate. When the graph is determined to be inappropriate by the user, information processing device 120 may change the display method of composition list 1 in accordance with an input operation by the user with respect to input unit 110. For example, information processing device 120 may distinguish an attribute designated by the user as an attribute of a group and determine a variable designated by the user as a variable used for a coordinate axis of the graph included in processed image 2.

<In case of second attribute>

FIG. 9 is a diagram illustrating an example of processed image 2 generated with respect to composition list 1b of the second attribute.

As illustrated in FIG. 9, output processor 126 generates processed image 2 showing composition list 1b according to the second display method. Processed image 2 includes a plurality of graphs 64 arrayed in a matrix pattern. Each of the plurality of graphs 64 has an abscissa axis representing a composition coefficient of an added element determined by display method determiner 125 and an ordinate axis representing a composition coefficient of an added element determined by display method determiner 125. In other words, each of plurality of graphs 64 is associated with a combination of two added elements. With respect to each of the plurality of composition formulas shown in composition list 1b, output processor 126 specifies graph 64 associated with each combination of the two added elements contained in the composition formula. In addition, output processor 126 plots a coordinate point at coordinates indicated by respective composition coefficients of the two added elements on specified graph 64. A coordinate point is denoted by a black dot in the example in FIG. 9. Note that composition coefficients respectively represented by the ordinate axis and the abscissa axis may be normalized or need not be normalized.

Specifically, as illustrated in FIG. 9, when nine added elements or, namely, the element Ti, the element Mo, the element Mn, the element Al, the element Cr, the element Cu, the element V, the element Ni, and the element Nb are determined by display method determiner 125, output processor 126 generates processed image 2 including $9 \times 9$-number of graphs 64. For example, graph 64a among graphs 64 has an abscissa axis representing a composition coefficient of the element Nb and an ordinate axis representing a composition coefficient of the element Ti. In addition, nine graphs having abscissa axes respectively corresponding to mutually different added elements are arrayed in a lateral direction and nine graphs having ordinate axes respectively corresponding to mutually different added elements are arrayed in a longitudinal direction.

$9\times9$-number of graphs 64 described above also include nine graphs 64 in which an abscissa axis and an ordinate axis represent composition coefficients of a same element. Nine graphs 64 are arrayed in a diagonal direction from top left to bottom right in the array of $9\times9$-number of graphs 64. In nine graphs 64 described above, since composition coefficients of the same element are respectively represented by the abscissa axis and the ordinate axis, a value of the abscissa axis and a value of the ordinate axis are the same at each coordinate point. Note that instead of graph 64 showing composition coefficients of a same element as described above, output processor 126 may generate a histogram showing a composition coefficient and an occurrence rate of the added element and include the histogram in processed image 2. An abscissa axis in the histogram represents a composition coefficient of an added element and an ordinate axis represents an occurrence rate at which the added element with the composition coefficient occurs.

In the example in FIG. 9, on graph 64a, a coordinate point of a compound that does not contain at least one of the element Nb and the element Ti is plotted on the abscissa axis or the ordinate axis. On the other hand, a coordinate point of a compound that contains both the element Nb and the element Ti is plotted at a position deviated from the abscissa axis and the ordinate axis. Therefore, by viewing graph 64a, the user can readily determine the presence of a compound containing both the element Nb and the element Ti. In addition, in the present embodiment, graphs 64 corresponding to every one of combinations of the two added elements are displayed as in the example in FIG. 9. Therefore, by viewing graphs 64, the user can readily comprehend whether or not a compound containing any two added elements is included in the plurality of compounds shown in composition list 1 and can readily comprehend composition coefficients of the two added elements.

In addition, when a property value is linked with each of the composition formulas of a plurality of compounds in composition list 1d as in FIG. 3D, output processor 126 may display a coordinate point by adding a color or a marker in accordance with the property value to the coordinate point. Accordingly, the user can readily comprehend what kind of property value is obtained by what kind of combination of added elements and what kind of composition coefficients of the added elements.

Furthermore, for example, when added elements are dopants, in the example in FIG. 9, a type of a dopant and a concentration of the dopant contained in each of the plurality of compounds are represented. It can be said that the concentration of a dopant is a composition coefficient or a component ratio of an added element. When types of dopants differ from each other between materials respectively containing dopants, properties of the materials also differ from each other. In other words, a dopant type having desired properties in the materials can be comprehended. Similarly, when concentrations of dopants slightly differ from each other between materials respectively containing dopants, properties of the materials also differ from each other. In other words, a dopant concentration having desired properties in the materials can be comprehended from a minute difference in concentrations of the dopant. Note that the materials are compounds. In material development, in order to create a material having desired properties, a plurality of compounds that are different from one another in at least one of a type of a dopant and a concentration of the dopant may be generated and the compounds may be compared with each other. In a step of comparing such a plurality of compounds, the larger the number of compounds, the more troublesome it becomes to organize the compounds. Therefore, a comparison of a large number of disorganized compounds becomes a factor in preventing efficiency of material development from being improved. In addition, even when conditions of generation increase in order to generate a plurality of compounds that are different from one another in both a type of a dopant and a concentration of the dopant, organizing the plurality of compounds becomes a hassle. As a result, a comparison of a large number of disorganized compounds becomes a factor in preventing efficiency of material development from being improved.

However, in the present embodiment, as illustrated in FIG. 9, a type of a dopant and a concentration of the dopant contained in each of the plurality of compounds are displayed in an organized manner. Accordingly, a type of a dopant and a concentration of the dopant contained in each of the plurality of compounds can be readily comprehended. As a result, efficiency of material development can be improved.

<In case of third attribute>

FIG. 10A is a diagram illustrating an example of processed image 2 generated with respect to composition list 1c of the third attribute. FIGS. 10B, 10C, and 10D are diagrams illustrating, in an enlarged manner, a part of processed image 2 in FIG. 10A.

As illustrated in FIG. 10A, output processor 126 generates processed image 2 showing composition list 1c according to the third display method. Processed image 2 includes message information 2a, principal component analysis result 2b, first attribute processed images 2c and 2d, and second attribute processed image 2e.

For example, using a character string, message information 2a indicates that input data being composition list 1c needs to be reviewed. In other words, message information 2a indicates that an attribute of a group including a plurality of compounds shown in composition list 1c does not fall under the first attribute nor the second attribute and that there is no appropriate display method with respect to composition list 1c.

First attribute processed image 2c is an image showing composition list 1c according to the first display method and includes a graph with two coordinate axes based on the first principal component and the second principal component. In other words, output processor 126 causes display method determiner 125 to determine, according to the first display method, an abscissa variable and an ordinate variable to be used for the two coordinate axes of the graph for displaying composition list 1c. The abscissa variable and the ordinate variable are, for example, variables based on the first principal component and the second principal component.

In a specific example, as illustrated in FIG. 10B, output processor 126 generates graph 65 having an abscissa axis representing an abscissa variable which is based on the first principal component and which is determined by display method determiner 125 and an ordinate axis representing an ordinate variable which is based on the second principal component and which is determined by display method determiner 125. In addition, output processor 126 plots a coordinate point of each compound shown in composition list 1c on graph 65, generates first attribute processed image 2c including graph 65, and includes first attribute processed image 2c in processed image 2.

In this example, graph 65 is generated according to the first display method despite the attribute of composition list 1c not being the first attribute. Therefore, as illustrated in FIG. 10B, a calculation of respective values of the abscissa variable and the ordinate variable used for the two coordinate axes of graph 65 requires composition coefficients of a larger number of elements as compared to the example illustrated in FIG. 7A.

As a result, it is difficult for a user having viewed first attribute processed image 2c to read a trend and an overall picture of a plurality of compounds from first attribute processed image 2c. Therefore, the user can readily comprehend that the plurality of compounds shown in composition list 1c needs to be reviewed.

First attribute processed image 2d is an image showing composition list 1c according to the first display method and includes a graph with two coordinate axes based on the third principal component and the fourth principal component. In other words, output processor 126 causes display method determiner 125 to determine, according to the first display method, an abscissa variable and an ordinate variable to be used for the two coordinate axes of the graph for displaying composition list 1c. The abscissa variable and the ordinate variable are, for example, variables based on the third principal component and the fourth principal component.

In a specific example, as illustrated in FIG. 10C, output processor 126 generates graph 66 having an abscissa axis representing an abscissa variable which is based on the third principal component and which is determined by display method determiner 125 and an ordinate axis representing an ordinate variable which is based on the fourth principal component and which is determined by display method determiner 125. In addition, output processor 126 plots a coordinate point of each compound shown in composition list 1c on graph 66, generates first attribute processed image 2d including graph 66, and includes first attribute processed image 2d in processed image 2.

In this example too, graph 66 is generated according to the first display method despite the attribute of composition list 1c not being the first attribute. Therefore, as illustrated in FIG. 10C, a calculation of respective values of the abscissa variable and the ordinate variable used for the two coordinate axes of graph 66 requires composition coefficients of a larger number of elements as compared to the example illustrated in FIG. 7A.

As a result, it is difficult for a user having viewed first attribute processed image 2d to read a trend and an overall picture of a plurality of compounds from first attribute processed image 2d. Therefore, the user can readily comprehend that the plurality of compounds shown in composition list 1c needs to be reviewed.

Second attribute processed image 2e is an image showing composition list 1c according to the second display method and includes a plurality of graphs with two coordinate axes representing composition coefficients of two added elements. In other words, output processor 126 causes display method determiner 125 to determine, according to the second display method, a plurality of added elements to be used for displaying composition list 1c.

However, when an attribute of a group of the plurality of compounds shown in composition list 1c is the third attribute, there is a possibility that many added elements are to be determined by display method determiner 125. In a specific example, 61 added elements may be determined. Using each of such a large number of added elements for an ordinate axis and an abscissa axis equates to generating $61 \times 61$-number of graphs and, as a result, comprehending coordinate points of the plurality of compounds included in composition list 1c becomes difficult.

In consideration thereof, as illustrated in FIG. 10D, output processor 126 optionally extracts a maximum of 10 added elements from the 61 added elements. In addition, output processor 126 specifies all combinations of two added elements among the 10 added elements and generates graph 67 for each of the combinations. In other words, $10 \times 10$-number of graphs 67 are generated. The abscissa axis and the ordinate axis of graphs 67 represent composition coefficients of the two added elements.

In addition, as illustrated in FIG. 10D, output processor 126 plots a coordinate point of each compound shown in composition list 1c on any one of graphs 67, generates second attribute processed image 2e including graph 67, and includes second attribute processed image 2e in processed image 2. Furthermore, the number of all added elements determined by display method determiner 125 and the number of added elements extracted from all of the added elements and used in a plurality of graphs 67 may be notated by a text in second attribute processed image 2e. For example, a notation of "10 of 61 added elements displayed" may be used.

However, even when limiting the number of added elements from 61 to 10 as described above, composition list 1c is displayed based only on a part of a large number of added elements and an entirety of composition list 1c is not displayed in second attribute processed image 2e. Consequently, it is difficult for a user having viewed second attribute processed image 2e to read a trend and an overall picture of a plurality of compounds from second attribute processed image 2e. Therefore, the user can readily comprehend that the plurality of compounds shown in composition list 1c needs to be reviewed.

As described above, displaying processed image 2 illustrated in FIG. 10A enables the user to reselect a compound to be shown in composition list 1c. In other words, by performing an input operation with respect to input unit 110, the user can change a composition formula, add a composition formula, or delete a composition formula of a compound shown in composition list 1c. When such a reselection is performed, information processing device 120 obtains composition list 1 after the reselection and repetitively executes processing similar to that described above based on composition list 1. In addition, if a reselection is performed not only when processed image 2 illustrated in FIG. 10A is displayed but also when processed image 2 illustrated in FIGS. 7A to 9 is displayed, information processing device 120 may repetitively execute processing similar to that described above based on composition list 1 after the reselection.

FIG. 11 is a diagram for describing an effect of information processing device 120 according to the present embodiment. Note that FIG. 11 illustrates processed image 2 by information processing device 120 according to the present embodiment in comparison with a comparative example such as graphs 71 and 72.

In material development, experiments are carried out for various purposes and data collection is performed. It is expected that data of experiments carried out for various purposes are accumulated at one location to be used as a large-scale data set. However, if the data is accumulated at one location, visual observation alone cannot determine an attribute of a composition list being a data set made up of a large number of accumulated pieces of data and confirming contents of the composition list takes a long time. In addition, in conventional material development, a method of accumulating experimental data or, in other words, which of the data is to be accumulated to create a composition list is left to the discretion of individuals. For example, even if a composition list can be readily confirmed by a creator of the composition list, it takes a long time for a third person to confirm contents of the composition list since the third person is unaware of the attribute of the composition list.

However, in information processing device 120 according to the present embodiment, the attribute can be distinguished and processed image 2 in accordance with the attribute can be displayed.

For example, as illustrated in FIG. 11, accumulated data includes composition lists A, B, and C. In addition, in a comparative example, each of a plurality of composition formulas shown in the composition lists is handled as a vector having composition coefficients as vector elements and a principal component analysis is performed with respect to the vectors. Subsequently, graphs 71 and 72 are generated. An abscissa axis and an ordinate axis of each of graphs 71 and 72 represent a principal component as a variable. Note that graph 71 is generated from composition list A and graph 72 is generated from composition list B. Such graphs 71 and 72 are scatter diagrams that represent composition formulas shown in composition lists A and B as coordinate points and are generated by a same method regardless of the attributes of composition lists A and B.

While the fact that composition lists A and B have some kind of distribution can be read from graphs 71 and 72, determining a specific spread requires confirming each element of a principal component. In addition, a set made up of a plurality of elements contained in a principal component is a vector, a dimension of which corresponds to the number of elements included in composition list A or B. Therefore, even if the plurality of elements (in other words, the vector) are confirmed, there is no guarantee that what the distribution of coordinate points means or what kind of composition formula a coordinate point is indicating can be readily interpreted from graphs 71 and 72.

On the other hand, in the present embodiment, processed image 2 suitable for an attribute of a composition list is generated and displayed. For example, if composition list A is composition list 1a in FIG. 3A, processed image 2 includes graph 60 described above. If each composition formula shown in composition list A is normalized, respective composition coefficients of the element Ga and the element In are replaced in a complementary manner in a direction of the axis of abscissa of graph 60. In addition, in a direction of the axis of ordinate of graph 60, due to an increase in the respective composition coefficients of the element Ga and the element In, the respective composition coefficients of the element Li, the element Zr, and the element Hf decrease. In other words, graph 60 is a scatter diagram using coordinate axes focusing on composition coefficients of the elements. Accordingly, for example, a coordinate point on a right side of the axis of abscissa represents a composition formula in which the composition coefficient of the element In is large and the composition coefficient of the element Ga is small, and a coordinate point on a left side of the axis of abscissa represents a composition formula in which the composition coefficient of the element In is small and the composition coefficient of the element Ga is large. In addition, the fact that the composition formula contains the same amounts of the element In and the element Ga at coordinate 0 of the axis of abscissa can be read from graph 60.

On the other hand, if composition list B is composition list 1b in FIG. 3B, composition list B shows a plurality of composition formulas having the element Fe as a principal component. In addition, the composition formulas contain only small amounts of various elements. In the present embodiment, processed image 2 including a plurality of graphs 64 is displayed with respect to such composition list B. Processed image 2 is a scatter diagram having coordinate axes that focus on every one of the various elements (in other words, dopants). Accordingly, combinations of the various elements can be readily read from processed image 2.

In this manner, in the present embodiment, an appropriate display method of processed image 2 showing a composition list is determined in accordance with an attribute of the composition list. Therefore, the composition list can be readily comprehended and material development can be appropriately supported. On the other hand, in graphs 71 and 72, since display in accordance with an attribute of the composition list is not performed, it is difficult to readily comprehend a distribution of the composition formulas shown in the composition list from the graphs. In other words, in the present embodiment, even when handling large-scale data, an appropriate display method can be automatically determined for each piece of large-scale data and the large-scale data can be displayed accordingly.

[Flow of processing by information processing device 120]

FIG. 12 is a flowchart illustrating an example of the processing operations of information processing device 120 according to the present embodiment.

(Step S10)

First, obtainer 123 obtains material information 1 that is composition list 1 in response to, for example, an input operation to input unit 110 by the user and outputs material information 1 to attribute distinguisher 124, display method determiner 125, and output processor 126.

(Step S11)

Attribute distinguisher 124 obtains material information 1 from obtainer 123 and, based on a plurality of compounds shown in material information 1, distinguishes an attribute of a group to which the plurality of compounds belong. In addition, attribute distinguisher 124 notifies display method determiner 125 of the distinguished attribute.

(Step S12)

Display method determiner 125 obtains material information 1 outputted from obtainer 123 and receives the attribute notified from attribute distinguisher 124. In addition, display method determiner 125 determines, using material information 1, a display method of material information 1 according to the attribute. For example, an abscissa variable and an ordinate variable are determined as a first display method when the attribute is the first attribute and at least one added element is determined as a second display method when the attribute is the second attribute.

(Step S13)

Output processor 126 obtains material information 1 outputted from obtainer 123 and receives the display method notified from display method determiner 125. In addition, output processor 126 generates processed image 2 showing material information 1 according to the notified display method. For example, when the notified display method is the first display method, processed image 2 including a graph having an abscissa axis representing the abscissa variable and an ordinate axis representing the ordinate variable is generated. On the other hand, when the notified display method is the second display method, processed image 2 including one or more graphs having coordinate axes respectively representing a composition coefficient of an added element is generated.

(Step S14)

Output processor 126 outputs generated processed image 2 as an image signal to, for example, displayer 130. Accordingly, on displayer 130, processed image 2 on which a coordinate point of each of a plurality of compounds is plotted is displayed.

FIG. 13A is a flowchart illustrating an example of processing operations of attribute distinguisher 124 according to the present embodiment. In other words, FIG. 13A is a flow chart illustrating details of an example of processing of step S11 in FIG. 12.

(Step S111)

Attribute distinguisher 124 normalizes, with respect to each of the plurality of compounds shown in material information 1, one or more composition coefficients contained in a composition formula of the compound.

(Step S112)

Next, attribute distinguisher 124 performs a principal component analysis with respect to the plurality of composition formulas after the normalization.

(Step S113)

Next, attribute distinguisher 124 determines whether or not a cumulative contribution ratio from the first principal component to the fourth principal component exceeds 99%.

(Step S114)

When attribute distinguisher 124 determines in step S113 that the cumulative contribution ratio is 99% or lower (No in step S113), attribute distinguisher 124 further determines whether or not a contribution ratio of the first principal component is 1.5 times a contribution ratio of the second principal component or more.

(Step S115)

When attribute distinguisher 124 determines in step S113 that the cumulative contribution ratio exceeds 99% (Yes in step S113), attribute distinguisher 124 classifies an attribute of a group to which a plurality of compounds shown in material information 1 belongs into the first attribute.

(Step S116)

In addition, when attribute distinguisher 124 determines in step S114 that the contribution ratio of the first principal component is 1.5 times the contribution ratio of the second principal component or more (Yes in step S114), attribute distinguisher 124 classifies an attribute of a group to which a plurality of compounds shown in material information 1 belongs into the second attribute.

(Step S117)

On the other hand, when attribute distinguisher 124 determines in step S114 that the contribution ratio of the first principal component is less than 1.5 times the contribution ratio of the second principal component (No in step S114), attribute distinguisher 124 classifies an attribute of a group to which a plurality of compounds shown in material information 1 belongs into the third attribute.
FIG. 13B is a flowchart illustrating an example of processing operations of display method determiner 125 according to the present embodiment. In other words, FIG. 13B is a flow chart illustrating details of an example of processing of step S12 in FIG. 12.

(Step S121)

Display method determiner 125 determines which of the first attribute, the second attribute, and the third attribute has been distinguished by attribute distinguisher 124 as the attribute.

(Step S122)

In addition, when the attribute is distinguished as the first attribute by attribute distinguisher 124, display method determiner 125 first obtains a first principal component from a result of a principal component analysis, extracts a plurality of elements using a norm from the first principal component, and discretizes each of the plurality of elements.

(Step S123)

Next, display method determiner 125 determines an axis variable based on the plurality of discretized elements.

The axis variable is an abscissa variable or an ordinate variable. Accordingly, an axis variable based on the first principal component is determined.

Display method determiner 125 also executes processing of steps S122 and S123 with respect to each of the second principal component, the third principal component, and the fourth principal component. Accordingly, with respect to each of the second to fourth principal components, an axis variable based on the principal component is determined. The first display method is determined by determining such axis variables.

(Step S124)

On the other hand, when the attribute is distinguished as the second attribute by attribute distinguisher 124, display method determiner 125 first obtains a first principal component from a result of a principal component analysis and, based on each element contained in the first principal component, selects one or more first primary elements from composition list 1.

(Step S125)

Next, display method determiner 125 selects each of one or more elements that have a mean value of composition coefficients of larger than 0 and a variance of 0 in composition list 1 as a second primary element.

(Step S126)

Next, display method determiner 125 determines each of one or more elements other than the first primary element and the second primary element from composition list 1 as an added element.

(Step S127)

In addition, with respect to each of the one or more added elements, display method determiner 125 determines an axis variable representing a composition coefficient of the added element as a variable to be used for a coordinate axis of a graph included in processed image 2. The second display method is determined by determining such axis variables.

(Step S128)

Furthermore, when the attribute is distinguished as the third attribute by attribute distinguisher 124, display method determiner 125 determines, for example, message information 2a in FIG. 10A and the like. In other words, display of processed image 2 including message information 2a and the like is determined as the third display method.

[Degree of freedom]

While attribute distinguisher 124 distinguishes an attribute of a group to which a plurality of compounds shown in material information 1 belongs according to the flow chart illustrated in FIG. 13A, attribute distinguisher 124 may distinguish the attribute of the group using a degree of freedom. In other words, attribute distinguisher 124 distinguishes the attribute based on a degree of freedom with respect to a composition of a plurality of compounds specified from material information 1. Generally, a degree of freedom is obtained by subtracting, from the number of variables that can be independently selected among one or more variables or, in other words, among a total number of variables, the number of relational expressions (for example, constraint conditions such as a binding condition and a restraint condition) which are satisfied between the variables. Such a degree of freedom can also be described as the number of independent variables.

Specifically, attribute distinguisher 124 executes processing of steps S111 and S112 in FIG. 13A. In step S112, attribute distinguisher 124 counts the number (for example, g-number) of all of the elements contained in the plurality of composition formulas indicated in material information 1. In addition, with respect to each of the plurality of composition formulas indicated in material information 1, attribute distinguisher 124 converts the composition formula into a vector, the number of elements of which is the counted number or, in other words, a vector made up of g-number of vector elements. An i-th (where i is an integer of 1 or more and g or less) vector element of the vector represents a composition coefficient of an element corresponding to the i-th vector element. Next, attribute distinguisher 124 performs a principal component analysis with respect to the plurality of vectors.

Attribute distinguisher 124 derives the number of principal components that exceed 99% in a cumulative contribution ratio as a degree of freedom by a principal component analysis with respect to the plurality of vectors or, in other words, a principal component analysis with respect to the plurality of compounds. Specifically, attribute distinguisher 124 derives n as the degree of freedom when a cumulative contribution ratio from the first principal component to an (n-1)-th principal component is 99% or lower and a cumulative contribution ratio from the first principal component to an n-th principal component exceeds 99%.

For example, when each composition formula is converted into a vector made up of g-number of vector elements as described above, the degree of freedom represents a spread of points in a g-dimensional space indicated by the vector and corresponds to the number of independent variables. When the constraint conditions described above do not exist among the composition formulas, since a numerical value of a constant multiple with respect to each composition coefficient contained in composition formulas has no meaning, the degree of freedom is (g - 1). Specifically, when g = 8, the degree of freedom is 7. In addition, when the constraint conditions exist among the composition formulas as in (Expression 1) "$Li_{2-3a-4b}(Ga_{1-x}In_x)_a(Zr_{1-y}Hf_y)_{1+b}O_3$" described above, the degree of freedom is calculated by further subtracting a number corresponding to the constraint conditions from g. In other words, the degree of freedom is calculated by g - 1 - "number corresponding to constraint conditions". The degree of freedom calculated in this manner corresponds to the number of principal components that exceed 99% in the cumulative contribution ratio described above.

Furthermore, when the degree of freedom is equal to or smaller than a first threshold, attribute distinguisher 124 distinguishes the first attribute as the attribute. In addition, when the degree of freedom is larger than the first threshold and equal to or smaller than a second threshold, attribute distinguisher 124 distinguishes the second attribute as the attribute. Note that the second threshold is a value larger than the first threshold. Furthermore, when the degree of freedom is larger than the second threshold, attribute distinguisher 124 distinguishes the third attribute as the attribute. Note that each of the first threshold and the second threshold is an integer of 1 or more and a degree of freedom larger than the first threshold and equal to or smaller than the second threshold indicates the number of added elements. For example, the first threshold is 4 and the second threshold is 10. In this case, attribute distinguisher 124 distinguishes the first attribute as the attribute when the degree of freedom is 4 or smaller, distinguishes the second attribute as the attribute when the degree of freedom is 5 or larger and 10 or smaller, and distinguishes the third attribute as the attribute when the degree of freedom is 11 or larger. Alternatively, the first threshold is 6 and the second threshold is 10. In this case, attribute distinguisher 124 distinguishes the first attribute as the attribute when the degree of freedom is 6 or smaller, distinguishes the second attribute as the attribute when the degree of freedom is 7 or larger and 10 or smaller, and distinguishes the third attribute as the attribute when the degree of freedom is 11 or larger.

At this point, when the degree of freedom is larger than the first threshold and equal to or smaller than the second threshold, attribute distinguisher 124 may further determine whether or not a contribution ratio of the first principal component is 1.5 times a contribution ratio of the second principal component or more as in step S114 in FIG. 13A. In addition, when attribute distinguisher 124 determines that the contribution ratio of the first principal component is 1.5 times the contribution ratio of the second principal component or more, attribute distinguisher 124 may distinguish the second attribute as the attribute. Specifically, when the degree of freedom is larger than the first threshold and equal to or smaller than the second threshold and the contribution ratio of the first principal component which is obtained by a principal component analysis with respect to a plurality of compounds is 1.5 times the contribution ratio of the second principal component or more, attribute distinguisher 124 distinguishes the second attribute as the attribute. In this case, attribute distinguisher 124 may distinguish the third attribute as the attribute when the contribution ratio of the first principal component is less than 1.5 times the contribution ratio of the second principal component even though the degree of freedom is larger than the first threshold and equal to or smaller than the second threshold. In other words, when an attribute of a group is neither the first attribute nor the second attribute, attribute distinguisher 124 may distinguish the third attribute as the attribute of the group. However, when the third attribute is distinguished as the attribute in a case where the contribution ratio of the first principal component is less than 1.5 times the contribution ratio of the second principal component, a degree of freedom is substantially not used to distinguish whether the attribute of the group is the second attribute or the third attribute. Therefore, when the degree of freedom is larger than the first threshold and equal to or smaller than the second threshold and the contribution ratio of the first principal component is less than 1.5 times the contribution ratio of the second principal component, attribute distinguisher 124 may distinguish an attribute that is none of the first attribute, the second attribute, and the third attribute as the attribute of the group.

Note that such distinguishing of an attribute using a degree of freedom may be considered a superordinate concept of processing illustrated by the flow chart in FIG. 13A.

When an attribute of a group is distinguished using a degree of freedom as described above, display method determiner 125 determines a display method of material information 1 in accordance with the attribute according to the flow chart illustrated in FIG. 13B.

In addition, with respect to each of independent variables, the number of which corresponds to the degree of freedom, display method determiner 125 may determine a coordinate axis representing the independent variable as a coordinate axis included in processed image 2. In other words, a coordinate axis included in a graph of processed image 2 is determined. Output processor 126 generates and outputs processed image 2 having the determined coordinate axis.

For example, when the first threshold is 4 and the second threshold is 10, attribute distinguisher 124 distinguishes the first attribute as the attribute of the group when 4 is derived as the degree of freedom. In this case, display method determiner 125 may determine a display method in accordance with, for example, processed image 3 illustrated in FIG. 16B to be described later as the display method of material information 1 corresponding to the first attribute. A map included in processed image 3 has four coordinate axes, each of which representing a variable. In other words, display method determiner 125 determines four independent variables in accordance with the degree of freedom with respect to four coordinate axes A1 to A4. As a result, processed image 3 illustrated in FIG. 16B is generated by output processor 126. Note that each of the four independent variables may represent a composition coefficient of an element contained in a composition formula or may represent any of the first to fourth principal components. In addition, in a coordinate point defined by four coordinate axes A1 to A4, a property value of a compound having a composition formula corresponding to the coordinate point may be indicated by a color or a shade of color.

Alternatively, when the first threshold is 6 and the second threshold is 10, attribute distinguisher 124 distinguishes the first attribute as the attribute of the group when 6 is derived as the degree of freedom. In this case, display method determiner 125 may determine a display method in accordance with, for example, processed image 3 illustrated in FIG. 18B to be described later as the display method of material information 1 corresponding to the first attribute. A map included in processed image 3 has six coordinate axes, each of which representing a variable. In other words, display method determiner 125 determines six independent variables in accordance with the degree of freedom with respect to six coordinate axes A1 to A6. As a result, processed image 3 illustrated in FIG. 18B is generated by output processor 126. Note that, as in the previously described example, each of the six independent variables may represent a composition coefficient of an element contained in a composition formula or may represent any of the first to sixth principal components. In addition, in a coordinate point defined by six coordinate axes A1 to A6, a property value of a compound having a composition formula corresponding to the coordinate point may be indicated by a color or a shade of color.

[Advantageous effects, etc., of Embodiment 1]

As described above, information processing device 120 according to the present embodiment distinguishes an attribute of a group to which a plurality of compounds belong based on two or more elements of each of the plurality of compounds and a component ratio of the two or more elements, and outputs material information 1 according to a display method appropriate to the attribute. Therefore, the two or more elements of each of the plurality of compounds and a component ratio of the two or more elements can be output and displayed in a mode appropriate for the plurality of compounds. As a result, an appropriate display of material information 1 can be automatically obtained and efficiency of a material search of searching for a novel material from the plurality of compounds can be improved. In other words, material development can be appropriately supported.

In addition, with respect to each of the plurality of compounds in an output of material information 1, information processing device 120 generates processed image 2 showing coordinates derived according to the display method described above from at least one of two or more elements constituting the compound and a component ratio of the two or more elements and outputs processed image 2.

Accordingly, a coordinate of each of the plurality of compounds is derived from at least one of the two or more elements constituting the compound and the component ratio of the two or more elements and processed image 2 showing the coordinate of each of the plurality of compounds is displayed. For example, the coordinates are indicated by coordinate points on a graph included in processed image 2. In addition, the coordinates are derived according to a determined display method. Therefore, a distribution of respective coordinate points of the plurality of compounds can be displayed in an appropriate mode in accordance with an attribute of a group to which the plurality of compounds belong. As a result, for example, a searcher performing a material search can readily comprehend a distribution of the plurality of compounds from a higher perspective and improve efficiency of the material search.

Furthermore, when each of the plurality of compounds contains elements having a common characteristic among the plurality of compounds, information processing device 120 may distinguish the first attribute as the attribute.

Accordingly, a distribution of respective coordinate points of the plurality of compounds can be displayed in a mode suitable for a case where each of the plurality of compounds contains elements having a common characteristic among the plurality of compounds. As a result, for example, a searcher performing a material search can readily comprehend a distribution of the plurality of compounds from a higher perspective and improve efficiency of the material search.

Furthermore, attribute distinguisher 124 distinguishes the attribute based on a degree of freedom regarding compositions of the compounds, the compositions being identified from material information 1.

Accordingly, an attribute of a group to which a plurality of compounds belong is distinguished based on a distribution of the compounds in a space for expressing a composition of the plurality of compounds and material information 1 is outputted according to a display method appropriate to the distribution. Therefore, the two or more elements of each of the plurality of compounds and a component ratio of the two or more elements can be displayed in a readily understood manner from the perspective of a distribution of the plurality of compounds and efficiency of a material search of searching for a novel material from the plurality of compounds can be improved.

Furthermore, attribute distinguisher 124: distinguishes the attribute as a first attribute when the degree of freedom is less than or equal to a first threshold; distinguishes the attribute as a second attribute when the degree of freedom is greater than the first threshold and less than or equal to a second threshold; and distinguishes the attribute as a third attribute when the degree of freedom is greater than the second threshold.

Accordingly, since the attribute of a group is classified into the first attribute, the second attribute, or the third attribute depending on the magnitude of the degree of freedom, the appropriate display method that is in accordance with the distribution of the compounds can be determined from among three types of display methods.

Furthermore, for each of independent variables, display method determiner 125 determines, as a coordinate axis included in the image, a coordinate axis indicating the independent variable, a total number of the independent variables being dependent on the degree of freedom. Furthermore, output processor 126 performs the outputting process by generating the image including the coordinate axis determined, and outputting the image.

Accordingly, for example, when the degree of freedom is four, with respect to each of four independent variables, a coordinate axis representing the independent variable is determined as a coordinate axis of an image showing a coordinate of each of the plurality of compounds. Therefore, the two or more elements of each of the plurality of compounds and a component ratio of the two or more elements can be displayed in a readily understood manner using coordinate axes with the number corresponding to the degree of freedom.

Furthermore, attribute distinguisher 124 derives, as the degree of freedom, a total number of principal components having a cumulative contribution ratio exceeding 99 percent, by performing a principal component analysis on the compounds.

Accordingly, the degree of freedom can be appropriately derived.

Furthermore, attribute distinguisher 124 distinguishes the attribute as the second attribute when the degree of freedom is greater than the first threshold and less than or equal to the second threshold and a contribution ratio of a first principal component obtained by performing a principal component analysis on the compounds is at least 1.5 times a contribution ratio of a second principal component.

Accordingly, since the attribute of the group is distinguished as the second attribute when the contribution ratio of the first principal component is 1.5 times of the contribution ratio of the second principal component or more, an attribute of a group to which a plurality of compounds respectively containing a dopant can be distinguished as the second attribute. Therefore, material information 1 can be displayed in a readily understood manner according to a display method suitable for a compound containing a dopant. In other words, a composition coefficient of an element corresponding to the dopant can be displayed in a readily understood manner. In addition, a degree of freedom can be handled as the number of added elements.

Furthermore, information processing device 120: distinguishes the attribute as a first attribute when each of the compounds is a solid solution; distinguishes the attribute as a second attribute when each of the compounds includes a dopant. In addition, information processing device 120 distinguishes the attribute as a third attribute when each of the compounds is not a solid solution and does not include a dopant.

Accordingly, when the plurality of compounds are a solid solution, material information can be displayed in a readily understood manner according to a display method suitable for the solid solution. In other words, a magnitude relationship or a component ratio of each of the two or more elements contained in the solid solution can be displayed in a readily understood manner. Furthermore, when the plurality of compounds contain a dopant, material information can be displayed in a readily understood manner according to a display method suitable for a compound containing a dopant. In other words, a composition coefficient of an element with respect to the dopant can be displayed in a readily understood manner. Moreover, when the plurality of compounds are not a solid solution and do not contain a dopant, material information can be displayed according to, for example, an error notification-type display method, as in the example illustrated in FIG. 10A.

Furthermore, information processing device 120 identifies at least one type of feature for the compounds based on a variation in the component ratio of the two or more elements included in each of the compounds. In addition, information processing device 120: distinguishes the attribute as a first attribute when the at least one type of feature satisfies a first condition; distinguishes the attribute as a second attribute when the at least one type of feature satisfies a second condition; and distinguishes the attribute as a third attribute when the at least one type of feature satisfies a third condition.

Accordingly, an attribute of a group to which the plurality of compounds belong is classified into a first attribute, a second attribute, or a third attribute based on variability of a component ratio of the two or more elements. Therefore, a distribution of respective coordinate points of the plurality of compounds can be displayed by an appropriate display method in accordance with a tendency of the variability. In other words, when the plurality of compounds are to be used for a material search, a policy of the material search can be read from material information 1 and a distribution of respective coordinate points of the plurality of compounds can be displayed by a display method in accordance with the policy. As a result, material search can be made more efficient.

Furthermore, information processing device 120 performs a principal component analysis on the compounds using the normalized component ratio of the two or more elements, and distinguishes the attribute as the first attribute, the second attribute, or the third attribute based on a result of the principal component analysis. In addition, when the attribute is distinguished as the first attribute or the second attribute, information processing device 120 determines the display method of material information 1 by determining, as the coordinate axis included in processed image 2, a coordinate axis indicating variables obtained from the result of the principal component analysis.

Accordingly, an attribute of a group to which the plurality of compounds belong is classified into a first attribute, a second attribute, or a third attribute based on a result of a principal component analysis with respect to the plurality of compounds. As a result, the attribute can be appropriately classified. In addition, for example, when a characteristic result of the principal component analysis has been obtained with respect to the first attribute or the second attribute, the coordinate of each of the plurality of compounds is defined by a coordinate axis representing a variable obtained from the characteristic result of the principal component analysis. Therefore, in each of the first attribute and the second attribute, a distribution of respective coordinate points of the plurality of compounds can be displayed using an appropriate coordinate axis in accordance with the result of the principal component analysis. Accordingly, when the plurality of compounds are to be used for a material search, a policy of the material search can be read from material information 1 and a distribution of respective coordinate points of the plurality of compounds can be displayed by a display method in accordance with the policy. As a result, material search can be made more efficient.

In addition, when the first attribute is distinguished, information processing device 120 discretizes each of one or more coefficients used in an n-th principal component obtained by the principal component analysis and determines a coordinate axis representing a variable expressed based on the one or more discretized coefficients as a coordinate axis included in processed image 2.

Accordingly, when the attribute of the group to which the plurality of compounds belong is the first attribute, for example, each of one or more nonzero coefficients used in an n-th principal component is discretized to -1 or 1. In addition, a variable used for a coordinate axis included in processed image 2 indicates, for example, a sum of composition coefficients of one or more elements, each of which having been multiplied by -1 or 1. Therefore, in this case, a magnitude relationship of composition coefficients and a component ratio of one or more elements contained in the compound can be readily read from coordinate points of the compound having been plotted according to the coordinate axis. As a result, material search can be made more efficient.

Furthermore, information processing device 120 calculates a convex hull for the coordinates of each of the compounds; superimposes, on the image, composition information indicating a composition of a compound corresponding to a vertex of the convex hull calculated; and outputs the image on which the composition information is superimposed. For example, the composition information may be a composition formula.

Accordingly, a distribution of respective coordinate points of the plurality of compounds shown in processed image 2 and compositions of the compounds can be readily comprehended from processed image 2. In other words, when composition information is superimposed on every one of the compounds, a plurality of pieces of composition information may overlap with each other or a coordinate point may become hidden by composition information and, in such cases, both a distribution of coordinate points and compositions of compounds become difficult to understand. However, in the present embodiment, since the composition information of a compound corresponding to a vertex of a convex hull is superimposed but the composition information of other compounds is not superimposed, overlapping of a plurality of pieces of composition information and concealment of coordinate points by composition information can be suppressed. As a result, a distribution of coordinate points and a composition of each compound can be readily comprehended.

In addition, when the second attribute is distinguished, information processing device 120 classifies a plurality of elements contained in the plurality of compounds into one or more first elements and one or more second elements based on one or more coefficients used in the first principal component obtained by the principal component analysis. In addition, information processing device 120 specifies at least one element as an added element from the one or more second elements. Furthermore, with respect to each of the at least one added element, information processing device 120 determines a coordinate axis representing a composition coefficient of the added element as a variable as a coordinate axis included in processed image 2. In this case, in the first principal component, a coefficient of each of the one or more second elements is smaller than a coefficient of each of the one or more first elements and a variance of composition coefficients of each of the at least one added element is larger than 0. Note that the first element is the first primary element described above and the second element is an element other than the first primary element among the plurality of elements. For example, the second element is an added element or the second primary element described above.

Accordingly, for example, when the second attribute is distinguished with respect to a plurality of compounds, each containing an added element such as a dopant, respective coordinate points of the plurality of compounds are plotted on a graph having a coordinate axis representing a composition coefficient of the added element. Therefore, the presence of a plurality of compounds, which are slightly different in composition coefficients of the added element from each other, can be readily comprehended from processed image 2 or a graph having the coordinate axis. As a result, material search can be made more efficient.

In the embodiment described above, attribute distinguisher 124 distinguishes an attribute with respect to a group including all compounds shown in composition list 1 obtained by obtainer 123. However, attribute distinguisher 124 may distinguish an attribute with respect to a group including a part of the compounds instead of all compounds shown in composition list 1. For example, attribute distinguisher 124 may perform clustering with respect to all compounds shown in composition list 1 and distinguish an attribute for each cluster obtained as a result of the clustering.

While processed image 2 shows composition list 1 using a graph including coordinate axes in the embodiment described above, processed image 2 may show composition list 1 in other modes using a table, a chart, characters, or the like. In addition, output processor 126 may generate processed image 2 showing an attribute distinguished by attribute distinguisher 124 or generate processed image 2 showing, in a table format, coordinates calculated with respect to each of the plurality of compounds.

Furthermore, as in the case of composition list 1d in FIG. 3D, when a property value of each compound is associated with a composition formula of the compound, output processor 126 may plot a coordinate point of the compound in the graph in a mode in accordance with the property value of the compound. For example, output processor 126 may plot a coordinate point having a color or a shape in accordance with the property value.

[Embodiment 2]

[Configuration of information processing system 100a]

FIG. 14 is a diagram illustrating an example of a configuration of information processing system 100a according to the present embodiment.

Information processing system 100a according to the present embodiment obtains variable information relating to a plurality of compounds and displays a processed image including a plurality of maps in accordance with the variable information. The plurality of maps indicate properties of a plurality of compounds. Such information processing system 100a is provided in, for example, processing system 1000 in place of information processing system 100 according to the first embodiment.

Specifically, information processing system 100a obtains variable information relating to a plurality of compounds in response to an input operation by a user of information processing system 100a. In addition, information processing system 100a generates and displays a processed image in accordance with the variable information. Alternatively, information processing system 100a may transmit the processed image as an image signal to terminal system 500 via communication network Nt and cause terminal system 500 to display the processed image.

Alternatively, information processing system 100a obtains variable information via communication network Nt from terminal system 500. Information processing system 100a generates a processed image in accordance with the variable information in a similar manner to that described above even when the variable information is obtained from terminal system 500. Information processing system 100a may display the processed image or cause terminal system 500 to display the processed image.

Alternatively, when variable information is stored in database 600, information processing system 100a may read the variable information via communication network Nt from database 600. Information processing system 100a generates a processed image in accordance with the variable information in a similar manner to that described above even when the variable information is read from database 600. Information processing system 100a may display the processed image or cause terminal system 500 to display the processed image.

Such information processing system 100a includes input unit 110, information processing device 120a, and displayer 130.

Input unit 110 receives an input operation by a user of information processing system 100a and outputs an input signal in accordance with the input operation to information processing device 120a. Such input unit 110 is constituted of, for example, a keyboard, a touch sensor, a touchpad, or a mouse.

Information processing device 120a is a computer and includes communicator 121a, controller 122a, obtainer 123a, generator 127, and output processor 126a.

Communicator 121a has a communication function and communicates with terminal system 500 and database 600 via communication network Nt. Communication by communicator 121a may be wireless communication or wired communication. In addition, a type of wireless communication is not particularly limited. Controller 122a controls communicator 121a, obtainer 123a, generator 127, and output processor 126a.

Obtainer 123a obtains an input signal from input unit 110. In addition, obtainer 123a obtains a communication signal from terminal system 500 or database 600 via communicator 121a. In this case, the input signal or the communication signal obtained by obtainer 123a indicates variable information. Variable information is information relating to k-number (where k is an integer of 3 or more and 6 or less) of variables that determine a composition of a compound. In other words, obtainer 123a according to the present embodiment obtains variable information. In addition, the k-number of variables that determine a composition of a compound are variables x, y, a, b, M3', M4', and the like contained in composition formula "$Li_{2-sa-4b}(M3_{1-x}M3'_x)_a(M4_{1-y}M4'_y)_{1+b}O_3$". Variable M3' denotes a homologous element and variable M4' denotes a homologous element. Note that M3 and M4 denote elements determined in advance. Therefore, it can be described that an attribute of a group including a plurality of compounds having a composition determined by k-number of variables is the first attribute in the first embodiment described above. Consequently, it can be described that information processing device 120a according to the present embodiment generates and displays a plurality of maps indicating a property of each of a plurality of compounds included in a group of the first attribute.

Generator 127 generates a processed image including an array map constituted of an array of a plurality of maps based on variable information obtained by obtainer 123a. The plurality of maps indicate a property of each of a plurality of compounds having a composition determined by the k-number of variables described above. Generator 127 generates a processed image including an array map by arraying the plurality of maps.

Output processor 126a outputs the processed image generated by generator 127 as an image signal. For example, output processor 126a outputs the image signal to displayer 130. Note that output processor 126a may transmit the image signal to terminal system 500 as a communication signal via communicator 121a.

Displayer 130 obtains the image signal outputted from output processor 126a of information processing device 120a and displays the processed image according to the image signal.

Such information processing device 120a may be constituted of, for example, a processor such as a CPU, a volatile memory and a non-volatile memory, and a program stored in the non-volatile memory. In this case, a functional configuration of information processing device 120a is realized as the processor executes the program.

[Content of processing when k = 3]

(Processing of obtainer 123a)

For example, obtainer 123a obtains variable information relating to three (in other words, k = 3) variables which determine a composition of a compound.

FIG. 15A is a diagram illustrating an example of variable information.

Variable information 4 is information relating to three variables that determine a composition of a compound expressed by, for example, composition formula "$Li_{2-3a}(La_{1-x}Al_x)_a(Ti_{1-y}Zr_y)O_3$". The three variables are first variable x, second variable y, and third variable a for expressing a composition coefficient of each of a plurality of elements contained in a compound. For example, when the variables are to be used to search for a novel material or a novel compound, the variables are also referred to as search variables.

Such variable information 4 indicates, together with the composition formula, respective ranges of application of first variable x, second variable y, and third variable a. A range of application is a set of one or more numerical values or a numerical range which can be assumed by a variable. For example, respective ranges of application of first variable x and second variable y are "0.0, 0.1, 0.2, 0.3, 0.4, 0.5, 0.6, 0.7, 0.8, 0.9, and 1.0". The range of application of third variable a is "0.0, 0.05, 0.1, 0.15, and 0.2".

A composition of a compound expressed by the composition formula "$Li_{2-3a}(La_{1-x}Al_x)_a(Ti_{1-y}Zr_y)O_3$" is determined in accordance with a combination of values respectively applied or assigned to first variable x, second variable y, and third variable a.

(Processing of generator 127)

Generator 127 obtains a property value of each of a plurality of compounds having a composition determined by the three variables. In a similar manner to the first embodiment, for example, a property value may be a physical property value such as conductivity or density of the compound, a predicted value of a property of the compound obtained by machine learning, or a calculated value obtained by computational science. Generator 127 may obtain a property value stored in database 600 via communicator 121a or obtain a property value via communicator 121a from terminal system 500. Furthermore, generator 127 may obtain a property value in accordance with an input operation with respect to input unit 110 by the user. In addition, generator 127 generates a processed image including an array map by arraying a plurality of maps representing respective property values of the plurality of compounds.

FIG. 15B is a diagram illustrating an example of a processed image according to the present embodiment.

As illustrated in FIG. 15B, generator 127 generates array map Mb1 made up of a plurality of maps Ma and generates processed image 3 including array map Mb1. Each of the plurality of maps Ma has first coordinate axis A1 representing first variable x and second coordinate axis A2 representing second variable y. The number of maps Ma is equivalent to the number of values that third variable a can assume. In the example in FIG. 15A, the number of values that third variable a can assume is 5. Therefore, generator 127 generates five maps Ma. Each of five maps Ma is associated with a value of third variable a. Generator 127 arrays five maps Ma along third coordinate axis A3 that is parallel to first coordinate axis A1. At this point, generator 127 arrays five maps Ma in a descending order of values of third variable a corresponding to each map Ma. In addition, generator 127 maps, at a coordinate indicated by respective values of first variable x, second variable y, and third variable a on array map Mb1, a property value of a compound having a composition formula corresponding to the coordinate. The property value may be indicated by, for example, a color or a shade of color. Accordingly, array map Mb1 on which a property value is mapped is generated.

As described above, in the present embodiment, each of the plurality of maps Ma generated by generator 127 is expressed by first coordinate axis A1 representing first variable x included in k-number of variables and second coordinate axis A2 representing second variable y included in the k-number of variables. Furthermore, the plurality of maps Ma included in array map Mb1 are arrayed according to third variable a other than first variable x and second variable y among the k-number of variables. In addition, in the array of the plurality of maps Ma, generator 127 generates processed image 3 including array map Mb1 by arraying the plurality of maps Ma along third coordinate axis A3 representing third variable a. Third coordinate axis A3 is parallel to first coordinate axis A1. Note that third coordinate axis A3 may be parallel to second coordinate axis A2. In addition, each of first variable x, second variable y, and third variable a is a variable that determines a composition coefficient of an element contained in the compound. Furthermore, the number of the plurality of maps Ma arrayed along third coordinate axis A3 by generator 127 is the number of values that third variable a can assume. Note that the values that third variable a can assume may be values determined in advance such as "0.0, 0.05, 0.1, 0.15, and 0.2" illustrated in FIG. 15A or may be values optionally designated by the user.

Accordingly, even when component ratios of one or more elements having common characteristics differ according to the three variables among a plurality of compounds, processed image 3 appropriately showing information relating to the plurality of compounds can be generated.

[First content of processing when k = 4]

(Processing of obtainer 123a)

For example, obtainer 123a obtains variable information 4 relating to four (in other words, k = 4) variables which determine a composition of a compound.

FIG. 16A is a diagram illustrating another example of variable information 4.

Variable information 4 is information relating to four variables that determine a composition of a compound expressed by, for example, composition formula "$Li_{2-3a-4b}(La_{1-x}Al_x)_a(Ti_{1-y}Zr_y)_{1+b}O_3$". The four variables are first variable x, second variable y, third variable a, and fourth variable b for expressing a composition coefficient of each of a plurality of elements contained in a compound. In other words, variable information 4 in FIG. 16A is information in which fourth variable b has been added as a search variable to variable information 4 in FIG. 15A.

Such variable information 4 indicates, together with the composition formula, not only respective ranges of application of first variable x, second variable y, and third variable a but also a range of application of fourth variable b. For example, the range of application of fourth variable b is "0.0, 0.1, 0.2, and 0.3".

A composition of a compound expressed by the composition formula "$Li_{2-3a-4b}(La_{1-x}Al_x)_a(Ti_{1-y}Zr_y)_{1+b}O_3$" is determined in accordance with a combination of values respectively applied or assigned to first variable x, second variable y, third variable a, and fourth variable b.

(Processing of generator 127)

Generator 127 obtains a property value of each of a plurality of compounds having a composition determined by the four variables. In a similar manner to that described above, generator 127 may obtain a property value from database 600 or terminal system 500 or may obtain a property value in accordance with an input operation with respect to input unit 110 by the user. In addition, generator 127 generates processed image 3 including array map Mb1 by arraying a plurality of maps Ma representing respective property values of the plurality of compounds.

FIG. 16B is a diagram illustrating another example of processed image 3 according to the present embodiment.

As illustrated in FIG. 16B, generator 127 generates array map Mb1 made up of a plurality of maps Ma and generates processed image 3 including array map Mb1. In a similar manner to that described above, each of the plurality of maps Ma has first coordinate axis A1 representing first variable x and second coordinate axis A2 representing second variable y. The number of maps Ma is equivalent to the number of combinations of values that third variable a can assume and values that fourth variable b can assume. In the example in FIG. 16A, the number of values that third variable a can assume is 5, and in the example in FIG. 16A, the number of values that fourth variable b can assume is 4. Therefore, generator 127 generates 20 (in other words, 5 × 4) maps Ma. Each of 20 maps Ma is associated with respective values of third variable a and fourth variable b.

Generator 127 arrays five maps Ma along third coordinate axis A3 that is parallel to first coordinate axis A1. At this point, generator 127 arrays five maps Ma in a descending order of values of third variable a corresponding to each map Ma. Furthermore, generator 127 arrays four maps Ma along fourth coordinate axis A4 that is parallel to second coordinate axis A2. At this point, generator 127 arrays four maps Ma in a descending order of values of fourth variable b corresponding to each map Ma. Accordingly, 20 maps Ma are arrayed in a matrix pattern along third coordinate axis A3 and fourth coordinate axis A4 according to third variable a and fourth variable b. In addition, generator 127 maps, at a coordinate indicated by respective values of first variable x, second variable y, third variable a, and fourth variable b on 20 maps Ma, a property value of a compound having a composition formula corresponding to the coordinate. Accordingly, array map Mb1 on which a property value is mapped is generated.

In this manner, in the present embodiment, generator 127 generates processed image 3 including array map Mb1 by arraying the plurality of maps Ma along third coordinate axis A3 representing third variable a and fourth coordinate axis A4 representing fourth variable b. Fourth variable b is a variable other than first variable x, second variable y, and third variable a among k-number of variables. Third coordinate axis A3 is parallel to first coordinate axis A1 and fourth coordinate axis A4 is parallel to second coordinate axis A2.

In addition, each of first variable x, second variable y, third variable a, and fourth variable b is a variable that determines a composition coefficient of an element contained in the compound. Furthermore, the number of maps Ma arrayed along third coordinate axis A3 by generator 127 is the number of values that third variable a can assume, and the number of maps Ma arrayed along fourth coordinate axis A4 by generator 127 is the number of values that fourth variable b can assume. Note that the values that fourth variable b can assume may be values determined in advance such as "0.0, 0.1, 0.2, and 0.3" illustrated in FIG. 16A or may be values optionally designated by the user.

Accordingly, even when component ratios of one or more elements having common characteristics differ according to the four variables among a plurality of compounds, processed image 3 appropriately showing information relating to the plurality of compounds can be generated.

[Second content of processing when k = 4]

(Processing of obtainer 123a)

Obtainer 123a may obtain information that differs from variable information 4 illustrated in FIG. 16A or, in other words, obtain variable information 4 illustrated in FIG. 16C. Variable information 4 illustrated in FIG. 16C is also information relating to four variables (where k = 4) that determine a composition of a compound in a similar manner to variable information 4 illustrated in FIG. 16A.

FIG. 16C is a diagram illustrating yet another example of variable information 4.

Variable information 4 is information relating to four variables that determine a composition of a compound expressed by, for example, composition formula "$M1(M2_{1-x-y}M2'_xM2''_y)$". The four variables are first variable x and second variable y for expressing respective composition coefficients of a plurality of elements contained in the compound and third variable M (M2, M2', and M2") and fourth variable M1 for expressing the plurality of elements contained in the compound. Note that third variable M (M2, M2', and M2") is a variable made of a combination of variable M2, variable M2', and variable M2" and will be hereinafter also simply referred to as third variable M.

Such variable information 4 indicates, together with the composition formula, respective ranges of application of first variable x and second variable y and respective ranges of application of third variable M and fourth variable M1. Respective ranges of application of first variable x and second variable y are "0.0, 0.1, 0.2, 0.3, 0.4, 0.5, 0.6, 0.7, 0.8, 0.9, and 1.0". However, first variable x and second variable y satisfy a condition expressed as $x + y \leq 1$.

A range of application of third variable M is "Ge, Zn, Si, Cd, and In". In other words, the range of application of third variable M indicates that each of variable M2, variable M2', and variable M2" can assume any of the element Ge, the element Zn, the element Si, the element Cd, and the element In. Note that the elements that can be respectively assumed by variable M2, variable M2', and variable M2" differ from each other. Therefore, the range of application of combinations of variable M2, variable M2', and variable M2" which constitute third variable M is "Ge-Zn-Si, Ge-Cd-Si, Ge-In-Si, Zn-Cd-Si, Zn-In-Si, Cd-In-Si, Zn-Cd-Ge, Zn-In-Ge, Cd-In-Ge, and Cd-In-Zn". In addition, a range of application of fourth variable M1 is "Se, O, As, and N". In other words, the range of application of fourth variable M1 indicates that fourth variable M1 can assume the element Se, the element O, the element As, or the element N.

A composition of a compound expressed by the composition formula "$M1(M2_{1-x-y}M2'_xM2''_y)$" is determined in accordance with a combination of values or elements respectively applied or assigned to first variable x, second variable y, third variable M, and fourth variable M1.

(Processing of generator 127)

Generator 127 obtains a property value of each of a plurality of compounds having a composition determined by the four variables. In a similar manner to that described above, generator 127 may obtain a property value from database 600 or terminal system 500 or may obtain a property value in accordance with an input operation with respect to input unit 110 by the user. In addition, generator 127 generates processed image 3 including array map Mb1 by arraying a plurality of maps Maa representing respective property values of the plurality of compounds.

FIG. 16D is a diagram illustrating yet another example of processed image 3 according to the present embodiment.

As illustrated in FIG. 16D, generator 127 generates array map Mb1 made up of a plurality of maps Maa and generates processed image 3 including array map Mb1. Each of the plurality of maps Maa has a triangular shape and has, in a similar manner to that described above, first coordinate axis A1a representing first variable x and second coordinate axis A2a representing second variable y. The number of maps Maa is equivalent to the number of combinations of element sets that third variable M can assume and elements that fourth variable M1 can assume. Note that the element sets that can be assumed by third variable M are combinations of the elements that can be respectively assumed by variable M2, variable M2', and variable M2". In the example in FIG. 16C, the number of element sets that third variable M can assume is 10, and in the example in FIG. 16C, the number of elements that fourth variable M1 can assume is 4. Therefore, generator 127 generates 40 (in other words, $10 \times 4$) maps Maa. Each of 40 maps Maa is associated with respective element sets or elements of third variable M and fourth variable M1.

Generator 127 arrays 10 maps Maa along third coordinate axis A3 that is parallel to first coordinate axis A1. Furthermore, generator 127 arrays four maps Maa along fourth coordinate axis A4 that is orthogonal to third coordinate axis A3. Accordingly, 40 maps Maa are arrayed in a matrix pattern along third coordinate axis A3 and fourth coordinate axis A4 according to third variable M and fourth variable M1. In addition, generator 127 maps, at a coordinate indicated by respective values, element sets, or elements of first variable x, second variable y, third variable M, and fourth variable M1 on 40 maps Maa, a property value of a compound having a composition formula corresponding to the coordinate. Accordingly, array map Mb1 on which a property value is mapped is generated.

In this manner, in the present embodiment, generator 127 generates processed image 3 including array map Mb1 by arraying the plurality of maps Maa along third coordinate axis A3 representing third variable M and fourth coordinate axis A4 representing fourth variable M1. Fourth variable M1 is a variable other than first variable x, second variable y, and third variable M among k-number of variables. Third coordinate axis A3 is parallel to first coordinate axis A1a and fourth coordinate axis A4 is orthogonal to third coordinate axis A3.

Even when a composition formula is expressed by four variables such as those illustrated in FIG. 16C, processed image 3 appropriately showing information relating to a plurality of compounds can be generated.

[Third content of processing when k = 4]

When variable information 4 relating to four (in other words, k = 4) variables is obtained by obtainer 123a, variable information 4 may be information relating to four variables that determine a composition of a compound expressed by composition formula "$A_pD_qE_r$". In other words, a composition formula of each of a plurality of compounds is expressed by "$A_pD_qE_r$". In this case, in the composition formula, A denotes at least one element, D denotes a third variable representing an element, and E denotes a fourth variable representing an element. Furthermore, p denotes a composition coefficient of the at least one element denoted by A, q denotes a first variable representing a composition coefficient of the element denoted by D, and r denotes a second variable representing a composition coefficient of the element denoted by E.

In this case, the number of maps Ma arrayed along third coordinate axis A3 by generator 127 is the number of elements that third variable D can assume. In addition, the number of maps Ma arrayed along fourth coordinate axis A4 by generator 127 is the number of elements that fourth variable E can assume. Note that elements which third variable D and fourth variable E can respectively assume may be, for example, elements determined in advance such as the element Ti, the element Mo, the element Mn, the element Al, the element Cr, the element Cu, the element V, the element Ni, and the element Nb. Alternatively, elements which third variable D and fourth variable E can respectively assume may be elements optionally designated by the user.

In other words, as many maps Ma corresponding to combinations of elements that can be respectively assumed by third variable D and fourth variable E as the number of the combinations can be arrayed in a matrix pattern along third coordinate axis A3 and fourth coordinate axis A4. Note that first coordinate axis A1 on map Ma represents first variable q and second coordinate axis A2 on map Ma represents second variable r. Generator 127 generates array map Mb1 by arraying such a plurality of maps Ma in a matrix pattern. In addition, generator 127 maps, with respect to each of a plurality of compounds expressed by composition formula "$A_pD_qE_r$", a property value of the compound at a position in accordance with the composition formula of the compound on array map Mb1.

In addition, the composition formula "$A_pD_qE_r$" is equivalent to (Expression 2) "$Fe_{1-x-y}M_xM'_y$" according to the first embodiment. In other words, A denotes the element Fe. Variables D and E correspond to variables M and M'. Variables p, q, and r respectively correspond to "1-x-y", x, and y. Therefore, when variable information 4 is information relating to the four variables that determine a composition of a compound expressed by the composition formula "$A_pD_qE_r$", it can be described that information processing device 120a generates array map Mb1 with respect to a plurality of compounds belonging to a group of the second attribute. In other words, it can be described that information processing device 120a generates array map Mb1 with respect to a plurality of compounds respectively including an added element such as a dopant. Accordingly, a property value of a plurality of compounds respectively including an added element such as a dopant can be displayed in a readily understood manner.

[Content of processing when k = 5]

(Processing of obtainer 123a)

For example, obtainer 123a obtains variable information 4 relating to five (in other words, k = 5) variables which determine a composition of a compound.

FIG. 17A is a diagram illustrating another example of variable information 4.

Variable information 4 is information relating to five variables that determine a composition of a compound expressed by, for example, composition formula "$Li_{2-3a-4b}(La_{1-x}M3'_x)_a(Ti_{1-y}Zr_y)_{1+b}O_3$". The five variables are first variable $x$, second variable $y$, third variable $a$, and fourth variable $b$ for expressing a composition coefficient of each of a plurality of elements contained in a compound and fifth variable $M3'$ representing an element contained in the compound. In other words, variable information 4 in FIG. 17A is information in which fifth variable $M3'$ has been added as a search variable to variable information 4 in FIG. 16A.

Such variable information 4 indicates, together with the composition formula, not only respective ranges of application of first variable $x$, second variable $y$, third variable $a$, and fourth variable $b$ but also a range of application of fifth variable $M3'$. For example, the range of application of fifth variable $M3'$ is not a set of one or more numerical values or a numerical range but a set of one or more elements which fifth variable $M3'$ can assume. In a specific example, as shown in FIG. 17A, the range of application of fifth variable $M3'$ indicates that fifth variable $M3'$ can assume the element Al or the element In.

A composition of a compound expressed by the composition formula "$Li_{2-3a-4b}(La_{1-x}M3'_x)_a(Ti_{1-y}Zr_y)_{1+b}O_3$" is determined in accordance with a combination of values or elements respectively applied or assigned to first variable $x$, second variable $y$, third variable $a$, fourth variable $b$, and fifth variable $M3'$.

(Processing of generator 127)

Generator 127 obtains a property value of each of a plurality of compounds having a composition determined by the five variables. In a similar manner to that described above, generator 127 may obtain a property value from database 600 or terminal system 500 or may obtain a property value in accordance with an input operation with respect to input unit 110 by the user. In addition, generator 127 generates processed image 3 including array map Mb1 by arraying a plurality of maps Maa representing respective property values of the plurality of compounds.

FIG. 17B is a diagram illustrating another example of processed image 3 according to the present embodiment.

As illustrated in FIG. 17B, generator 127 generates two array maps Mb1 respectively made up of a plurality of maps Ma and generates processed image 3 including multi-array map Mb2 made up of two array maps Mb1. In other words, generator 127 generates as many array maps Mb1 illustrated in FIG. 16B as the number of elements (in other words, two) which fifth variable $M3'$ can assume. In a specific example, as illustrated in FIG. 17B, generator 127 generates array map Mb1 corresponding to "fifth variable $M3'$ = element Al" and array map Mb1 corresponding to "fifth variable $M3'$ = element In". In addition, generator 127 arrays two array maps Mb1 along fifth coordinate axis A5 that is respectively parallel to first coordinate axis A1 and third coordinate axis A3. Fifth coordinate axis A5 is an axis used for fifth variable $M3'$.

Generator 127 maps, at each coordinate in two array maps Mb1, a property value of a compound having a composition formula corresponding to the coordinate. The coordinate is a position indicated by respective values or elements of first variable $x$, second variable $y$, third variable $a$, fourth variable $b$, and fifth variable $M3'$ contained in the composition formula of the compound. Accordingly, multi-array map Mb2 on which a property value is mapped is generated.

In this manner, in the present embodiment, generator 127 generates, for each compound group specified by fifth variable $M3'$, array map Mb1 with respect to a plurality of compounds contained in the compound group. For example, when there are two compound groups, one compound group of the two compound groups represent a plurality of compounds which correspond to "fifth variable $M3'$ = element Al" and which are expressed by composition formula "$Li_{2-3a-4b}(La_{1-x}Al_x)_a(Ti_{1-y}Zr_y)_{1+b}O_3$". The other compound group of the two compound groups represents a plurality of compounds which correspond to "fifth variable $M3'$ = element In" and which are expressed by composition formula "$Li_{2-3a-4b}(La_{1-x}In_x)_a(Ti_{1-y}Zr_y)_{1+b}O_3$".

In addition, generator 127 generates processed image 3 including multi-array map Mb2 made up of a plurality of array maps Mb1 by arraying the plurality of array maps Mb1 along fifth coordinate axis A5 representing fifth variable $M3'$. In this case, fifth variable $M3'$ is a variable other than first variable $x$, second variable $y$, third variable $a$, and fourth variable b among k-number of variables. In addition, fifth coordinate axis A5 is parallel to third coordinate axis A3. Note that fifth coordinate axis A5 may be parallel to fourth coordinate axis A4. Furthermore, fifth variable M3' represents an element contained in a compound and the number of array maps Mb1 arrayed along fifth coordinate axis A5 by generator 127 is the number of elements that fifth variable M3' can assume. Note that the elements which fifth variable M3' can assume may be elements determined in advance such as the element Al and the element In illustrated in FIG. 17A or may be elements optionally designated by the user.

Accordingly, even when one or more elements having common characteristics and the component ratios of the one or more elements differ according to the five variables among a plurality of compounds, processed image 3 appropriately showing information relating to the plurality of compounds can be generated.

[Content of processing when k = 6]

(Processing of obtainer 123a)

For example, obtainer 123a obtains variable information 4 relating to six (in other words, k = 6) variables which determine a composition of a compound.

FIG. 18A is a diagram illustrating another example of variable information 4.

Variable information 4 is information relating to six variables that determine a composition of a compound expressed by, for example, composition formula "$Li_{2-3a-4b}(M3_{1-x}M3'_x)_a(M4_{1-y}M4'_y)_{1+b}O_3$". Note that M3 and M4 in the composition formula "$Li_{2-3a-4b}(M3_{1-x}M3'_x)_a(M4_{1-y}M4'_y)_{1+b}O_3$" respectively represent, for example, the element La and the element Ti. The six variables are first variable x, second variable y, third variable a, and fourth variable b for expressing a composition coefficient of each of a plurality of elements contained in the compound and fifth variable M3' and six variable M4' representing an element contained in the compound. In other words, variable information 4 in FIG. 18A is information in which sixth variable M4' has been added as a search variable to variable information 4 in FIG. 17A.

Such variable information 4 indicates, together with the composition formula, not only respective ranges of application of first variable x, second variable y, third variable a, fourth variable b, and fifth variable M3' but also a range of application of sixth variable M4'. For example, the range of application of sixth variable M4' is a set of one or more elements which sixth variable M4' can assume in a similar manner to fifth variable M3'. In a specific example, as shown in FIG. 18A, the range of application of sixth variable M4' indicates that sixth variable M4' can assume the element Zr or the element Hf.

A composition of a compound expressed by the composition formula "$Li_{2-3a-4b}(M3_{1-x}M3'_x)_a(M4_{1-y}M4'_y)_{1+b}O_3$" is determined in accordance with a combination of values or elements respectively applied or assigned to first variable x, second variable y, third variable a, fourth variable b, fifth variable M3', and sixth variable M4'.

(Processing of generator 127)

Generator 127 obtains a property value of each of a plurality of compounds having a composition determined by the six variables. In a similar manner to that described above, generator 127 may obtain a property value from database 600 or terminal system 500 or may obtain a property value in accordance with an input operation with respect to input unit 110 by the user. In addition, generator 127 generates processed image 3 including array map Mb1 by arraying a plurality of maps Maa representing respective property values of the plurality of compounds.

FIG. 18B is a diagram illustrating another example of processed image 3 according to the present embodiment.

As illustrated in FIG. 18B, generator 127 generates four array maps Mb1 respectively made up of a plurality of maps Ma and generates processed image 3 including multi-array map Mb2 made up of the four array maps Mb1. In other words, generator 127 generates as many array maps Mb1 illustrated in FIG. 16B as the number of combinations of elements (in other words, 2 × 2) which fifth variable M3' and sixth variable M4' can respectively assume. In a specific example, as illustrated in FIG. 18B, generator 127 generates array map Mb1 corresponding to a combination of "fifth variable M3' = element Al" and "six variable M4' = element Hf", array map Mb1 corresponding to a combination of "fifth variable M3' = element Al" and "six variable M4' = element Zr", array map Mb1 corresponding to a combination of "fifth variable M3' = element In" and "six variable M4' = element Hf", and array map Mb1 corresponding to a combination of "fifth variable M3' = element In" and "six variable M4' = element Zr". In addition, generator 127 arrays four array maps Mb1 along fifth coordinate axis A5 that is respectively parallel to first coordinate axis A1 and third coordinate axis A3 and sixth coordinate axis A6 that is respectively parallel to second coordinate axis A2 and fourth coordinate axis A4. Fifth coordinate axis A5 is an axis used for fifth variable M3' and sixth coordinate axis A6 is an axis used for sixth variable M4'.

Generator 127 maps, at each coordinate in four array maps Mb1, a property value of a compound having a composition formula corresponding to the coordinate. The coordinate is a position indicated by respective values or elements of first variable x, second variable y, third variable a, fourth variable b, fifth variable M3', and sixth variable M4' contained in the composition formula of the compound. Accordingly, multi-array map Mb2 on which a property value is mapped is generated.

In this manner, in the present embodiment, generator 127 generates, for each compound group specified by fifth variable M3' and sixth variable M4', array map Mb1 with respect to a plurality of compounds contained in the compound group. For example, when there are four compound groups, a first compound group of the four compound groups represents a plurality of compounds which correspond to the combination of "fifth variable M3' = element Al" and "sixth variable M4' = element Hf" and which are expressed by composition formula "$Li_{2-3a-4b}(La_{1-x}Al_x)_a(Ti_{1-y}Hf_y)_{1+b}O_3$". A second compound group represents a plurality of compounds which correspond to the combination of "fifth variable M3' = element Al" and "sixth variable M4' = element Zr" and which are expressed by composition formula "$Li_{2-3a-4b}(La_{1-x}Al_x)_a(Ti_{1-y}Zr_y)_{1+b}O_3$". A third compound group represents a plurality of compounds which correspond to the combination of "fifth variable M3' = element In" and "sixth variable M4' = element Hf" and which are expressed by composition formula "$Li_{2-3a-4b}(La_{1-x}In_x)_a(Ti_{1-y}Hf_y)_{1+b}O_3$". A fourth compound group represents a plurality of compounds which correspond to the combination of "fifth variable M3' = element In" and "sixth variable M4' = element Zr" and which are expressed by composition formula "$Li_{2-3a-4b}(La_{1-x}In_x)_a(Ti_{1-y}Zr_y)_{1+b}O_3$".

In addition, generator 127 generates processed image 3 including multi-array map Mb2 made up of four array maps Mb1 by arraying four array maps Mb1 along fifth coordinate axis A5 representing fifth variable M3' and sixth coordinate axis A6 representing sixth variable M4'. Fifth variable M3' is one of two variables other than first variable x to fourth variable b among k-number of (in other words, six) variables, and sixth variable M4' is the other of two variables other than first variable x to fourth variable b among k-number of (in other words, six) variables. Fifth coordinate axis A5 is parallel to third coordinate axis A3 and sixth coordinate axis A6 is parallel to fourth coordinate axis A4. Note that conversely, fifth coordinate axis A5 may be parallel to fourth coordinate axis A4 and sixth coordinate axis A6 may be parallel to third coordinate axis A3.

Furthermore, fifth variable M3' represents an element contained in a compound and sixth variable M4' represents an element contained in the compound which differs from the element represented by fifth variable M3'. The number of array maps Mb1 arrayed along fifth coordinate axis A5 by generator 127 is the number of elements that fifth variable M3' can assume. In addition, the number of array maps Mb1 arrayed along sixth coordinate axis A6 by generator 127 is the number of elements that sixth variable M4' can assume. Note that the elements which sixth variable M4' can assume may be elements determined in advance such as the element Zr and the element Hf illustrated in FIG. 18A or may be elements optionally designated by the user.

Accordingly, even when one or more elements having common characteristics and the component ratios of the one or more elements differ according to the five variables among a plurality of compounds, processed image 3 appropriately showing information relating to the plurality of compounds can be generated.

[Flow of processing by information processing device 120a]

FIG. 19 is a flowchart illustrating an example of the processing operations of information processing device 120a according to the present embodiment.

(Step S21)

First, obtainer 123 obtains variable information 4 in response to, for example, an input operation to input unit 110 by the user and outputs variable information 4 to generator 127.

(Step S22)

Generator 127 obtains variable information 4 from obtainer 123a and generates array map Mb1 constituted of an array of a plurality of maps Ma based on variable information 4. At this point, generator 127 maps a property value of each of a plurality of compounds having a composition determined by k-number of variables included in variable information 4 on array map Mb1. Accordingly, generator 127 generates processed image 3 including array map Mb1 on which the property value is mapped.

(Step S23)

Output processor 126a outputs processed image 3 as an image signal to displayer 130. As a result, processed image 3 is displayed on displayer 130.

[Advantageous effects of Embodiment 2]

As described above, by arraying a plurality of maps Ma showing information of each of a plurality of compounds having a composition determined by k-number of variables based on variable information 4 relating to the k-number of variables, information processing device 120a according to the present embodiment generates and outputs processed image 3 including array map Mb1 constituted of an array of the plurality of maps Ma. For example, when k = 3, maps Ma are expressed by first coordinate axis A1 representing first variable x and second coordinate axis A2 representing second variable y. In addition, the plurality of maps Ma are arrayed according to third variable a.

Accordingly, at positions corresponding to first variable x, second variable y, and third variable a on the plurality of maps Ma, information of a compound with a composition determined by the three variables is mapped. The information of the compound is, for example, a property value of the compound. Therefore, property values and the like of a plurality of compounds having a composition determined by the three variables can be readily comprehended from the plurality of maps Ma. As a result, material search can be made more efficient and material development can be appropriately supported.

In addition, information processing device 120a generates processed image 3 including array map Mb1 by arraying the plurality of maps Ma along third coordinate axis A3 representing third variable a. In addition, third coordinate axis A3 is parallel to first coordinate axis A1 or second coordinate axis A2.

Accordingly, the plurality of maps Ma classified according to third variable a are displayed arrayed in the direction of first coordinate axis A1 or second coordinate axis A2. Therefore, map Ma corresponding to third variable a can be readily found and, furthermore, information on a compound having a composition determined by first variable x, second variable y, and third variable a can be readily comprehended from map Ma.

In addition, each of first variable x, second variable y, and third variable a is a variable that determines a composition coefficient of an element contained in the compound. Furthermore, the number of the plurality of maps Ma arrayed along third coordinate axis A3 is the number of values determined in advance that third variable a can assume.

Accordingly, since each of first variable x, second variable y, and third variable a is a variable that determines a composition coefficient of an element contained in a compound, information of a plurality of compounds having a composition coefficient determined by the three variables can be readily comprehended from the plurality of maps Ma.

In addition, when k = 4, information processing device 120a generates processed image 3 including array map Mb1 by arraying the plurality of maps Ma along third coordinate axis A3 representing third variable a and fourth coordinate axis A4 representing fourth variable b. In this case, third coordinate axis A3 is parallel to first coordinate axis A1 and fourth coordinate axis A4 is parallel to second coordinate axis A2.

Accordingly, the plurality of maps Ma classified according to third variable a and fourth variable b are displayed arrayed in, for example, a matrix pattern in the direction of first coordinate axis A1 and second coordinate axis A2. Therefore, map Ma corresponding to third variable a and fourth variable b can be readily found and, furthermore, information on a compound having a composition determined by first variable x to fourth variable b can be readily comprehended from map Ma.

In addition, each of first variable x to fourth variable b is a variable that determines a composition coefficient of an element contained in the compound. Furthermore, the number of maps Ma arrayed along third coordinate axis A3 is the number of values determined in advance that third variable a can assume, and the number of maps Ma arrayed along fourth coordinate axis A4 is the number of values determined in advance that fourth variable b can assume. Note that an order of the array can be sorted according to feature amounts corresponding to elements. A feature amount is, for example, an atomic weight, a period, or a group. Alternatively, an order may be determined according to a sorting method designated by the user.

Accordingly, since each of first variable x to fourth variable b is a variable that determines a composition coefficient of an element contained in a compound, a property value of a plurality of compounds having a composition coefficient determined by the four variables can be readily comprehended from the plurality of maps Ma.

In addition, when k = 4, a composition formula of each of a plurality of compounds may be expressed by "$A_p D_q E_r$". In the composition formula, A denotes at least one element, D denotes a third variable representing an element, E denotes a fourth variable representing an element, and p denotes a composition coefficient. Furthermore, q denotes a first variable representing a composition coefficient of the element denoted by D and r denotes a second variable representing a composition coefficient of the element denoted by E. In this case, the number of maps Ma arrayed along third coordinate axis A3 is the number of elements determined in advance that the third variable can assume, and the number of maps Ma arrayed along fourth coordinate axis A4 is the number of elements determined in advance that the fourth variable can assume.

Accordingly, first variable q and second variable r are variables that determine a composition coefficient of an element contained in the compound, and third variable D and fourth variable E are variables that determine an element contained in the compound. Therefore, as many maps Ma corresponding to combinations of elements that can be respectively assumed by third variable D and fourth variable E as the number of the combinations can be arrayed in, for example, a matrix pattern. In addition, map Ma corresponding to a combination of elements respectively determined by third variable D and fourth variable E can be readily found. Furthermore, information on a compound having a composition determined by first variable q to fourth variable E can be readily comprehended from map Ma.

In addition, when k = 5, information processing device 120a generates, for each compound group specified by fifth variable M3', array map Mb1 with respect to a plurality of compounds contained in the compound group. Furthermore, information processing device 120a generates processed image 3 including multi-array map Mb2 made up of a plurality of array maps Mb1 by arraying the plurality of array maps Mb1 along fifth coordinate axis A5 representing fifth variable M3'. Fifth coordinate axis A5 is parallel to one of third coordinate axis A3 and fourth coordinate axis A4.

Accordingly, the plurality of array maps Mb1 classified according to fifth variable M3' are displayed arrayed in the direction of first coordinate axis A1 or second coordinate axis A2 or, in other words, the direction of third coordinate axis A3 or fourth coordinate axis A4. Therefore, array map Mb1 corresponding to fifth variable M3' can be readily found and, furthermore, information on a compound having a composition determined by first variable x to fifth variable M3' can be readily comprehended from array map Mb1.

In addition, fifth variable M3' represents an element contained in a compound and the number of array maps Mb1 arrayed along fifth coordinate axis A5 is the number of elements determined in advance that fifth variable M3' can assume.

Accordingly, since fifth variable M3' is a variable that determines an element contained in a compound, array map Mb1 corresponding to a plurality of compounds respectively having the element determined by fifth variable M3' can be readily found from a plurality of array maps Mb1. Furthermore, information on a compound having a composition determined by first variable x to fifth variable M3' can be readily comprehended from array map Mb1.

In addition, when k = 6, information processing device 120a generates, for each compound group specified by fifth variable M3' and sixth variable M4', array map Mb1 with respect to a plurality of compounds contained in the compound group. In addition, information processing device 120a generates processed image 3 including multi-array map Mb2 made up of a plurality of array maps Mb1 by arraying the plurality of array maps Mb1 along fifth coordinate axis A5 representing fifth variable M3' and sixth coordinate axis A6 representing sixth variable M4'. Fifth coordinate axis A5 is parallel to one of third coordinate axis A3 and fourth coordinate axis A4 and sixth coordinate axis A6 is parallel to the other coordinate axis.

Accordingly, the plurality of array maps Mb1 classified according to fifth variable M3' and sixth variable M4' are displayed arrayed in, for example, a matrix pattern in the direction of first coordinate axis A1 and second coordinate axis A2 or, in other words, the direction of third coordinate axis A3 and fourth coordinate axis A4. Therefore, array map Mb1 corresponding to fifth variable M3' and sixth variable M4' can be readily found and, furthermore, information on a compound having a composition determined by first variable x to sixth variable M4' can be readily comprehended from array map Mb1.

In addition, each of fifth variable M3' and sixth variable M4' is a variable that represents an element contained in the compound. Furthermore, the number of array maps Mb1 arrayed along fifth coordinate axis A5 is the number of elements determined in advance that fifth variable M3' can assume, and the number of array maps Mb1 arrayed along sixth coordinate axis A6 is the number of elements determined in advance that sixth variable M4' can assume.

Accordingly, since each of fifth variable M3' and sixth variable M4' is a variable that determines an element contained in a compound, array map Mb1 corresponding to a plurality of compounds respectively having the element represented by fifth variable M3' and the element represented by sixth variable M4' can be readily found from a plurality of array maps Mb1. Furthermore, information on a compound having a composition determined by first variable x to sixth variable M4' can be readily comprehended from array map Mb1.

<Other aspects>

Although the various information processing devices according to the present disclosure have been described on the basis of Embodiments 1 and 2, the present disclosure is not limited to these embodiments. Forms obtained by applying various modifications to Embodiment 1 or 2 that may be conceived by those skilled in the art, forms obtained by combining the constituent elements in different embodiments, and the like are also included in the scope of the present disclosure insofar as such embodiments do not depart from the essence of the present disclosure.

For example, while information processing device 120 displays processed image 2 including a graph which has coordinate axes determined according to an attribute and on which a coordinate point of each compound is plotted in the first embodiment, a property value as described in the second embodiment may be superimposed on the graph. Specifically, a property value expressed by a color or a shade of color may be superimposed on each coordinate shown in the graph.

In addition, a plurality of graphs generated in the first embodiment may be displayed arrayed as in the second embodiment using a coordinate axis or a variable not used in the graphs.

Furthermore, while an axis variable (in other words, an abscissa variable or an ordinate variable) used for a coordinate axis is determined and a graph having the coordinate axis is generated in the first embodiment, a correspondence relationship between the axis variable and the coordinate axis may be determined in advance. Specifically, an axis variable based on a first principal component may be determined in advance to be used for the abscissa axis and an axis variable based on a second principal component may be determined in advance to be used for the ordinate axis.

In addition, information processing device 120a obtains variable information 4 in the second embodiment. Variable information 4 is information relating to k-number of variables that determine a composition of a compound. Therefore, variable information 4 may also be described as information relating to a plurality of compounds and indicating, with respect to each of the plurality of compounds, contents relating to two or more elements constituting the compound and a component ratio of the two or more elements. Furthermore, in the second embodiment, information processing device 120a generates processed image 3 including array map Mb1 by arraying a plurality of maps Ma showing information of each of a plurality of compounds having a composition determined by the k-number of variables based on variable information 4. At this point, information processing device 120a has discriminated an attribute of a group to which the plurality of compounds belong by specifying the number k based on variable information 4. In other words, information processing device 120a has distinguished whether the attribute of the group to which the plurality of compounds belong is an attribute of k = 3, an attribute of k = 4, an attribute of k = 5, or an attribute of k = 6. In addition, information processing device 120a determines a display method of variable information 4 according to the distinguished attribute and outputs variable information 4 according to the determined display method. The display method of variable information 4 is a method of arranging a plurality of maps Ma or the like.

Therefore, even with information processing device 120a according to the second embodiment, variable information 4 is obtained as material information 1, an attribute of a group to which a plurality of compounds belong is distinguished based on material information 1, a display method of material information 1 is determined in accordance with the distinguished attribute, and material information 1 is outputted according to the determined display method in a similar manner to information processing device 120 according to the first embodiment.

Furthermore, while the information processing devices according to the respective embodiments described above constitute a part of an information processing system and processing system 1000, the information processing devices may include all of the elements of the information processing system or processing system 1000. For example, information processing device 120 illustrated in FIG. 2A may include input unit 110 and displayer 130. In addition, the information processing may also include a plurality of processors. Furthermore, the information processing device may be implemented as a single computer device or may be implemented as computer devices communicably connected to each other. In other words, the plurality of constituent elements included in the information processing devices according to the respective embodiments described above need not be included in a same single device and may be distributed among and arranged in mutually different devices.

Furthermore, while each constituent element is constituted of dedicated hardware in the respective embodiments described above, each constituent element may be realized by executing a software program suitable for each constituent element. Each of the constituent elements may be realized by a program executer such as a CPU or a processor reading and executing a software program recorded in a recording medium such as a hard disk or a semiconductor memory. In this case, the program for achieving the information processing devices and the like according to the respective embodiments described above may cause a processor to execute the respective steps included in at least one of the flowcharts illustrated in FIG. 12, FIG. 13A, FIG. 13B, and FIG. 19.

(Hardware configuration)

Specifically, processing system 1000 described above may be configured by a computer system composed of, for example, a microprocessor, a ROM, a RAM, a hard disk drive, a display unit, a keyboard, and a mouse. The RAM or the hard disk drive stores a program. The microprocessor operates according to the program, so that the functions of processing system 1000 are achieved. Here, the program includes a plurality of instruction codes indicating instructions to be given to the computer so as to achieve a specific function.

In addition, some or all of the constituent elements included in processing system 1000 described above may be realized as a single system large scale integration (LSI). The system LSI is a super multifunctional LSI manufactured by integrating a plurality of constituent elements onto a signal chip. To be more specific, the system LSI is a computer system configured with a microprocessor, a ROM, and a RAM, for example. The RAM stores a computer program. The microprocessor operates according to the computer program, so that a function of the system LSI is achieved.

Furthermore, some or all of the elements included in processing system 1000 described above may be implemented as an IC card or standalone module that can be inserted into and removed from a computer. The IC card or the module is a computer system configured with a microprocessor, a ROM, a RAM, and the like, for example. The IC card or the module may include the aforementioned super multifunctional LSI. The microprocessor operates according to the computer program, so that a function of the IC card or the module is achieved. The IC card or the module may be tamper-resistant.

Furthermore, the present disclosure may be an information processing method executed by processing system 1000 described above. Furthermore, this information processing method may be realized by a computer executing a program, or may be realized by a digital signal of the program.

Moreover, the present disclosure may be may be the program or digital signal recorded on a non-transitory computer-readable recording medium. The recording medium is, for example, a flexible disk, a hard disk, a CD-ROM, an MO, a DVD, a DVD-ROM, a DVD-RAM, a Blu-ray (registered trademark) disc (BD), or a semiconductor memory. The program may be the digital signal recorded on a non-transitory recording medium.

Furthermore, the present disclosure may be the aforementioned program or digital signal transmitted via a telecommunication line, a wireless or wired communication line, a network represented by the Internet, data broadcasting, or the like.

Moreover, the present disclosure may be a computer system including a microprocessor and a memory. The memory may store the aforementioned program and the microprocessor may operate according to the program.

Furthermore, by transferring the recording medium having the aforementioned program or digital signal recorded thereon or by transferring the aforementioned program or digital signal via the aforementioned network or the like, the present disclosure may be implemented by a different independent computer system.

Furthermore, processing system 1000 may be implemented by a server and a terminal that is in the possession of a user and is connected to the server via a network.

[Industrial Applicability]

The present disclosure produces the advantageous effect of being able to display information related to compounds in an easily understandable manner.

[Reference Signs List]

    1 material information (composition list)
    1a to 1d composition list
    2, 3 processed image
    2a message information
    2b principal component analysis result
    2c, 2d first attribute processed image
    2e second attribute processed image
    4 variable information
    60 to 67, 71, 72 graph
    100, 100a information processing system
    110, 510 input unit
    120,120a information processing device
    121, 121a, 521 communication unit
    122, 122a, 522 limiter
    123, 123a, 523 obtainer
    124 attribute distinguisher
    125 display method determiner
    126, 126a, 526 output processor
    127 generator
    130, 530 displayer
    500 terminal system
    520 terminal device
    600 database
    1000 processing system
    nt communication network

**Claims**

1. An information processing method performed by a computer, the information processing method comprising:

obtaining material information that is information relating to compounds and indicating, for each of the compounds, two or more elements making up the compound and information relating to a component ratio of the two or more elements in the compound;

distinguishing an attribute of a group to which the compounds belong, based on the material information;

determining a display method for the material information according to the attribute distinguished; and

executing an outputting process of outputting the material information according to the display method determined.

**2.** The information processing method according to claim 1, wherein

in the executing of the outputting process, the outputting process is performed by generating an image showing, for each of the compounds, coordinates derived, according to the display method, from at least one of (i) the two or more elements making up the compound or (ii) the component ratio of the two or more elements in the compound, and outputting the image.

**3.** The information processing method according to claim 2, wherein

in the distinguishing of the attribute, the attribute is distinguished based on a degree of freedom regarding compositions of the compounds, the compositions being identified from the material information.

**4.** The information processing method according to claim 3, wherein

in the distinguishing of the attribute:

the attribute is distinguished as a first attribute when the degree of freedom is less than or equal to a first threshold;

the attribute is distinguished as a second attribute when the degree of freedom is greater than the first threshold and less than or equal to a second threshold; and

the attribute is distinguished as a third attribute when the degree of freedom is greater than the second threshold, and

the second threshold is a value greater than the first threshold.

**5.** The information processing method according to claim 3 or claim 4, wherein

the determining of the display method includes:

for each of independent variables, determining, as a coordinate axis included in the image, a coordinate axis indicating the independent variable, a total number of the independent variables being dependent on the degree of freedom, and

in the executing of the outputting process, the outputting process is performed by generating the image including the coordinate axis determined, and outputting the image.

**6.** The information processing method according to any one of claims 3 to 5, further comprising:

deriving, as the degree of freedom, a total number of principal components having a cumulative contribution ratio exceeding 99 percent, by performing a principal component analysis on the compounds.

**7.** The information processing method according to claim 4, wherein

in the distinguishing of the attribute, the attribute is distinguished as the second attribute when the degree of freedom is greater than the first threshold and less than or equal to the second threshold and a contribution ratio of a first principal component obtained by performing a principal component analysis on the compounds is at least 1.5 times a contribution ratio of a second principal component.

**8.** The information processing method according to claim 2, wherein

in the distinguishing of the attribute:

the attribute is distinguished as a first attribute when each of the compounds is a solid solution;

the attribute is distinguished as a second attribute when each of the compounds includes a dopant; and

the attribute is distinguished as a third attribute when each of the compounds is not a solid solution and does not include a dopant.

9. The information processing method according to claim 2 or claim 8, wherein
in the distinguishing of the attribute:

at least one type of feature for the compounds is identified based on a variation in the component ratio of the two or more elements included in each of the compounds;
the attribute is distinguished as a first attribute when the at least one type of feature satisfies a first condition;
the attribute is distinguished as a second attribute when the at least one type of feature satisfies a second condition; and
the attribute is distinguished as a third attribute when the at least one type of feature satisfies a third condition.

10. The information processing method according to claim 9, wherein

in the obtaining of the material information, for each of the compounds, the material information indicating a normalized component ratio of the two or more elements is obtained,
in the distinguishing of the attribute, a principal component analysis is performed on the compounds using the normalized component ratio of the two or more elements, and the attribute is distinguished as the first attribute, the second attribute, or the third attribute based on a result of the principal component analysis, and
in the determining of the display method, when the attribute is distinguished as the first attribute or the second attribute, the display method of the material information is determined by determining, as the coordinate axis included in the image, a coordinate axis indicating variables obtained from the result of the principal component analysis.

11. The information processing method according to claim 10, wherein

when the attribute is distinguished as the first attribute in the distinguishing of the attribute,
in the determining of the display method:
each of one or more coefficients to be used for an n-th principal component obtained in the principal component analysis is discretized, where n is an integer greater than or equal to 1; and
a coordinate axis indicating a variable represented based on the one or more coefficients discretized is determined as the coordinate axis included in the image.

12. The information processing method according to any one of claims 2 and 8 to 11, wherein
the executing of the outputting process includes:

calculating a convex hull for the coordinates of each of the compounds;
superimposing, on the image, composition information indicating a composition of a compound corresponding to a vertex of the convex hull calculated; and
outputting the image on which the composition information is superimposed.

13. The information processing method according to claim 10 or claim 11, wherein

when the attribute is distinguished as the second attribute in the distinguishing of the attribute,
in the determining of the display method:

elements included in the compounds are classified into one or more first elements and one or more second elements, based on one or more coefficients to be used for a first principal component obtained in the principal component analysis;
at least one element from the one or more second elements is identified as at least one added element; and
for each of the at least one added element, a coordinate axis indicating the at least one added element as a composition coefficient is determined as the coordinate axis included in the image,

in the first principal component, an absolute value of a coefficient of each of the one or more second elements is smaller than an absolute value of a coefficient of each of the one or more first elements, and
a variance of the composition coefficients of each of the at least one added element is greater than 0.

14. An information processing method performed by a computer, the information processing method comprising:

obtaining variable information relating to k variables used for representing a composition of a compound, where

**EP 4 425 503 A1**

k is an integer greater than or equal to 3 and less than or equal to 6;
generating an image including an array map composed of an array of maps, by arraying the maps based on the variable information, the maps indicating information of compounds each having a composition represented by the k variables; and
outputting the image, wherein
the maps are each represented by a first coordinate axis indicating a first variable included in the k variables and a second coordinate axis indicating a second variable included in the k variables, and
the maps included in the array map are arrayed according to a third variable other than the first variable and the second variable among the k variables.

15. The information processing method according to claim 14, wherein

in the generating of the image, the image including the array map is generated by arraying the maps along a third coordinate axis indicating the third variable, and
the third coordinate axis is parallel to the first coordinate axis or the second coordinate axis.

16. The information processing method according to claim 15, wherein

the first variable, the second variable, and the third variable are each a variable used for representing a composition coefficient of an element included in the compound, and
a total number of the maps that are arrayed along the third coordinate axis in the generating of the image is a value that can be assumed by the third variable.

17. The information processing method according to claim 14, wherein

in the generating of the image, the image including the array map is generated by arraying the maps along a third coordinate axis indicating the third variable and a fourth coordinate axis indicating a fourth variable,
the fourth variable is a variable other than the first variable, the second variable, and the third variable among the k variables,
the third coordinate axis is parallel to the first coordinate axis, and
the fourth coordinate axis is parallel to the second coordinate axis or orthogonal to the third coordinate axis.

18. The information processing method according to claim 17, wherein

the first variable, the second variable, and the third variable, and the fourth variable are each a variable used for representing a composition coefficient of an element included in the compound, and
a total number of the maps that are arrayed along the fourth coordinate axis in the generating of the image is a value that can be assumed by the fourth variable.

19. The information processing method according to claim 17, wherein

a composition formula of each of the compounds is represented by ApDqEr, where:

A in the composition formula denotes at least one element;
D in the composition formula is the third variable indicating an element;
E in the composition formula is the fourth variable indicating an element;
p in the composition formula is a composition coefficient of the at least one element indicated by A;
q in the composition formula is the first variable indicating a composition coefficient of the element indicated by D; and
r in the composition formula is the second variable indicating a composition coefficient of the element indicated by E,

a total number of maps arrayed along the third coordinate axis in the generating of the image is equal to a total number of elements that can be assumed by the third variable, and
a total number of maps arrayed along the fourth coordinate axis in the generating of the image is a total number of elements that can be assumed by the fourth variable.

20. The information processing method according to claim 18, wherein

in the generating of the image:

> for each compound group identified by a fifth variable, the array map is generated for the compounds included in the compound group; and
> the image including a multi-array map composed of the array maps is generated by arraying the array maps along a fifth coordinate axis indicated by the fifth variable, wherein

the fifth variable is a variable other than the first variable, the second variable, the third variable, and the fourth variable among the k variables, and
the fifth coordinate axis is parallel to one of the third coordinate axis or the fourth coordinate axis.

21. The information processing method according to claim 20 wherein

the fifth variable indicates an element included in the compound, and
a total number of the array maps that are arrayed along the fifth coordinate axis in the generating of the image is a total number of elements that can be assumed by the fifth variable.

22. The information processing method according to claim 18 wherein

in the generating of the image:

> for each compound group identified by a fifth variable and a sixth variable, the array map is generated for the compounds included in the compound group; and
> the image including a multi-array map composed of the array maps is generated by arraying the array maps along a fifth coordinate axis indicated by the fifth variable and a sixth coordinate axis indicated by the sixth variable, wherein

the fifth variable is one of two variables other than the first variable to the fourth variable among the k variables,
the sixth variable is a remaining one of the two variables,
the fifth coordinate axis is one of the third coordinate axis or the fourth coordinate axis, and
the sixth coordinate axis is a remaining one of the third coordinate axis or the fourth coordinate axis.

23. The information processing method according to claim 22 wherein

the fifth variable indicates an element included in the compound,
the sixth variable indicates an element included in the compound and different from the element indicated by the fifth variable,
a total number of the array maps that are arrayed along the fifth coordinate axis in the generating of the image is a total number of elements that can be assumed by the fifth variable, and
a total number of the array maps that are arrayed along the sixth coordinate axis in the generating of the image is a total number of elements that can be assumed by the sixth variable.

24. An information processing device comprising:

an obtainer that obtains material information that is information relating to compounds and indicating, for each of the compounds, two or more elements making up the compound and information relating to a component ratio of the two or more elements in the compound;
attribute distinguisher that distinguishes an attribute of a group to which the compounds belong, based on the material information;
a display method determiner that determines a display method for the material information according to the attribute distinguished; and
an output processor that outputs the material information according to the display method determined.

25. An information processing device comprising:

an obtainer that obtains variable information relating to k variables used for representing a composition of a compound, where k is an integer greater than or equal to 3 and less than or equal to 6;
a generator that generates an image including an array map composed of an array of maps by arraying the maps, based on the variable information, the maps indicating information of compounds each having a composition represented by the k variables; and

an output processor that outputs the image, wherein

the maps are each represented by a first coordinate axis indicating a first variable included in the k variables and a second coordinate axis indicating a second variable included in the k variables, and

the maps included in the array map are arrayed according to a third variable other than the first variable and the second variable among the k variables.

26. A program for causing a computer to execute:

obtaining material information that is information relating to compounds and indicating, for each of the compounds, two or more elements making up the compound and information relating to a component ratio of the two or more elements in the compound;

distinguishing an attribute of a group to which the compounds belong, based on the material information;

determining a display method for the material information according to the attribute distinguished; and

outputting an image showing the material information according to the display method determined.

27. A program for causing a computer to execute:

obtaining variable information relating to k variables used for representing a composition of a compound, where k is an integer greater than or equal to 3 and less than or equal to 6;

generating an image including an array map composed of an array of maps by arraying the maps, based on the variable information, the maps indicating information of compounds each having a composition represented by the k variables; and

outputting the image, wherein

the maps are each represented by a first coordinate axis indicating a first variable included in the k variables and a second coordinate axis indicating a second variable included in the k variables, and

the maps included in the array map are arrayed according to a third variable other than the first variable and the second variable among the k variables.

FIG. 1

# FIG. 2A

100

110

| Input unit |

120

Information processing device

123
| Obtainer |

124
| Attribute distinguisher |

125
| Display method determiner |

126
| Output processor |

121
| Communicator |

122
| Controller |

| Displayer | 130

# FIG. 2B

500

510

| Input unit |

520

Terminal device

523
| Obtainer |

526
| Output processor |

521
| Communicator |

522
| Controller |

| Displayer | 530

# FIG. 3A

1 (1a)

| formula |
|---|
| Li1.55Hf0.8Zr0.2In0.105Ga0.045O3 |
| Li1Hf0.99Zr0.11In0.1Ga0.1O3 |
| Li0.35Hf0.39Zr0.91In0.03Ga0.12O3 |
| Li1.6Zr1.1O3.0 |
| Li0.65Hf1.3In0.015Ga0.035O3 |
| Li1Hf0.11Zr0.99In0.1Ga0.1O3 |
| Li1Hf0.44Zr0.66In0.18Ga0.02O3 |
| Li1.45Hf0.11Zr0.99In0.03Ga0.02O3 |
| Li1Hf0.77Zr0.33In0.14Ga0.06O3.0 |
| Li0.5Hf0.78Zr0.52In0.01Ga0.09O3 |
| Li0.5Hf0.52Zr0.78In0.05Ga0.05O3 |
| Li1.7Hf0.9Zr0.1In0.1O3 |
| Li1Hf1.1In0.12Ga0.08O3 |
| Li0.35Hf1.04Zr0.26In0.15O3 |
| Li1.3Hf0.77Zr0.33In0.09Ga0.01O3.0 |
| Li0.2Zr1.3In0.04Ga0.16O3 |
| Li0.65Hf0.91Zr0.39In0.045Ga0.005O3.0 |
| Li0.6Hf0.72Zr0.48In0.16Ga0.04O3 |
| Li1.15Hf0.77Zr0.33In0.03Ga0.12O3.0 |
| Li0.65Hf1.04Zr0.26In0.035Ga0.015O3 |

# FIG. 3B

1 (1b)

| formula |
|---|
| Cr0.006Fe0.986Ni0.008 |
| Nb0.009V0.01Fe0.981 |
| Nb0.006Cr0.01Fe0.984 |
| Ti0.009Al0.005Fe0.986 |
| Ti0.008Fe0.987Mo0.005 |
| Nb0.009Fe0.982Ni0.009 |
| Nb0.006Al0.005Fe0.989 |
| Nb0.005V0.009Fe0.986 |
| Nb0.003Al0.002Fe0.995 |
| Nb0.007Fe0.985Mo0.008 |
| Ti0.01Mn0.003Fe0.987 |
| Ti0.007V0.006Fe0.987 |
| Fe0.998Cu0.001Mo0.001 |
| Ti0.002Cr0.004Fe0.994 |
| Al0.003V0.004Fe0.993 |
| Fe0.997Ni0.003 |
| Ti0.002V0.005Fe0.993 |
| V0.009Cr0.004Fe0.987 |

# FIG. 3C

1 (1c)

| formula |
| --- |
| PdKRh3 |
| TlTaMn3 |
| BrIrCs3 |
| ScBeSe3 |
| ZrLaSr3 |
| TlNaCl3 |
| SiCaPd3 |
| ZnNCl3 |
| SnHfLa3 |
| LiKF3 |
| OsLaCl3 |
| LaMoP3 |
| RhPI3 |
| TcMoHg3 |
| PdReSr3 |
| YHfSc3 |
| TiPPt3 |
| CNBr3 |

# FIG. 3D

1 (1d)

| formula | value |
|---|---|
| Li1.55Hf0.8Zr0.2In0.105Ga0.045O3 | 0.63 |
| Li1Hf0.99Zr0.11In0.1Ga0.1O3 | 0.63 |
| Li0.35Hf0.39Zr0.91In0.03Ga0.12O3 | 0.18 |
| Li1.6Zr1.1O3.0 | 0.01 |
| Li0.65Hf1.3In0.015Ga0.035O3 | 0.32 |
| Li1Hf0.11Zr0.99In0.1Ga0.1O3 | 0.39 |
| Li1Hf0.44Zr0.66In0.18Ga0.02O3 | 0.03 |
| Li1.45Hf0.11Zr0.99In0.03Ga0.02O3 | 0.98 |
| Li1Hf0.77Zr0.33In0.14Ga0.06O3.0 | 0.45 |
| Li0.5Hf0.78Zr0.52In0.01Ga0.09O3 | 0.37 |
| Li0.5Hf0.52Zr0.78In0.05Ga0.05O3 | 1.00 |
| Li1.7Hf0.9Zr0.1In0.1O3 | 0.34 |
| Li1Hf1.1In0.12Ga0.08O3 | 0.25 |
| Li0.35Hf1.04Zr0.26In0.15O3 | 0.29 |
| Li1.3Hf0.77Zr0.33In0.09Ga0.01O3.0 | 0.39 |
| Li0.2Zr1.3In0.04Ga0.16O3 | 0.70 |
| Li0.65Hf0.91Zr0.39In0.045Ga0.005O3.0 | 0.09 |
| Li0.6Hf0.72Zr0.48In0.16Ga0.04O3 | 0.23 |
| Li1.15Hf0.77Zr0.33In0.03Ga0.12O3.0 | 0.95 |
| Li0.65Hf1.04Zr0.26In0.035Ga0.015O3 | 0.55 |

**FIG. 4A**

First attribute

**FIG. 4B**

Second attribute

**FIG. 4C**

Third attribute

# FIG. 5

# FIG. 6A

Magnitude of
element of first
principal component

Principal elements

# FIG. 6B

Mean value of
composition
coefficients

Principal element
(mean value > 0, variance = 0)

Variance of
composition
coefficients

Principal
elements

Added elements

# FIG. 7A

First attribute: $Li_{2-3a-4b}(Ga_{1-x}In_x)_a(Zr_{1-y}Hf_y)_{1+b}O_3$ 60

Increase in Zr, Hf, Li

Increase in Ga, In

(Zr, Hf, Li)-(In, Ga)

(In)-(Ga)

Increase in Ga ⟷ Increase in In

# FIG. 7B

First attribute: $Li_{2-3a-4b}(Ga_{1-x}In_x)_a(Zr_{1-y}Hf_y)_{1+b}O_3$ 61

Fourth principal component

Third principal component

## FIG. 8A

## FIG. 8B

FIG. 9

EP 4 425 503 A1

# FIG. 10A

2

┌─────────────────────────────────────┐
│ Input data needs to be reviewed │ ～2a
└─────────────────────────────────────┘

Principal component analysis result ～2b

Component No.

2e

Second attribute
processed image

2c

First attribute processed
image
[corresponding to first
principal component
and second principal
component]

2d

First attribute processed
image
[corresponding to third
principal component
and fourth principal
component]

EP 4 425 503 A1

# FIG. 10B

First attribute processed image
[corresponding to first principal component
and second principal component]

(Ir, Cd, Pd, K, Si, Ba)
-(P, Sb, Te, B, W, Ca, Hf, Zn, Ti, Cs, Se, Y, Nb, Re, Tc, Sc)

# FIG. 10C

First attribute processed image
[corresponding to third principal component
and fourth principal component]

(P, Sb, Pd, Sn, B, Be, H, V, K, W, O)
-(Te, Hf, Cs, Re, Y, Ba, Nb, In, Tc, Se, Hg, Ge, Zn, Sr, Rh, Ta, Ir, S, Li)

(Te, Hf, B, W, V, H, Pd, Nb, Ca, Ir, Ta, P, Al)
-(Sn, Y, Hg, Pt, Se, Co, Be, Re, Sc, K, Cs, F, Ti,
Ru, Mn, Os, Na, S, Tc, Mg, Pb, Cl)

# FIG. 10D

Second attribute processed image
Principal elements: Ir, Cd, Pd Added elements: 10 out of 61 displayed

2e

67

# FIG. 11

Display according to principal component analysis

Display suitable for attribute of composition list

Composition List A

formula

Li1.55Hf0.8Zr0.2In0.105Ga0.045O3
Li1Hf0.99Zr0.11In0.1Ga0.1O3
Li0.35Hf0.39Zr0.91In0.03Ga0.12O3
Li1.6Zr1.1O3.0

Accumulated data

Composition List A

Composition List B

Composition List C

Composition List B

formula

Cr0.006Fe0.986Ni0.008
Nb0.009V0.01Fe0.981
Nb0.006Cr0.01Fe0.984
Ti0.009Al0.005Fe0.986
Ti0.008Fe0.987Mo0.005

Increase in Zr, Hf, Li

$(Zr, Hf, Li)-(In, Ga)$

Increase in Ga, In

Increase in Ga

$(In)-(Ga)$

Increase in In

$Fe_{1-x-y}M_xM'_y$

# FIG. 12

```
            ┌──────────┐
            │  Start   │
            └────┬─────┘
                 │
                 ▼
    ┌───────────────────────────┐
    │ Obtain material information│  ～S10
    └─────────────┬─────────────┘
                  │
                  ▼
    ┌───────────────────────────┐
    │ Distinguish attribute based│  ～S11
    │ on material information    │
    └─────────────┬─────────────┘
                  │
                  ▼
    ┌───────────────────────────┐
    │ Determine display method   │  ～S12
    │ according to distinguished │
    │ attribute                  │
    └─────────────┬─────────────┘
                  │
                  ▼
    ┌───────────────────────────┐
    │ Generate Image according   │  ～S13
    │ to display method          │
    └─────────────┬─────────────┘
                  │
                  ▼
    ┌───────────────────────────┐
    │      Output image          │  ～S14
    └─────────────┬─────────────┘
                  │
                  ▼
            ┌──────────┐
            │   End    │
            └──────────┘
```

# FIG. 13A

# FIG. 13B

```
                        ┌─────────┐
                        │  Start  │ ╱S12
                        └────┬────┘
                             │          S121
                             ▼         ╱
        First attribute  ╱Is attribute╲   Third attribute
      ┌─────────────────╱ first attribute, second╲─────────────────┐
      │                 ╲ attribute, or third    ╱                 │
      │                  ╲    attribute?        ╱                   │
      │                   ╲──────┬─────────────╱                    │
Determine           Determine  │Second              Determine
first display       second display│attribute        third display
method              method      ▼      ╱S124         method          ╱S128
┌──────────────┐   ┌──────────────────────┐    ┌──────────────────┐
│Processing of │   │Select first primary  │    │Determine message │
│n-th principal│   │element based on each │    │information       │
│component     │   │element of first      │    └──────────────────┘
│(n=1; n≤4; n++)│  │principal component   │
└──────┬───────┘   └──────────┬───────────┘
       │    ╱S122             │        ╱S125
       ▼                      ▼
┌──────────────┐   ┌──────────────────────┐
│Obtain n-th   │   │Select element of which│
│principal     │   │mean of composition   │
│component and │   │coefficients >        │
│discretize    │   │0 and variance = 0 as │
│each element  │   │second primary element│
└──────┬───────┘   └──────────┬───────────┘
       │    ╱S123             │        ╱S126
       ▼                      ▼
┌──────────────┐   ┌──────────────────────┐
│Determine axis│   │Determine element other│
│variable based│   │than first primary    │
│on each       │   │element and second    │
│discretized   │   │primary element as    │
│element       │   │added element         │
└──────┬───────┘   └──────────┬───────────┘
       │                      │        ╱S127
       ▼                      ▼
┌──────────────┐   ┌──────────────────────┐
│Processing of │   │Determine axis variable│
│n-th principal│   │representing composition│
│component)    │   │coefficient of added  │
└──────┬───────┘   │element               │
       │           └──────────┬───────────┘
       │                      │                                     │
       └──────────────────────┼─────────────────────────────────────┘
                              ▼
                        ┌─────────┐
                        │ Output  │
                        └─────────┘
```

EP 4 425 503 A1

# FIG. 14

# FIG. 15A

4

Compound: $Li_{2-3a}(La_{1-x}Al_x)_a(Ti_{1-y}Zr_y)O_3$

| Variable | Range of application |
|---|---|
| a | 0.0, 0.05, 0.1, 0.15, 0.2 |
| x | 0.0, 0.1, 0.2, 0.3, 0.4, 0.5, 0.6, 0.7, 0.8, 0.9, 1.0 |
| y | 0.0, 0.1, 0.2, 0.3, 0.4, 0.5, 0.6, 0.7, 0.8, 0.9, 1.0 |

# FIG. 15B

# FIG. 16A

Compound: $Li_{2-3a-4a}(La_{1-x}Al_x)_a(Ti_{1-y}Zr_y)_{1+b}O_3$ — 4

| Variable | Range of application |
|----------|---------------------|
| a | 0.0, 0.05, 0.1, 0.15, 0.2 |
| b | 0.0, 0.1, 0.2, 0.3 |
| x | 0.0, 0.1, 0.2, 0.3, 0.4, 0.5, 0.6, 0.7, 0.8, 0.9, 1.0 |
| y | 0.0, 0.1, 0.2, 0.3, 0.4, 0.5, 0.6, 0.7, 0.8, 0.9, 1.0 |

# FIG. 16B

# FIG. 16C

Compound: $M1(M2_{1-x-y}M2'_xM2''_y)$

| Variable | Range of application |
|---|---|
| x | 0.0, 0.1, 0.2, 0.3, 0.4, 0.5, 0.6, 0.7, 0.8, 0.9, 1.0 |
| y | 0.0, 0.1, 0.2, 0.3, 0.4, 0.5, 0.6, 0.7, 0.8, 0.9, 1.0 |
| M1 | Se, O, As, N |
| M (M2, M2', M2'') | Ge, Zn, Si, Cd, In (Ge-Zn-Si, Ge-Cd-Si, Ge-In-Si, Zn-Cd-Si, Zn-In-Si, Cd-In-Si, Zn-Cd-Ge, Zn-In-Ge, Cd-In-Ge, Cd-In-Zn) |

Where $x + y \leq 1$

# FIG. 16D

# FIG. 17A

4

Compound: $Li_{2-3a-4b}(La_{1-x}M3'_x)_a(Ti_{1-y}Zr_y)_{1+b}O_3$

| Variable | Range of application |
|---|---|
| a | 0.0, 0.05, 0.1, 0.15, 0.2 |
| b | 0.0, 0.1, 0.2, 0.3 |
| x | 0.0, 0.1, 0.2, 0.3, 0.4, 0.5, 0.6, 0.7, 0.8, 0.9, 1.0 |
| y | 0.0, 0.1, 0.2, 0.3, 0.4, 0.5, 0.6, 0.7, 0.8, 0.9, 1.0 |
| M3' | Al, In |

$Li_{2-3a-4b}(La_{1-x}Al_x)_a(Ti_{1-y}Zr_y)_{1+b}O_3$

$Li_{2-3a-4b}(La_{1-x}In_x)_a(Ti_{1-y}Zr_y)_{1+b}O_3$

EP 4 425 503 A1

# FIG. 18A

Compound: $Li_{2-3a-4b}(M3_{1-x}M3'_x)_a(M4_{1-y}M4'_y)_{1+b}O_3$

| Variable | Range of application |
|----------|----------------------|
| a | 0.0, 0.05, 0.1, 0.15, 0.2 |
| b | 0.0, 0.1, 0.2, 0.3 |
| x | 0.0, 0.1, 0.2, 0.3, 0.4, 0.5, 0.6, 0.7, 0.8, 0.9, 1.0 |
| y | 0.0, 0.1, 0.2, 0.3, 0.4, 0.5, 0.6, 0.7, 0.8, 0.9, 1.0 |
| M3 | La |
| M3′ | Al, In |
| M3, M3′ | La-Al, La-In |
| M4 | Ti |
| M4′ | Zr, Hf |
| M4, M4′ | Ti-Zr, Ti-Hf |

# FIG. 18B

# FIG. 19

```
        ┌─────────────┐
        │    Start    │
        └──────┬──────┘
               │
               ▼
    ┌──────────────────────┐
    │   Obtain variable    │ ～S21
    │   information        │
    └──────────┬───────────┘
               │
               ▼
    ┌──────────────────────┐
    │ Generate image based │ ～S22
    │ on variable information │
    └──────────┬───────────┘
               │
               ▼
    ┌──────────────────────┐
    │    Output image      │ ～S23
    │                      │
    └──────────┬───────────┘
               │
               ▼
        ┌─────────────┐
        │     End     │
        └─────────────┘
```

## INTERNATIONAL SEARCH REPORT

| | International application No. |
|---|---|
| | **PCT/JP2022/036261** |

### A. CLASSIFICATION OF SUBJECT MATTER

*G16C 20/80*(2019.01)i; *G16C 60/00*(2019.01)i
FI: G16C20/80; G16C60/00

According to International Patent Classification (IPC) or to both national classification and IPC

### B. FIELDS SEARCHED

Minimum documentation searched (classification system followed by classification symbols)

G16C20/80; G16C60/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | 金子 弘昌. 化学のためのPythonによるデータ解析・機械学習入門, 第1版, 株式会社オーム社 村上 和夫. 25 October 2019, pp. 111-127, ISBN: 978-4-274-22441-6 non-official translation (KANEKO, Hiromasa. Introduction to Using Python for Data Analysis and Machine Learning in Science. 1st ed. Ohmsha. Murakami, Kazumi.) pp. 112-127 | 1, 24, 26 |
| Y | pp. 112-127 | 2-13 |
| Y | JP 2004-182839 A (MITSUBISHI CHEMICAL CORP.) 02 July 2004 (2004-07-02) paragraph [0030], fig. 4 | 2-13 |
| X | paragraph [0030], fig. 4 | 14-23, 25, 27 |
| Y | 中村 雅之 外2名, 環境センシングの研究 (1), NTT R&D, 10 July 1993, vol. 42 no. 7, pp. 933-940, ISSN: 0915-2326 non-official translation (NAKAMURA, Masayuki et al. Environmental Sensing Research.) pp. 933-940 | 3-7, 9-13 |
| A | JP 2006-318048 A (KYOTO UNIVERSITY) 24 November 2006 (2006-11-24) entire text, all drawings | 1-27 |

✓ Further documents are listed in the continuation of Box C.　　✓ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **07 December 2022** | **20 December 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT

International application No.

**PCT/JP2022/036261**

**C.** DOCUMENTS CONSIDERED TO BE RELEVANT

| Category* | Citation of document, with indication, where appropriate, of the relevant passages | Relevant to claim No. |
|---|---|---|
| A | JP 4780554 B2 (YAMATO, Hiroshi) 28 September 2011 (2011-09-28)<br>entire text, all drawings | 1-27 |

Form PCT/ISA/210 (second sheet) (January 2015)

**INTERNATIONAL SEARCH REPORT**
Information on patent family members

International application No.

**PCT/JP2022/036261**

| Patent document cited in search report | | | Publication date (day/month/year) | Patent family member(s) | | | Publication date (day/month/year) |
|---|---|---|---|---|---|---|---|
| JP | 2004-182839 | A | 02 July 2004 | (Family: none) | | | |
| JP | 2006-318048 | A | 24 November 2006 | WO | 2006/121057 | A1 | |
| JP | 4780554 | B2 | 28 September 2011 | (Family: none) | | | |

Form PCT/ISA/210 (patent family annex) (January 2015)

**EP 4 425 503 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006318048 A

- JP 2001503546 W